# EUROPÄISCHE PATENTANMELDUNG

(11) **EP 0 939 078 A2**
(43) Veröffentlichungstag der Anmeldung: **01.09.1999**
(21) Anmeldenummer: 98123788.6
(22) Anmeldetag: 17.10.1995
(51) Int. Cl.: C07D 209/48, A01N 37/46

(54) **Substituierte Phthalimido-Zimtsäurederivate mit herbizider Wirkung**

(30) Priorität: 28.10.1994 DE 4438578
(62) Teilanmeldung aus: 95936508.1
(71) Anmelder: BASF AKTIENGESELLSCHAFT, 67056 Ludwigshafen (DE)
(72) Erfinder: Heistracher, Elisabeth Dr., 68159 Mannheim (DE); Plath, Peter Dr., 67227 Frankenthal (DE); Sweder von dem Bussche-Hünnefeld, Christoph Dr., 68623 Lampertheim (DE); Hamprecht, Gerhard Dr., 69469 Weinheim (DE); Klintz, Ralf Dr., 34292 Ahnatal (DE); Schäfer, Peter Dr., 67308 Ottersheim (DE); Westphalen, Karl-Otto Dr., 67346 Speyer (DE); Walter, Helmut Dr., 67283 Obrigheim (DE); Gerber, Matthias Dr., 67117 Limburgerhof (DE); Misslitz, Ulf Dr., 67433 Neustadt (DE)

(57) **Zusammenfassung**

Substituierte Phthalimido-Zimtsäurederivate I 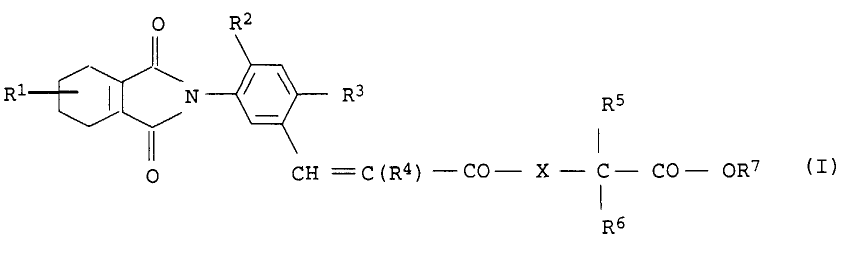 wobei
- R¹=: H, C₁-C₄-Alkyl;
- R²=: H, Halogen;
- R³=: CN, NO₂, Halogen, C₁-C₆-Alkyl, C₁-C₆-Halogenalkyl, C₁-C₆-Alkoxy, C₁-C₆-Halogenalkoxy;
- R⁴=: H, CN, NO₂, Halogen, C₁-C₆-Alkyl;
- R⁵=: H, C₁-C₆-Alkyl, C₂-C₆-Alkenyl, C₂-C₆-Alkinyl, C₁-C₆-Halogenalkyl, C₁-C₆-Cyanoalkyl, C₁-C₆-Hydroxyalkyl, C₁-C₆-Mercaptoalkyl, C₁-C₆-Alkoxy-C₁-C₆-alkyl, C₁-C₆-Alkylthio-C₁-C₆-alkyl;
- R⁶=: einen der unter R⁵ genannten Reste oder CN, NO₂, C₁-C₆-Alkoxy, C₁-C₆-Halogenalkoxy, C₁-C₆-Alkylthio, C₁-C₆-Halogenalkylthio, C₃-C₆-Cycloalkyl, C₃-C₆-Cycloalkyl-C₁-C₆-alkyl, (C₁-C₆-Alkoxy)carbonyl-C₁-C₆-alkyl, Hydroxycarbonyl-C₁-C₆-alkyl, (C₁-C₆-Alkyl-amino)carbonyl-C₁-C₆-alkyl, Di(C₁-C₆-alkyl)aminocarbonyl-C₁-C₆-alkyl, Aminocarbonyl-C₁-C₆-alkyl, (C₁-C₆-Alkyl)carbonyl-C₁-C₆-alkyl, C₁-C₆-Alkoxyimino-C₁-C₆-alkyl, Hydroxyimino-C₁-C₆-alkyl, Di-(C₁-C₆-alkoxy)-C₁-C₆-alkyl, Di-(C₁-C₆-alkylthio)-C₁-C₆-alkyl, C₃-C₆-Halogenalkenyl, (C₁-C₆-Alkoxy)carbonyl, COOH, (C₁-C₆-Alkylamino)carbonyl, Di-(C₁-C₆-alkyl)aminocarbonyl, Aminocarbonyl-(C₁-C₆-alkyl)carbonyl, (C₁-C₆-Halogenalkyl)carbonyl, geg. subst. Aryl, Hetaryl, Aryl-C₁-C₆-alkyl oder Hetaryl-C₁-C₆-alkyl;
oder R⁵ und R⁶ zusammen = Alkylenkette, in der eine Methyleneinheit durch -O- oder eine C₁-C₄-Alkyliminogruppe ersetzt sein kann;
- R⁷=: H, C₁-C₆-Alkyl, C₃-C₆-Alkenyl, C₃-C₆-Alkinyl, C₂-C₆-Halogenalkyl, C₁-C₆-Cyanoalkyl, C₁-C₆-Alkoxy-C₂-C₆-alkyl, C₁-C₆-Alkylthio-C₂-C₆-alkyl, (C₁-C₆-Alkoxy)carbonyl-C₁-C₆-alkyl, C₃-C₆-Cycloalkyl, C₃-C₆-Cycloalkyl-C₁-C₆-alkyl, geg. subst. Phenyl oder Benzyl;
- X=: s oder N(R⁸), wobei
R⁸ für einen der unter R⁷ genannten Reste oder für (C₁-C₆-Alkoxy)carbonyl, (C₁-C₆-Halogenalkoxy)carbonyl, (C₁-C₆-Alkyl)carbonyl, (C₁-C₆-Halogenalkyl)carbonyl, Tri-(C₁-C₆-alkyl)-silyl, Aryloxycarbonyl, Arylmethoxycarbonyl steht oder zusammen mit R⁶ eine Alkyenkette, in der eine nicht endständige Methyleneinheit durch Sauerstoff oder eine C₁-C₄-Alkyliminogruppe oder in der die N-gebundene Methyleneinheit durch eine Carbonylgruppe ersetzt sein kann, bedeutet;
sowie die Salze von I.

Verwendung: Herbizide; Desiccation/Defoliation von Pflanzen.

## Beschreibung

Die vorliegende Erfindung betrifft neue substituierte Phthalimido-Zimtsäurederivate der Formel I 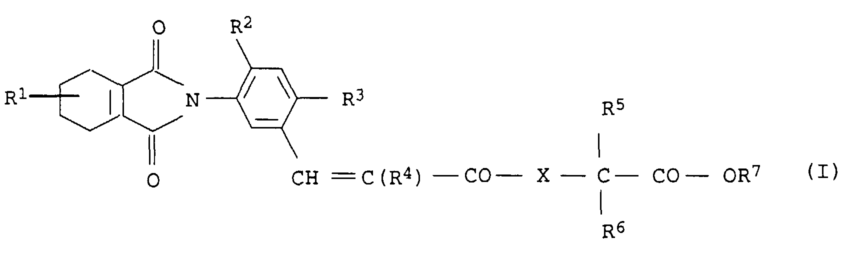 in der die Substituenten folgende Bedeutung haben:
- R¹: Wasserstoff oder C₁-C₄-Alkyl;
- R²: Wasserstoff oder Halogen;
- R³: Cyano, Nitro, Halogen, C₁-C₆-Alkyl, C₁-C₆-Halogenalkyl, C₁-C₆-Alkoxy oder C₁-C₆-Halogenalkoxy;
- R⁴: Wasserstoff, Cyano, Nitro, Halogen oder C₁-C₆-Alkyl;
- R⁵: Wasserstoff, C₁-c₆-Alkyl, C₂-C₆-Alkenyl, C₂-C₆-Alkinyl, C₁-C₆-Halogenalkyl, C₁-C₆-Cyanoalkyl, C₁-C₆-Hydroxyalkyl, C₁-C₆-Mercaptoalkyl, C₁-C₆-Alkoxy-C₁-C₆-alkyl oder C₁-C₆-Alkylthio-C₁-C₆-alkyl;
- R⁶: einen der unter R⁵ genannten Reste oder Cyano, Nitro, C₁-C₆-Alkoxy, C₁-C₆-Halogenalkoxy, C₁-C₆-Alkylthio, C₁-C₆-Halogenalkylthio, C₃-C₆-Cycloalkyl, C₃-C₆-Cycloalkyl-C₁-C₆-alkyl, (C₁-C₆-Alkoxy)carbonyl-C₁-C₆-alkyl, Hydroxycarbonyl-C₁-C₆-alkyl, (C₁-C₆-Alkylamino)carbonyl-C₁-C₆-alkyl, Di(C₁-C₆-alkyl)aminocarbonyl-C₁-C₆-alkyl, Aminocarbonyl-C₁-C₆-alkyl, (C₁-C₆-Alkyl)carbonyl-C₁-C₆-alkyl, C₁-C₆-Alkoxyimino-C₁-C₆-alkyl, Hydroxyimino-C₁-C₆-alkyl, Di-(C₁-C₆-alkoxy)-C₁-C₆-alkyl, Di-(C₁-C₆-alkylthio)-C₁-C₆-alkyl, C₃-C₆-Halogenalkenyl, (C₁-C₆-Alkoxy)carbonyl, Hydroxycarbonyl, (C₁-C₆-Alkylamino)carbonyl, Di-(C₁-C₆-alkyl)aminocarbonyl, Aminocarbonyl-(C₁-C₆-alkyl)carbonyl, (C₁-C₆-Halogenalkyl)carbonyl, Aryl, Hetaryl, Aryl-C₁-C₆-alkyl oder Hetaryl-C₁-C₆-alkyl, wobei die Aryl- und Heteroarylringe gewünschtenfalls ein bis drei Reste tragen können, ausgewählt aus der Gruppe bestehend aus Cyano, Nitro, Halogen, C₁-C₆-Alkyl, C₁-C₆-Halogenalkyl, C₁-C₆-Alkoxy, C₁-C₆-Halogenalkoxy, C₁-C₆-Alkylthio, C₁-C₆-Halogenalkylthio, Hydroxy, Mercapto und (C₁-C₆-Alkoxy)carbonyl;
oder
- R⁵ und R⁶: zusammen eine zwei- bis sechsgliedrige Alkylenkette, in der eine Methyleneinheit durch Sauerstoff oder eine C₁-C₄-Alkyliminogruppe ersetzt sein kann;
- R⁷: Wasserstoff, C₁-C₆-Alkyl, C₃-C₆-Alkenyl, C₃-C₆-Alkinyl, C₂-C₆-Halogenalkyl, C₁-C₆-Cyanoalkyl, C₁-C₆-Alkoxy-C₂-C₆-alkyl, C₁-C₆-Alkylthio-C₂-C₆-alkyl, (C₁-C₆-Alkoxy)carbonyl-C₁-C₆-alkyl, C₃-C₆-Cycloalkyl, C₃-C₆-Cycloalkyl-C₁-C₆-alkyl, Phenyl oder Benzyl, wobei die Phenylringe jeweils ein bis drei Reste tragen können, ausgewählt aus der Gruppe bestehend aus Cyano, Nitro, Halogen, C₁-C₆-Alkyl, C₁-C₆-Halogenalkyl, C₁-C₆-Alkoxy, C₁-C₆-Halogenalkoxy, C₁-C₆-Alkylthio, C₁-C₆-Halogenalkylthio und (C₁-C₆-Alkoxy)carbonyl;
- X: Sauerstoff, Schwefel oder -N(R⁸)-, wobei
- R⁸: für einen der unter R⁷ genannten Reste oder für (C₁-C₆-Alkoxy)carbonyl, (C₁-C₆-Halogenalkoxy)carbonyl, (C₁-C₆-Alkyl)carbonyl, (C₁-C₆-Halogenalkyl)carbonyl, Tri-(C₁-C₆-alkyl)-silyl, Aryloxycarbonyl, Arylmethoxycarbonyl steht
oder zusammen mit R⁶ für eine drei- bis 5-gliedrige Alkylenkette, in der eine nicht endständige Methyleneinheit durch Sauerstoff oder eine C₁-C₄-Alkyliminogruppe oder in der die N-gebundene Methyleneinheit durch eine Carbonylgruppe ersetzt sein kann,
sowie die landwirtschaftliche brauchbaren Salze von I.

Außerdem betrifft die Erfindung neue Zwischenprodukte der Formeln IIa und IIb, wobei die Substituenten R² bis R⁷ und die Variable X die selben Bedeutungen wie bei den Verbindungen I haben.

Des weiteren betrifft die Erfindung
- die Verwendung der Verbindungen I als Herbizide und/oder zur Desiccation und/oder Defoliation von Pflanzen,
- herbizide Mittel und Mittel zur Desiccation und/oder Defoliation von Pflanzen, welche die Verbindungen I als wirksame Substanzen enthalten,
- Verfahren zur Herstellung der Verbindungen I und von herbiziden Mitteln und Mitteln zur Desiccation und/oder Defoliation von Pflanzen unter Verwendung der Verbindungen I, sowie
- Verfahren zur Bekämpfung von unerwünschtem Pflanzenwuchs und zur Desiccation und/oder Defoliation von Pflanzen mit den Verbindungen I.

Aus der EP-A 240 659, EP-A 300 387 und EP-A 400 427 sind bereits herbizid wirksame Zimtsäurederivate bekannt. Deren Wirkung ist jedoch, besonders bei niedrigen Aufwandmengen, nicht immer voll befriedigend.

Der Erfindung lag daher die Aufgabe zugrunde, Zimtsäurederivate mit verbesserter herbiziden Wirkung bereitzustellen.

Demgemäß wurden die eingangs definierten substituierten Phthalimido-Zimtsäurederivate der Formel I gefunden. Ferner wurden herbizide Mittel gefunden, die die Verbindungen I enthalten und eine sehr gute herbizide Wirkung besitzen. Außerdem wurden Verfahren zur Herstellung dieser Mittel und Verfahren zur Bekämpfung von unerwünschtem Pflanzenwuchs mit den Verbindungen I gefunden.

Die erfindungsgemäßen Verbindungen I eignen sich des weiteren zur Defoliation und Desiccation von Pflanzenteilen für z.B. Baumwolle, Kartoffel, Raps, Sonnenblume, Sojabohne oder Ackerbohnen, insbesondere für Baumwolle. Diesbezüglich wurden Mittel zur Desiccation und/oder Defoliation von Pflanzen, Verfahren zur Herstellung dieser Mittel und Verfahren zur Desiccation und/oder Defoliation von Pflanzen mit den Verbindungen I gefunden.

Die Verbindungen der Formel I können je nach Substitutionsmuster ein oder mehrere Chiralitätszentren enthalten und liegen dann als Enantiomeren- oder Diastereomerengemische vor. Gegenstand der Erfindung sind sowohl die reinen Enantiomeren oder Diastereomeren als auch deren Gemische.

Die substituierten Phthalimido-Zimtsäurederivate I können in Form ihrer landwirtschaftlich brauchbaren Salze vorliegen, wobei es auf die Art des Salzes in der Regel nicht ankommt. Im allgemeinen kommen die Salze von solchen Basen und diejenigen Säureadditionssalze in Betracht, bei denen die herbizide Wirkung im Vergleich zu der freien Verbindung I nicht negativ beeinträchtigt ist.

Als basische Salze eignen sich besonders diejenigen der Alkalimetalle, vorzugsweise Natrium- und Kaliumsalze, der Erdalkalimetalle, vorzugsweise Calcium- und Magnesiumsalze, die der Übergangsmetalle, vorzugsweise Zink- und Eisensalze, sowie Ammoniumsalze, bei denen das Ammoniumion gewünschtenfalls ein bis drei C₁-C₄-Alkyl-, Hydroxy-C₁-C₄-alkylsubstituenten und/oder einen Phenyl- oder Benzylsubstituenten tragen kann, vorzugsweise Diisopropylammonium-, Tetramethylammonium-, Tetrabutylammonium-, Trimethylbenzylammonium- und Trimethyl-(2-hydroxyethyl)-ammoniumsalze, des weiteren Phosphoniumsalze, Sulfoniumsalze wie vorzugsweise Tri-(C₁-C₄-alkyl)sulfonium-salze, und Sulfoxoniumsalze wie vorzugseise Tri-(C₁-C₄-alkyl)sulfoxoniumsalze.

Unter den Säureadditionssalzen sind in erster Linie die Hydrochloride und -bromide, Sulfate, Nitrate, Phosphate, Oxalate und die Dodecylbenzolsulfonate zu nennen.

Die für die Substituenten R¹ bis R⁸ oder als Reste an Phenyl-, Aryl- oder Heteroarylringen genannten organischen Molekülteile stellen - wie die Bedeutung Halogen - Sammelbegriffe für individuelle Aufzählungen der einzelnen Gruppenmitglieder dar. Sämtliche Kohlenstoffketten, also alle Alkyl-, Halogenalkyl, Alkenyl-, Alkinyl-, Alkoxy-, Alkylthio-, Halogenalkoxy-, Halogenalkylthio-, Alkenyloxy-, Alkinyloxy-, Alkoxycarbonyl-, Alkylimino- oder Alkoxyimino-Teile können geradkettig oder verzweigt sein. Halogenierte Substituenten tragen vorzugsweise ein bis fünf gleiche oder verschiedene Halogenatome.

Im einzelnen stehen beispielsweise:
- Halogen für: Fluor, Chlor, Brom oder Iod;
- C₁-C₄-Alkyl für: Methyl, Ethyl, n-Propyl, 1-Methylethyl, n-Butyl, 1-Methylpropyl, 2-Methylpropyl oder 1,1-Dimethylethyl;
- C₁-C₆-Alkyl sowie die Alkyl-Teile von C₃-C₆-Cycloalkyl-C₁-C₆-alkyl, C₁-C₆-Alkoxy-C₁-C₆-alkyl, Di-(C₁-C₆-alkoxy)-C₁-C₆-alkyl, C₁-C₆-Alkylthio-C₁-C₆-alkyl, Di-(C₁-C₆-alkylthio)-C₁-C₆-alkyl, (C₁-C₆-Alkyl)carbonyl-C₁-C₆-alkyl, (C₁-C₆-Alkoxy)carbonyl-C₁-C₆-alkyl, C₁-C₆-Alkoxyimino-C₁-C₆-alkyl, (C₁-C₆-Alkylamino)carbonyl-C₁-C₆-alkyl, Di(C₁-C₆-alkyl)aminocarbonyl-C₁-C₆-alkyl, Tri-(C₁-C₆-alkyl)-silyl, Aryl-C₁-C₆-alkyl und Hetaryl-C₁-C₆-alkyl für: einen der bei C₁-C₄-Alkyl genannten Reste oder n-Pentyl, 1-Methylbutyl, 2-Methylbutyl, 3-Methylbutyl, 2,2-Dimethylpropyl, 1-Ethylpropyl, 1,1-Dimethylpropyl, 1,2-Dimethylpropyl, n-Hexyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropl, 1,2,2-Trimethylpropyl, 1-Ethyl-1-methylpropyl oder 1-Ethyl-2-methylpropyl;
- die Alkylteile von C₁-C₆-Alkoxy-C₂-C₆-alkyl und C₁-C₆-Alkylthio-C₂-C₆-alkyl für: Ethyl, n-Propyl, 1-Methylethyl, n-Butyl, 1-Methylpropyl, 2-Methylpropyl, 1,1-Dimethylethyleinen oder einen der bei C₁-C₆-Alkyl zusätzlich aufgeführten Reste;
- C₂-C₆-Halogenalkyl für: C₁-C₆-Alkyl wie vorstehend genannt, mit Ausnahme von Methyl, das partiell oder vollständig durch Fluor, Chlor, Brom und/oder Iod substituiert ist, also z.B. 2-Fluorethyl, 2-Chlorethyl, 2-Bromethyl, 2-Iodethyl, 2,2-Difluorethyl, 2,2,2-Trifluorethyl, 2-Chlor-2-fluorethyl, 2-Chlor-2,2-difluorethyl, 2,2-Dichlor-2-fluorethyl, 2,2,2-Trichlorethyl, Petafluorethyl, 2-Fluorpropyl,3-Fluorpropyl, 2,2-Difluorpropyl, 2,3-Difluorpropyl, 2-Chlorpropyl, 3-Chlorpropyl, 2,3-Dichlorpropyl, 2-Brompropyl, 3-Brompropyl, 3,3,3-Trifluorpropyl, 3,3,3-Trichlorpropyl, 2,2,3,3,3-Pentafluorpropyl, Heptafluorpropyl, 1-(Fluormethyl)-2-fluorethyl, 1-(Chlormethyl)-2-chlorethyl, 1-(Brommethyl)-2-bromethyl, 4-Fluorbutyl, 4-Chlorbutyl, 4-Brombutyl, Nonafluorbutyl, 5-Fluorpentyl, 5-Chlorpentyl, 5-Brompentyl, 5-Iodpentyl, Undecafluorpentyl, 6-Fluorhexyl, 6-Chlorhexyl, 6-Bromhexyl, 6-Iodhexyl oder Dodecafluorhexyl;
- C₁-C₆-Halogenalkyl für: einen C₁-C₆-Alkylrest wie vorstehend genannt, der partiell oder vollständig durch Fluor, Chlor, Brom und/oder Iod substituiert ist, also z.B. Chlormethyl, Dichlormethyl, Trichlomethyl, Fluormethyl, Difluormethyl, Trifluormethyl, Chlorfluomethyl, Dichlorfluormethyl, Chlordifluormethyl oder einen der bei C₂-C₆-Halogenalkyl genannten Reste;
- C₃-C₆-Cycloalkyl sowie der Cycloalkyl-Teil von C₃-C₆-Cycloalkyl-C₁-C₆-alkyl für: Cyclopropyl, Cyclobutyl, Cyclopentyl oder Cyclohexyl;
- C₃-C₆-Alkenyl für: Prop-1-en-1-yl, Prop-2-en-1-yl, 1-Methylethenyl, n-Buten-1-yl, n-Buten-2-yl, n-Buten-3-yl, 1-Methyl-prop-1-en-1-yl, 2-Methyl-prop-1-en-1-yl, 1-Methyl-prop-2-en-1-yl oder 2-Methyl-prop-2-en-1-yl, n-Penten-1-yl, n-Penten-2-yl, n-Penten-3-yl, n-Penten-4-yl, 1-Methyl-but-1-en-1-yl, 2-Methyl-but-1-en-1-yl, 3-Methyl-but-1-en-1-yl, 1-Methyl-but-2-en-1-yl, 2-Methyl-but-2-en-1-yl, 3-Methyl-but-2-en-1-yl, 1-Methyl-but-3-en-1-yl, 2-Methyl-but-3-en-1-yl, 3-Methyl-but-3-en-1-yl, 1,1-Dimethyl-prop-2-en-1-yl, 1,2-Dimethyl-prop-1-en-1-yl, 1,2-Dimethyl-prop-2-en-1-yl, 1-Ethyl-prop-1-en-2-yl, 1-Ethyl-prop-2-en-1-yl, n-Hex-1-en-1-yl, n-Hex-2-en-1-yl, n-Hex-3-en-1-yl, n-Hex-4-en-1-yl, n-Hex-5-en-1-yl, 1-Methyl-pent-1-en-1-yl, 2-Mmethyl-pent-1-en-1-yl, 3-Methyl-pent-1-en-1-yl, 4-Methyl-pent-1-en-1-yl, 1-Methyl-pent-2-en-1-yl, 2-Methyl-pent-2-en-1-yl, 3-Methyl-pent-2-en-1-yl, 4-Methyl-pent-2-en-1-yl, 1-Methyl-pent-3-en-1-yl, 2-Methyl-pent-3-en-1-yl, 3-Methyl-pent-3-en-1-yl, 4-Methyl-pent-3-en-1-yl, 1-Methyl-pent-4-en-1-yl, 2-Methyl-pent-4-en-1-yl, 3-Methyl-pent-4-en-1-yl, 4-Methyl-pent-4-en-1-yl, 1,1-Dimethyl-but-2-en-1-yl, 1,1-Dimethyl-put-3-en-1-yl, 1,2-Dimethyl-but-1-en-1-yl, 1,2-Dimetyl-put-2-en-1-yl, 1,2-Dimethyl-but-3-en-1-yl, 1,3-Dimetyl-put-1-en-1-yl, 1,3-Dimethyl-but-2-en-1-yl, 1,3-Dimetyl-put-3-en-1-yl, 2,2-Dimethyl-but-3-en-1-yl, 2,3-Dimetyl-put-1-en-1-yl, 2,3-Dimethyl-but-2-en-1-yl, 2,3-Dimetyl-put-3-en-1-yl, 3,3-Dimethyl-but-1-en-1-yl, 3,3-Dimetyl-put-2-en-1-yl, 1-Ethyl-but-1-en-1-yl, 1-Ethyl-but-2-en-1-yl, 1-Ethyl-but-3-en-1-yl, 2-Ethyl-but-1-en-1-yl, 2-Ethyl-but-2-en-1-yl, 2-Ethyl-but-3-en-1-yl, 1,1,2-Trimethyl-prop-2-en-1-yl, 1-Ethyl-1-methyl-prop-2-en-1-yl, 1-Ethyl-2-methyl-prop-1-en-1-yl oder 1-Ethyl-2-methyl-prop-2-en-1-yl;
- C₂-C₆-Alkenyl für: Ethenyl oder einen der bei C₃-C₆-Alkenyl aufgeführten Reste;
- C₃-C₆-Halogenalkenyl für: C₃-C₆-Alkenyl wie vorstehend genannt, das partiell oder vollständig durch Fluor, Chlor und/oder Brom suabstituiert ist, also z.B. 2-Chlorallyl, 3-Chlorallyl oder 3,3-Dichlorallyl;
- C₃-C₆-Alkinyl für: Prop-1-in-1-yl, Prop-2-in-1-yl, But-1-in-1-yl, But-1-in-3-yl, But-1-in-4-yl, But-2-in-1-yl, n-Pent-1-in-1-yl, n-Pent-1-in-3-yl, n-Pent-1-in-4-yl, n-Pent-1-in-5-yl, n-Pent-2-in-1-yl, n-Pent-2-in-4-yl, n-Pent-2-in-5-yl, 3-Methyl-but-1-in-3-yl, 3-Methyl-but-1-in-4-yl, n-Hex-1-in-1-yl, n-Hex-1-in-3-yl, n-Hex-1-in-4-yl, n-Hex-1-in-5-yl, n-Hex-1-in-6-yl, n-Hex-2-in-1-yl, n-Hex-2-in-4-yl, n-Hex-2-in-5-yl, n-Hex-2-in-6-yl, n-Hex-3-in-1-yl, n-Hex-3-in-2-yl, 3-Methyl-pent-1-in-1-yl, 3-Methyl-pent-1-in-3-yl, 3-Methyl-pent-1-in-4-yl, 3-Methyl-pent-1-in-5-yl, 4-Methyl-pent-1-in-1-yl, 4-Methyl-pent-2-in-4-yl oder 4-Methyl-pent-2-in-5-yl;
- C₂-C₆-Alkinyl für: Ethinyl oder einen der bei C₃-C₆-Alkinyl aufgeführten Reste;
- C₁-C₆-Cyanoalkyl für: Cyanomethyl, 1-Cyanoeth-1-yl, 2-Cyanoeth-1-yl, 1-Cyanoprop-1-yl, 2-Cyanoprop-1-yl, 3-Cyanoprop-1-yl, 1-Cyanoprop-2-yl, 2-Cyanoprop-2-yl, 1-Cyanobut-1-yl, 2-Cyanobut-1-yl, 3-Cyanobut-1-yl, 4-Cyanobut-1-yl, 1-Cyanobut-2-yl, 2-Cyano-but-2-yl, 1-Cyanobut-3-yl, 2-Cyanobut-3-yl, 1-Cyano-2-methyl-prop-3-yl, 2-Cyano-2-methyl-prop-3-yl, 3-Cyano-2-methyl-prop-3-yl, 2-Cyano-methyl-prop-2-yl, 1-Cyanopent-1-yl, 2-Cyanopent-1-yl, 3-Cyanopent-1-yl, 4-Cyanopent-1-yl, 5-Cyanopent-1-yl, 1-Cyanopent-2-yl, 2-Cyanopent-2-yl, 1-Cyanopent-3-yl, 2-Cyanopent-3-yl, 1-Cyanopent-4-yl, 2-Cyanopent-4-yl, 3-Cyanopent-4-yl, 1-Cyano-2-ethyl-prop-3-yl, 1-Cyanohex-1-yl, 2-Cyanohex-1-yl, 3-Cyanohex-1-yl, 4-Cyanohex-1-yl, 5-Cyanohex-1-yl, 6-Cyanohex-1-yl, 1-Cyanohex-2-yl, 2-Cyanohex-2-yl, 1-Cyanohex-3-yl, 2-Cyanohex-3-yl, 1-Cyanohex-4-yl, 2-Cyanohex-4-yl, 3-Cyanohex-4-yl, 1-Cyano-2-ethyl-but-3-yl, 1-Cyano-2-ethyl-but-4-yl oder 1-Cyano-2-propyl-prop-3-yl;
- C₁-C₆-Hydroxyalkyl für: Hydroxymethyl, 1-Hydroxyeth-1-yl, 2-Hydroxyeth-1-yl, 1-Hydroxyprop-1-yl, 2-Hydroxyprop-1-yl, 3-Hydroxyprop-1-yl, 1-Hydroxyprop-2-yl, 2-Hydroxyprop-2-yl, 1-Hydroxybut-1-yl, 2-Hydroxybut-1-yl, 3-Hydroxybut-1-yl, 4-Hydroxybut-1-yl, 1-Hydroxybut-2-yl, 2-Hydroxy-but-2-yl, 1-Hydroxybut-3-yl, 2-Hydroxybut-3-yl, 1-Hydroxy-2-methyl-prop-3-yl, 2-Hydroxy-2-methyl-prop-3-yl, 3-Hydroxy-2-methyl-prop-3-yl, 2-Hydroxy-methyl-prop-2-yl, 1-Hydroxypent-1-yl, 2-Hydroxypent-1-yl, 3-Hydroxypent-1-yl, 4-Hydroxypent-1-yl, 5-Hydroxypent-1-yl, 1-Hydroxypent-2-yl, 2-Hydroxypent-2-yl, 1-Hydroxypent-3-yl, 2-Hydroxypent-3-yl, 1-Hydroxypent-4-yl, 2-Hydroxypent-4-yl, 3-Hydroxypent-4-yl, 1-Hydroxy-2-ethyl-prop-3-yl, 1-Hydroxyhex-1-yl, 2-Hydroxyhex-1-yl, 3-Hydroxyhex-1-yl, 4-Hydroxyhex-1-yl, 5-Hydroxyhex-1-yl, 6-Hydroxyhex-1-yl, 1-Hydroxyhex-2-yl, 2-Hydroxyhex-2-yl, 1-Hydroxyhex-3-yl, 2-Hydroxyhex-3-yl, 1-Hydroxyhex-4-yl, 2-Hydroxyhex-4-yl, 3-Hydroxyhex-4-yl, 1-Hydroxy-2-ethyl-but-3-yl, 1-Hydroxy-2-ethyl-but-4-yl oder 1-Hydroxy-2-propyl-prop-3-yl;
- C₁-C₆-Mercaptoalkyl für: Mercaptomethyl, 1-Mercaptoeth-1-yl, 2-Mercaptoeth-1-yl, 1-Mercaptoprop-1-yl, 2-Mercaptoprop-1-yl, 3-Mercaptoprop-1-yl, 1-Mercaptoprop-2-yl, 2-Mercaptoprop-2-yl, 1-Mercaptobut-1-yl, 2-Mercaptobut-1-yl, 3-Mercaptobut-1-yl, 4-Mercaptobut-1-yl, 1-Mercaptobut-2-yl, 2-Mercapto-but-2-yl, 1-Mercaptobut-3-yl, 2-Mercaptobut-3-yl, 1-Mercapto-2-methyl-prop-3-yl, 2-Mercapto-2-methyl-prop-3-yl, 3-Mercapto-2-methyl-prop-3-yl, 2-Mercapto-methyl-prop-2-yl, 1-Mercaptopent-1-yl, 2-Mercaptopent-1-yl, 3-Mercaptopent-1-yl, 4-Mercaptopent-1-yl, 5-Mercaptopent-1-yl, 1-Mercaptopent-2-yl, 2-Mercaptopent-2-yl, 1-Mercaptopent-3-yl, 2-Mercaptopent-3-yl, 1-Mercaptopent-4-yl, 2-Mercaptopent-4-yl, 3-Mercaptopent-4-yl, 1-Mercapto-2-ethyl-prop-3-yl, 1-Mercaptohex-1-yl, 2-Mercaptohex-1-yl, 3-Mercaptohex-1-yl, 4-Mercaptohex-1-yl, 5-Mercaptohex-1-yl, 6-Mercaptohex-1-yl, 1-Mercaptohex-2-yl, 2-Mercaptohex-2-yl, 1-Mercaptohex-3-yl, 2-Mercaptohex-3-yl, 1-Mercaptohex-4-yl, 2-Mercaptohex-4-yl, 3-Mercaptohex-4-yl, 1-Mercapto-2-ethyl-but-3-yl, 1-Mercapto-2-ethyl-but-4-yl oder 1-Mercapto-2-propyl-prop-3-yl;
- Hydroxycarbonyl-C₁-C₆-alkyl für: Hydroxycarbonylmethyl, 1-(Hydroxycarbonyl)eth-1-yl, 2-(Hydroxycarbonyl)eth-1-yl, 1-(Hydroxycarbonyl)prop-1-yl, 2-(Hydroxycarbonyl)prop-1-yl, 3-(Hydroxycarbonyl)prop-1-yl, 1-(Hydroxycarbonyl)prop-2-yl, 2-(Hydroxycarbonyl)prop-2-yl, 1-(Hydroxycarbonyl)but-1-yl, 2-(Hydroxycarbonyl)but-1-yl, 3-(Hydroxycarbonyl)but-1-yl, 4-(Hydroxycarbonyl)but-1-yl, 1-(Hydroxycarbonyl)but-2-yl, 2-(Hydroxycarbonyl)but-2-yl, 1-(Hydroxycarbonyl)but-3-yl, 2-(Hydroxycarbonyl)but-3-yl, 1-(Hydroxycarbonyl)-2-methyl-prop-3-yl, 2-(Hydroxycarbonyl)-2-methyl-prop-3-yl, 3-(Hydroxycarbonyl)-2-methyl-prop-3-yl, 2-(Hydroxycarbonylmethyl)-prop-2-yl, 1-(Hydroxycarbonyl)pent-1-yl, 2-(Hydroxycarbonyl)pent-1-yl, 3-(Hydroxycarbonyl)pent-1-yl, 4-(Hydroxycarbonyl)pent-1-yl, 5-(Hydroxycarbonyl)pent-1-yl, 1-(Hydroxycarbonyl)pent-2-yl, 2-(Hydroxycarbonyl)pent-2-yl, 1-(Hydroxycarbonyl)pent-3-yl, 2-(Hydroxycarbonyl)pent-3-yl, 1-(Hydroxycarbonyl)pent-4-yl, 2-(Hydroxycarbonyl)pent-4-yl, 3-(Hydroxycarbonyl)pent-4-yl, 1-(Hydroxycarbonyl)-2-ethyl-prop-3-yl, 1-(Hydroxycarbonyl)hex-1-yl, 2-(Hydroxycarbonyl)hex-1-yl, 3-(Hydroxycarbonyl)hex-1-yl, 4-(Hydroxycarbonyl)hex-1-yl, 5-(Hydroxycarbonyl)hex-1-yl, 6-(Hydroxycarbonyl)hex-1-yl, 1-(Hydroxycarbonyl)hex-2-yl, 2-(Hydroxycarbonyl)hex-2-yl, 1-(Hydroxycarbonyl)hex-3-yl, 2-(Hydroxycarbonyl)hex-3-yl, 1-(Hydroxycarbonyl)hex-4-yl, 2-(Hydroxycarbonyl)hex-4-yl, 3-(Hydroxycarbonyl)hex-4-yl, 1-(Hydroxycarbonyl)-2-ethyl-but-3-yl, 1-(Hydroxycarbonyl)-2-ethyl-but-4-yl oder 1-(Hydroxycarbonyl)-2-propyl-prop-3-yl;
- Hydroxyimino-C₁-C₆-alkyl für: Hydroxyiminomethyl, 1-(Hydroxyimino)eth-1-yl, 2-(Hydroxyimino)eth-1-yl, 1-(Hydroxyimino)prop-1-yl, 2-(Hydroxyimino)prop-1-yl, 3-(Hydroxyimino)prop-1-yl, 1-(Hydroxyimino)prop-2-yl, 2-(Hydroxyimino)prop-2-yl, 1-(Hydroxyimino)but-1-yl, 2-(Hydroxyimino)but-1-yl, 3-(Hydroxyimino)but-1-yl, 4-(Hydroxyimino)but-1-yl, 1-(Hydroxyimino)but-2-yl, 2-(Hydroxyimino)but-2-yl, 1-(Hydroxyimino)but-3-yl, 2-(Hydroxyimino)but-3-yl, 1-(Hydroxyimino)-2-methyl-prop-3-yl, 2-(Hydroxyimino)-2-methyl-prop-3-yl, 3-(Hydroxyimino)-2-methyl-prop-3-yl, 2-(Hydroxyiminomethyl)-prop-2-yl, 1-(Hydroxyimino)pent-1-yl, 2-(Hydroxyimino)pent-1-yl, 3-(Hydroxyimino)pent-1-yl, 4-(Hydroxyimino)pent-1-yl, 5-(Hydroxyimino)pent-1-yl, 1-(Hydroxyimino)pent-2-yl, 2-(Hydroxyimino)pent-2-yl, 1-(Hydroxyimino)pent-3-yl, 2-(Hydroxyimino)pent-3-yl, 1-(Hydroxyimino)pent-4-yl, 2-(Hydroxyimino)pent-4-yl, 3-(Hydroxyimino)pent-4-yl, 1-(Hydroxyimino)-2-ethyl-prop-3-yl, 1-(Hydroxyimino)hex-1-yl, 2-(Hydroxyimino)hex-1-yl, 3-(Hydroxyimino)hex-1-yl, 4-(Hydroxyimino)hex-1-yl, 5-(Hydroxyimino)hex-1-yl, 6-(Hydroxyimino)hex-1-yl, 1-(Hydroxyimino)hex-2-yl, 2-(Hydroxyimino)hex-2-yl, 1-(Hydroxyimino)hex-3-yl, 2-(Hydroxyimino)hex-3-yl, 1-(Hydroxyimino)hex-4-yl, 2-(Hydroxyimino)hex-4-yl, 3-(Hydroxyimino)hex-4-yl, 1-(Hydroxyimino)-2-ethyl-but-3-yl, 1-(Hydroxyimino)-2-ethyl-but-4-yl oder 1-(Hydroxyimino)-2-propyl-prop-3-yl;
- Aminocarbonyl-C₁-C₆-alkyl für: Aminocarbonylmethyl, 1-(Aminocarbonyl)eth-1-yl, 2-(Aminocarbonyl)eth-1-yl, 1-(Aminocarbonyl)prop-1-yl, 2-(Aminocarbonyl)prop-1-yl, 3-(Aminocarbonyl)prop-1-yl, 1-(Aminocarbonyl)prop-2-yl, 2-(Aminocarbonyl)prop-2-yl, 1-(Aminocarbonyl)but-1-yl, 2-(Aminocarbonyl)but-1-yl, 3-(Aminocarbonyl)but-1-yl, 4-(Aminocarbonyl)but-1-yl, 1-(Aminocarbonyl)but-2-yl, 2-(Aminocarbonyl)but-2-yl, 1-(Aminocarbonyl)but-3-yl, 2-(Aminocarbonyl)but-3-yl, 1-(Aminocarbonyl)-2-methyl-prop-3-yl, 2-(Aminocarbonyl)-2-methyl-prop-3-yl, 3-(Aminocarbonyl)-2-methyl-prop-3-yl, 2-(Aminocarbonylmethyl)-prop-2-yl, 1-(Aminocarbonyl)pent-1-yl, 2-(Aminocarbonyl)pent-1-yl, 3-(Aminocarbonyl)pent-1-yl, 4-(Aminocarbonyl)pent-1-yl, 5-(Aminocarbonyl)pent-1-yl, 1-(Aminocarbonyl)pent-2-yl, 2-(Aminocarbonyl)pent-2-yl, 1-(Aminocarbonyl)pent-3-yl, 2-(Aminocarbonyl)pent-3-yl, 1-(Aminocarbonyl)pent-4-yl, 2-(Aminocarbonyl)pent-4-yl, 3-(Aminocarbonyl)pent-4-yl, 1-(Aminocarbonyl)-2-ethyl-prop-3-yl, 1-(Aminocarbonyl)hex-1-yl, 2-(Aminocarbonyl)hex-1-yl, 3-(Aminocarbonyl)hex-1-yl, 4-(Aminocarbonyl)hex-1-yl, 5-(Aminocarbonyl)hex-1-yl, 6-(Aminocarbonyl)hex-1-yl, 1-(Aminocarbonyl)hex-2-yl, 2-(Aminocarbonyl)hex-2-yl, 1-(Aminocarbonyl)hex-3-yl, 2-(Aminocarbonyl)hex-3-yl, 1-(Aminocarbonyl)hex-4-yl, 2-(Aminocarbonyl)hex-4-yl, 3-(Aminocarbonyl)hex-4-yl, 1-(Aminocarbonyl)-2-ethyl-but-3-yl, 1-(Aminocarbonyl)-2-ethyl-but-4-yl oder 1-(Aminocarbonyl)-2-propyl-prop-3-yl;
- C₁-C₆-Alkoxy sowie die Alkoxy-Teile von C₁-C₆-Alkoxy-C₁-C₆-alkyl, C₁-C₆-Alkoxy-C₂-C₆-alkyl und Di-(C₁-C₆-alkoxy)-C₁-C₆-alkyl für: Methoxy, Ethoxy, n-Propoxy, 1-Methylethoxy, n-Butoxy, 1-Methylpropoxy, 2-Methylpropoxy oder 1,1-Dimethylethoxy, n-Pentoxy, 1-Methylbutoxy, 2-Methylbutoxy, 3-Methylbutoxy, 1,1-Dimethylpropoxy, 1,2-Dimethylpropoxy, 2,2-Dimethylpropoxy, 1-Ethylpropoxy, n-Hexoxy, 1-Methylpentoxy, 2-Methylpentoxy, 3-Methylpentoxy, 4-Methylpentoxy, 1,1-Dimethylbutoxy,1,2-Dimethylbutoxy, 1,3-Dimethylbutoxy, 2,2-Dimethylbutoxy, 2,3-Dimethylbutoxy, 3,3-Dimethylbutoxy, 1-Ethylbutoxy, 2-Ethylbutoxy, 1,1,2-Trimethylpropoxy, 1,2,2-Trimethyl-propoxy, 1-Ethyl-1-methylpropoxy oder 1-Ethyl-2-methylpropoxy;
- C₁-C₆-Halogenalkoxy für: C₁-C₆-Alkoxy wie vorstehend genannt, das partiell oder vollständig durch Fluor, Chlor, Brom und/oder Iod substituiert ist, also z.B. Difluormethoxy, Trifluormethoxy, Chlordifluormethoxy, Bromdifluormethoxy, 2-Fluorethoxy, 2-Chlorethoxy, 2-Bromethoxy, 2-Iodethoxy, 2,2-Difluorethoxy, 2,2,2-Trifluorethoxy, 2-Chlor-2-fluorethoxy, 2-Chlor-2,2-difluorethoxy, 2,2-Dichlor-2-fluorethoxy, 2,2,2-Trichlorethoxy, Pentafluorethoxy, 2-Fluorpropoxy, 3-Fluorpropoxy, 2-Chlorporpoxy, 3-Chlorpropoxy, 2-Brompropoxy, 3-Brompropoxy, 2,2-Difluorpropoxy, 2,3-Difluorpropoxy, 2,3-Dichlorpropoxy, 3,3,3-Trifluorpropoxy, 3,3,3-Trichlorpropoxy, 2,2,3,3,3-Pentafluorpropoxy, Heptafluorporpoxy, 1-(Fluormethyl)-2-fluorethoxy, 1-(Chlormethyl)-2-chlorethoxy, 1-(Brommethyl)-2-bromethoxy, 4-Fluorbutoxy, 4-Chlorbutoxy, 4-Brombutoxy, Nonafluorbutoxy, 5-Fluorpentoxy, 5-Chlorpentoxy, 5-Brompentoxy, 5-Iodpentoxy, Undecafluorpentoxy, 6-Fluorhexoxy, 6-Chlorhexoxy, 6-Bromhexoxy oder Dodecafluorhexoxy;
- C₁-C₆-Alkylthio sowie die Alkylthio-Teile von C₁-C₆-Alkylthio-C₁-C₆-alkyl, C₁-C₆-Alkylthio-C₂-C₆-alkyl und Di-(C₁-C₆-alkoxy)-C₁-C₆-alkyl für: Methylthio, Ethylthio, n-Propylthio, 1-Methylethylthio, n-Butylthio, 1-Methylpropylthio, 2-Methylpropylthio, 1,1-Dimethylethylthio, n-Pentylthio, 1-Methylbutylthio, 2-Methylbutylthio, 3-Methylbutylthio, 2,2-Di-methylpropylthio, 1-Ethylpropylthio, n-Hexylthio, 1,1-Dimethylpropylthio, 1,2-Dimethylpropylthio, 1-Methylpentylthio, 2-Methylpentylthio, 3-Methylpentylthio, 4-Methylpentylthio, 1,1-Dimethylbutylthio, 1,2-Dimethylbutylthio, 1,3-Dimethylbutylthio, 2,2-Dimethylbutylthio, 2,3-Dimethylbutylthio, 3,3-Dimethylbutylthio, 1-Ethylbutyl-thio, 2-Ethylbutylthio, 1,1,2-Trimethylpropylthio, 1,2,2-Trimethylpropylthio, 1-Ethyl-1-methylpropylthio oder 1-Ethyl-2-methylpropylthio;
- C₁-C₆-Halogenalkylthio für: C₁-C₆-Alkylthio wie vorstehend genannt, das partiell oder vollständig durch Fluor, Chlor, Brom und/oder Iod substituiert ist, also z.B. Difluormethylthio, Trifluormethylthio, Chlordifluormethylthio, Bromdifluormethylthio, 2-Fluorethylthio, 2-Chlorethylthio, 2-Bromethylthio, 2-Iodethylthio, 2,2-Difluorethylthio, 2,2,2-Trifluorethylthio, 2,2,2-Trichlorethylthio, 2-Chlor-2-fluorethylthio, 2-Chlor-2,2-difluorethylthio, 2,2,-Dichlor-2-fluorethylthio, Pentafluorethylthio, 2-Fluorpropylthio, 3-Fluorpropylthio, 2-Chlorpropylthio, 3-Chlorpropylthio, 2-Brompropylthio, 3-Brompropylthio, 2,2,-Difluorpropylthio, 2,3-Difluorpropylthio, 2,3-Dichlorpropylthio, 3,3,3-Trifluorpropylthio, 3,3,3-Trichlorpropylthio, 2,2,3,3,3-Pentafluorpropylthio, Heptafluorpropylthio, 1-(Fluormethyl)-2-fluorethylthio, 1-(Chlormethyl)-2-chlorethylthio, 1-(Brommethyl)-2-bromethylthio, 4-Fluorbutylthio, 4-Chlorbutylthio, 4-Brombutylthio, 5-Fluorpentylthio, 5-Chlorpentylthio, 5-Brompentylthio, 5-Iodpentylthio, Undecafluorpentylthio, 6-Fluorhexylthio oder 6-Chlorhexylthio;
- (C₁-C₆-Alkoxy)carbonyl sowie der Alkoxycarbonyl-Teil von (C₁-C₆-Alkoxy)carbonyl-C₁-C₆-alkyl für: Methoxycarbonyl, Ethoxycarbonyl, n-Propoxycarbonyl, 1-Methylethoxycarbonyl, n-Butoxycarbonyl, 1-Methylpropoxycarbonyl, 2-Methylpropoxycarbonyl, 1,1-Di-methylethoxycarbonyl, n-Pentoxycarbonyl, 1-Methylbutoxycarbonyl, 2-Methylbutoxycarbonyl, 3-Methylbutoxycarbonyl, 1,1-Dimethylpropoxycarbonyl, 1,2-Dimethylpropoxycarbonyl, 2,2-Dimethylpropoxycarbonyl, 1-Ethylpropoxycarbonyl, Hexoxycarbonyl, 1-Methylpentoxycarbonyl, 2-Methylpentoxycarbonyl, 3-Methylpentoxycarbonyl, 4-Methylpentoxycarbonyl, 1,1-Dimethylbutoxycarbonyl, 1,2-Dimethylbutoxycarbonyl, 1,3-Dimethylbutoxycarbonyl, 2,2-Dimethylbutoxycarbonyl, 2,3-Dimethylbutoxycarbonyl, 3,3-Dimethylbutoxycarbonyl, 1-Ethylbutoxycarbonyl, 2-Ethylbutoxycarbonyl, 1,1,2-Trimethylpropoxycarbonyl, 1,2,2-Trimethylpropoxycarbonyl, 1-Ethyl-1-methylpropoxycarbonyl oder 1-Ethyl-2-methylpropylcarbonyl;
- (C₁-C₆-Halogenalkoxy)carbonyl für: (C₁-C₆-Alkoxy)carbonyl wie voranstehend genannt, das partiell oder vollsltändig durch Fluor, Chlor und/oder Brom substituiert ist, also z.B. 2-Bromethoxycarbonyl, 2-Chlorethoxycarbonyl, 2-Fluorethoxycarbonyl, 2-Trifluormethyl-2-methylethoxycarbonyl oder 2-Trichlormethyl-2-methylethoxycarbonyl;
- (C₁-C₆-Alkyl)carbonyl sowie der Alkylcarbonyl-Teil von (C₁-C₆-Alkyl)carbonyl-C₁-C₆-alkyl für: Methylcarbonyl, Ethylcarbonyl, n-Propylcarbonyl, 1-Methylethylcarbonyl, n-Butylcarbonyl, 1-Methyl-propylcarbonyl, 2-Methylpropylcarbonyl, 1,1-Dimethylethylcarbonyl, n-Pentylcarbonyl, 1-Methylbutylcarbonyl, 2-Methylbutylcarbonyl, 3-Methylbutylcarbonyl, 1,1-Dimethylpropylcarbonyl, 1,2-Dimethylpropylcarbonyl, 2,2-Dimethylpropylcarbonyl, 1-Ethylpropylcarbonyl, n-Hexylcarbonyl, 1-Methylpentylcarbonyl, 2-Methylpentylcarbonyl, 3-Methylpentylcarbonyl, 4-Methylpentylcarbonyl, 1,1-Dimethylbutylcarbonyl, 1,2-Dimethylbutylcarbonyl, 1,3-Dimethylbutylcarbonyl, 2,2-Dimethylbutylcarbonyl, 2,3-Dimethylbutylcarbonyl, 3,3-Dimethylbutylcarbonyl, 1-Ethylbutylcarbonyl, 2-Ethylbutylcarbonyl, 1,1,2-Trimethylpropoylcarbonyl, 1,2,2-Trimethylpropylcarbonyl, 1-Ethyl-1-methylpropylcarbonyl oder 1-Ethyl-2-methylpropylcarbonyl;
- (C₁-C₆-Halogenalkyl)carbonyl für: (C₁-C₆-Alkyl)carbonyl wie voranstehend genannt, das partiell oder vollsltändig durch Fluor, Chlor und/oder Brom substituiert ist, also z.B. Chloracetyl, Dichloracetyl, Trichloracetyl, Fluoracetyl, Difluoracetyl, Trifluoracetyl, Chlorfluoracetyl, Dichlorfluoractyl, Chlordifluoracetyl, 1-Fluorpropionyl, 2-Fluorpropionyl, 2,2-Difluorpropionyl, 3,3,3-Trifluorpropionyl, 3-Chlor-3-fluorpropionyl, 3-Chlor-3,3-difluorpropionyl, 3,3-Dichlor-3-fluorpropionyl, Tri-chlorpropionyl oder Pentafluorpropionyl;
- Aminocarbonyl-(C₁-C₆-alkyl)carbonyl für: Aminocarbonylmethylcarbonyl, 1-(Aminocarbonyl)eth-1-ylcarbonyl, 2-(Aminocarbonyl)eth-1-ylcarbonyl, 1-(Aminocarbonyl)prop-1-ylcarbonyl, 2-(Aminocarbonyl)prop-1-ylcarbonyl, 3-(Aminocarbonyl)prop-1-ylcarbonyl, 1-(Aminocarbonyl)prop-2-ylcarbonyl, 2-(Aminocarbonyl)prop-2-ylcarbonyl, 1-(Aminocarbonyl)but-1-ylcarbonyl, 2-(Aminocarbonyl)but-1-ylcarbonyl, 3-(Aminocarbonyl)but-1-ylcarbonyl, 4-(Aminocarbonyl)but-1-ylcarbonyl, 1-(Aminocarbonyl)but-2-ylcarbonyl, 2-(Aminocarbonyl)but-2-ylcarbonyl, 1-(Aminocarbonyl)but-3-ylcarbonyl, 2-(Aminocarbonyl)but-3-ylcarbonyl, 1-(Aminocarbonyl)-2-methyl-prop-3-ylcarbonyl, 2-(Aminocarbonyl)-2-methyl-prop-3-ylcarbonyl, 3-(Aminocarbonyl)-2-methyl-prop-3-ylcarbonyl, 2-(Aminocarbonylmethyl)-prop-2-ylcarbonyl, 1-(Aminocarbonyl)pent-1-ylcarbonyl, 2-(Aminocarbonyl) pent-1-ylcarbonyl, 3-(Aminocarbonyl)pent-1-ylcarbonyl, 4-(Aminocarbonyl)pent-1-ylcarbonyl, 5-(Aminocarbonyl) pent-1-ylcarbonyl, 1-(Aminocarbonyl)pent-2-ylcarbonyl, 2-(Aminocarbonyl)pent-2-ylcarbonyl, 1-(Aminocarbonyl) pent-3-ylcarbonyl, 2-(Aminocarbonyl)pent-3-ylcarbonyl, 1-(Aminocarbonyl)pent-4-ylcarbonyl, 2-(Aminocarbonyl) pent-4-ylcarbonyl, 3-(Aminocarbonyl)pent-4-ylcarbonyl, 1-(Aminocarbonyl)-2-ethyl-prop-3-ylcarbonyl, 1-(Aminocarbonyl)hex-1-ylcarbonyl, 2-(Aminocarbonyl)hex-1-ylcarbonyl, 3-(Aminocarbonyl)hex-1-ylcarbonyl, 4-(Aminocarbonyl)hex-1-ylcarbonyl, 5-(Aminocarbonyl)hex-1-ylcarbonyl, 6-(Aminocarbonyl)hex-1-ylcarbonyl, 1-(Aminocarbonyl)hex-2-ylcarbonyl, 2-(Aminocarbonyl)hex-2-ylcarbonyl, 1-(Aminocarbonyl)hex-3-ylcarbonyl, 2-(Aminocarbonyl)hex-3-ylcarbonyl, 1-(Aminocarbonyl)hex-4-ylcarbonyl, 2-(Aminocarbonyl)hex-4-ylcarbonyl, 3-(Aminocarbonyl)hex-4-ylcarbonyl, 1-(Aminocarbonyl)-2-ethyl-but-3-ylcarbonyl, 1-(Aminocarbonyl)-2-ethyl-but-4-yl oder 1-(Aminocarbonyl)-2-propyl-prop-3-ylcarbonyl;
- C₁-C₄-Alkylimino für: Methylimino, Ethylimino, n-Propylimino, 1-Methylethylimino, n-Butylimino, 1-Methyl-propylimino, 2-Methylpropylimino oder 1,1-Dimethylethylimino;
- der Alkoxyimino-Teil von C₁-C₆-Alkoxyimino-C₁-C₆-alkyl für: Methoxyimino, Ethoxyimino, 1-Pro-poxyimino, 2-Propoxyimino, 1-Methylethoxyimino, n-Butoxyimino, sec.-Butoxyimino, tert.-Butoxyimino, 1-Methyl-1-propoxyimino, 2-Methyl-1-propoxyimino, 1-Methyl-2-propoxyimino, 2-Methyl-2-propoxyimino, n-Pentoxyimino, 2-Pentoxyimino, 3-Pentoxyimino, 4-Pentoxyimino, 1-Methyl-1-butoxyimino, 2-Methyl-1-butoxyimino, 3-Methyl-1-butoxyimino, 1-Methyl-2-butoxyimino, 2-Methyl-2-butoxyimino, 3-Methyl-2-butoxyimino, 1-Methyl-3-butoxyimino, 2-Methyl-3-butoxyimino, 3-Methyl-3-butoxyimino, 1,1-Dimethyl-2-propoxyimino, 1,2-Dimethyl-1-propoxyimino, 1,2-Dimethyl-2-propoxyimino, 1-Ethyl-1-propoxyimino, 1-Ethyl-2-propoxyimino, n-Hexoxyimino, 2-Hexoxyimino, 3-Hexoxyimino, 4-Hexoxyimino, 5-Hexoxyimino, 1-Methyl-1-pentoxyimino, 2-Methyl-1-pentoxyimino, 3-Methyl-1-pentoxyimino, 4-Methyl-1-pentoxyimino, 1-Methyl-2-pentoxyimino, 2-Methyl-2-pentoxyimino, 3-Methyl-2-pentoxyimino, 4-Methyl-2-pentoxyimino, 1-Methyl-3-pentoxyimino, 2-Methyl-3-pentoxyimino, 3-Methyl-3-pentoxyimino, 4-Methyl-3-pentoxyimino, 1-Methyl-4-pentoxyimino, 2-Methyl-4-pentoxyimino, 3-Methyl-4-pentoxyimino, 4-Methyl-4-pentoxyimino, 1,1-Dimethyl-2-butoxyimino, 1,1-Dimethyl-3-butoxyimino, 1,2-Dimethyl-1-butoxyimino, 1,2-Dimethyl-2-butoxyimino, 1,2-Dimethyl-3-butoxyimino, 1,3-Dimethyl-1-butoxyimino, 1,3-Dimethyl-2-butoxyimino, 1,3-Dimethyl-3-butoxyimino, 2,2-Dimethyl-3-butoxyimino, 2,3-Dimethyl-1-butoxyimino, 2,3-Dimethyl-2-butoxyimino, 2,3-Dimethyl-3-butoxyimino, 3,3-Dimethyl-1-butoxyimino, 3,3-Dimethyl-2-butoxyimino, 1-Ethyl-1-butoxyimino, 1-Ethyl-2-butenoxyimino, 1-Ethyl-3-butoxyimino, 2-Ethyl-1-butoxyimino, 2-Ethyl-2-butoxyimino, 2-Ethyl-3-butoxyimino, 1,1,2-Trimethyl-2-propoxyimino, 1-Ethyl-1-methyl-2-propoxyimino, 1-Ethyl-2-methyl-1-propoxyimino oder 1-Ethyl-2-methyl-2-propoxyimino;
- (C₁-C₆-Alkylamino)carbonyl sowie der Alkylaminocarbonyl-Teil von (C₁-C₆-Alkylamino)carbonyl-C₁-C₆-alkyl für: z. B. Methylaminocarbonyl, Ethylaminocarbonyl, n-Propylaminocarbonyl, 1-Methylethyl-aminocarbonyl, n-Butylaminocarbonylmethyl, 1-Methylpropyl-aminocarbonyl, 2-Methylpropylaminocarbonyl, 1,1-Dimethyl-ethylaminocarbonyl, n-Pentylaminocarbonyl, 1-Methyl-butylaminocarbonyl, 2-Methylbutylaminocarbonyl, 3-Methyl-butylaminocarbonyl, 1,1-Dimethylpropylaminocarbonyl, 2-Di-methylpropylaminocarbonyl, 2-Dimethylpropylaminocarbonyl, 1-Ethylpropylaminocarbonyl, n-Hexylaminocarbonyl, 1-Methyl-pentylaminocarbonyl, 2-Methylpentylaminocarbonyl, 3-Methyl-pentylaminocarbonyl, 1,2-Dimethylbutylaminocarbonyl, 1,3-Dimethylbutylaminocarbonyl, 2,2-Dimethylbutylaminocarbonyl, 2,3-Dimethylbutylaminocarbonyl, 3,3-Dimethylbutylaminocarbonyl, 1-Ethylbutylaminocarbonyl, 2-Ethylbutylaminocarbonyl, 1,1,2-Trimethylpropylaminocarbonyl, 1,2,2-Trimethylpropylaminocarbonyl, 1-Ethyl-1-methylpropylcarbonyl oder 1-Ethyl-2-methylpropylaminocarbonyl;
- Di(C₁-C₆-alkyl)aminocarbonyl sowie der Dialkylaminocarbonyl-Teil von Di(C₁-C₆-alkyl)aminocarbonyl-C₁-C₆-alkyl für: z. B. N,N-Dimethyl-aminocarbonylmethyl, N,N-Diethylaminocarbonyl, N,N-Diisopropylaminocarbonyl, N,N-Dibutylaminocarbonyl, N,N-Di-(1-methylpropyl)aminocarbonyl, N,N-Di-(2-Methylpropyl)-aminocarbonyl, N,N-Di-(1,1-Dimethylethyl)aminocarbonyl, N-Ethyl-N-Methylaminocarbonyl, N-Methyl-N-propylaminocarbonyl, N-Methyl-N-(1-Methylethyl)aminocar-bonyl, N-Butyl-N-methylaminocarbonyl, N-Methyl-N-(1-methylpropyl) amino-carbonyl, N-Methyl-N-(2-methylproyl) aminocarbonyl, N-(1,1-Dimethylethyl)-N-methylaminocar-bonyl, N-Ethyl-N-propylaminocarbonyl, N-Ethyl-N-(1-methylethyl)aminocarbonyl, N-Butyl-N-ethylaminocarbonyl, N-Ethyl-N-(1-methylpropyl)aminocarbonyl, N-Ethyl-N-(2-methylpropyl)aminocarbonyl, N-Ethyl-N-(1,1-dimethylethyl) aminocarbonyl, N-(1-Methyletyl)-N-propylaminocarbonyl, N-Butyl-N-propylaminocarbonyl, N-(1-Methylpropyl)-N-propylaminocarbonyl, N-(2-Methylpropyl)-N-propylaminocar-bonyl, N-(1,1-Dimethylethyl)-N-propylaminocarbonyl, N-Butyl-N-(1-methylethyl)aminocarbonyl, N-(1-Methylethyl)-N-(1-methylpropyl)aminocarbonyl, N-(1-Methylethyl)-N-(2-methylpropyl)aminocarbonyl, N-(1,1,-Dimethylethyl)-N-(1-methylethyl)aminocarbonyl, N-Butyl-N-(1-methylpropyl)amino-carbonyl, N-Butyl-N-(2-methylpropyl)aminocarbonyl, N-Butyl-N-(1,1-dimethylethyl)aminocarbonyl, N-(1-Methylpro-pyl)-N-(2-methylpropyl)aminocarbonyl, N-(1,1-Dimethyl-ethyl)-N-(1-methylpropyl)aminocarbonyl oder N-(1,1-Dimethyl-ethyl)-N-(2-methylpropyl)aminocarbonyl.

Unter Aryl ist insbesondere Phenyl oder Naphtyl zu verstehen.

Unter Heteroaryl ist vorzugsweise ein 5- oder 6-gliedriger aromatischer Heterocyclus, der gewünschtenfalls einen anellierten Benzolring tragen kann, zu verstehen. Besonders bevorzugte Heteroaromaten sind 2-Furyl, 3-Furyl, 2-Thienyl, 3-Thienyl, 2-Pyrrolyl, 3-Pyrrolyl, 3-Isoxazolyl, 4-Isoxazolyl, 5-Isoxazolyl, 3-Isothiazolyl, 4-Isothiazolyl, 5-Isothiazolyl, 3-Pyrazolyl, 4-Pyrazolyl, 5-Pyrazolyl, 2-Oxazolyl, 4-Oxazolyl, 5-Oxazolyl, 2-Thiazolyl, 4-Thiazolyl, 5-Thiazolyl, 2-Imidazolyl, 4-Imidazolyl, 1,2,4-Oxadiazol-3-yl, 1,2,4-Oxadiazol-5-yl, 1,2,4-Thiadiazol-3-yl, 1,2,4-Thiadiazol-5-yl, 1,2,4-Triazol-3-yl, 1,3,4-Oxadiazol-2-yl, 1,3,4-Thiadiazol-2-yl, 1,3,4-Triazol-2-yl, 1,2,3,4-Tetrazol-5-yl, 2-Pyridyl, 3-Pyridyl, 4-Pyridyl, 3-Pyridazinyl, 4-Pyridazinyl, 2-Pyrimidinyl, 4-Pyrimidinyl, 5-Pyrimidinyl, 2-Pyrazinyl, 1,3,5-Triazin-2-yl und 1,2,4-Triazin-3-yl.

Im Hinblick auf die Verwendung der erfindungsgemäßen Verbindungen der Formel I als Herbizide und/oder als defoliant/desiccant wirksame Verbindungen haben die Variablen vorzugsweise folgende Bedeutungen, und war jeweils für sich allein oder in Kombination:
R¹ Wasserstoff oder 4-Methyl;
R² Wasserstoff, Fluor oder Chlor;
R³ einen Substituenten aus der Gruppe 3.01 - 3.48 (Tabelle 1):

**Tabelle 1**

| Nr. | R³ | Nr. | R³ | Nr. | R³ |
|---|---|---|---|---|---|
| 3.01 | F | 3.19 | CH₂Br | 3.37 | O-sec-C₄H₉ |
| 3.02 | Cl | 3.20 | CHBr₂ | 3.38 | O-tert.-C₄H₉ |
| 3.03 | Br | 3.21 | CH₂CH₂F | 3.39 | O-CHF₂ |
| 3.04 | I | 3.22 | CH₂CHF₂ | 3.40 | O-CF₃ |
| 3.05 | CH₃ | 3.23 | CH₂CF₃ | 3.41 | O-CH₂CH₂F |
| 3.06 | C₂H₅ | 3.24 | CHFCH₃ | 3.42 | O-CH₂CHF₂ |
| 3.07 | n-C₃H₇ | 3.25 | CF₂CH₃ | 3.43 | O-CH₂CF₃ |
| 3.08 | i-C₃H₇ | 3.26 | CF₂CF₃ | 3.44 | O-CF₂CF₃ |
| 3.09 | n-C₄H₉ | 3.27 | CH₂CH₂Cl | 3.45 | O-CH₂CH₂Cl |
| 3.10 | i-C₄H₉ | 3.28 | CH(Cl)CH₃ | 3.46 | O-CH₂CH₂Br |
| 3.11 | sec-C₄H₉ | 3.29 | CH₂CH₂Br | 3.47 | CN |
| 3.12 | t-C₄H₉ | 3.30 | C(Br)CH₃ | 3.48 | NO2 |
| 3.13 | CH₂F | 3.31 | OCH₃ | | |
| 3.14 | CHF₂ | 3.32 | OCH₂CH₃ | | |
| 3.15 | CF₃ | 3.33 | O-n-C₃H₇ | | |
| 3.16 | CH₂Cl | 3.34 | O-i-C₃H₇ | | |
| 3.17 | CHCl₂ | 3.35 | O-n-C₄H₉ | | |
| 3.18 | CCl₃ | 3.38 | O-i-C₄H₉ | | |

besonders bevorzugt sind die Reste 3.01 - 3.05, 3.15, 3.31, 3.39, 3.40, 3.47 oder 3.48, insbesondere 3.01 - 3.04 und 3.47;
R⁴ Wasserstoff, Cyano, Nitro, Fluor, Chlor, Brom, Iod, Methyl, Etyl, n-Propyl oder 1-Methylethyl, insbesondere Wasserstoff, Cyano, Fluor, Chlor, Brom oder Methyl;
R⁵ einen Substituenten aus der Gruppe 5.01 - 5.51 (Tabelle 2):

**Tabelle 2**

| Nr. | R⁵ | | Nr. | R⁵ |
|---|---|---|---|---|
| 5.01 | H | | 5.27 | CHCl₂ |
| 5.02 | CH₃ | | 5.28 | CHBr₂ |
| 5.03 | C₂H₅ | | 5.29 | CF₃ |
| 5.04 | n-C₃H₇ | | 5.30 | CH(F)CH₃ |
| 5.05 | i-C₃H₇ | | 5.31 | CH(Cl)CH₃ |
| 5.06 | n-C₄H₉ | | 5.32 | CH(Br)CH₃ |
| 5.07 | i-C₄H₉ | | 5.33 | CH(I)CH₃ |
| 5.08 | sec-C₄H₉ | | 5.34 | CH₂CN |
| 5.09 | tert.-C₄H₉ | | 5.35 | CH(CH₃)CN |
| 5.10 | CH=CH₂ | | 5.36 | CH₂CH₂CN |
| 5.11 | CH₂-CH=CH₂ | | 5.37 | CH₂OH |
| 5.12 | CH₂-CH=CH-CH₃ | | 5.38 | CH(CH₃)OH |
| 5.13 | CH=CH₂-CH₃ | | 5.39 | CH₂OCH₃ |
| 5.14 | C(CH₃)=CH₂ | | 5.40 | CH₂OC₂H₅ |
| 5.15 | CH=C(CH₃)₂ | | 5.41 | CH(CH₃)OCH₃ |
| 5.16 | C(CH₃)=CH(CH₃) | | 5.42 | CH(CH₃)OC₂H₅ |
| 5.17 | CH₂-CH=C(CH₃)₂ | | 5.43 | CH₂SH |
| 5.18 | -C≡CH | | 5.44 | CH(CH₃)SH |
| 5.19 | CH₂-C≡CH | | 5.45 | CH₂CH₂SH |
| 5.20 | C≡C-CH₃ | | 5.46 | CH₂SCH₃ |
| 5.21 | CH₂-C≡C-CH₃ | | 5.47 | CH₂SC₂H₅ |
| 5.22 | CH₂F | | 5.48 | CH(CH₃)SCH₃ |
| 5.23 | CH₂Cl | | 5.49 | CH(CH₃)SC₂H₅ |
| 5.24 | CH₂Br | | 5.50 | CH₂CH₂SCH₃ |
| 5.25 | CH₂I | | 5.51 | CH₂CH₂SC₂H₅ |
| 5.26 | CHF₂ | | | |

R⁶ einen Substituenten aus der Gruppe 6.01 - 6.147 (Tabelle 3):

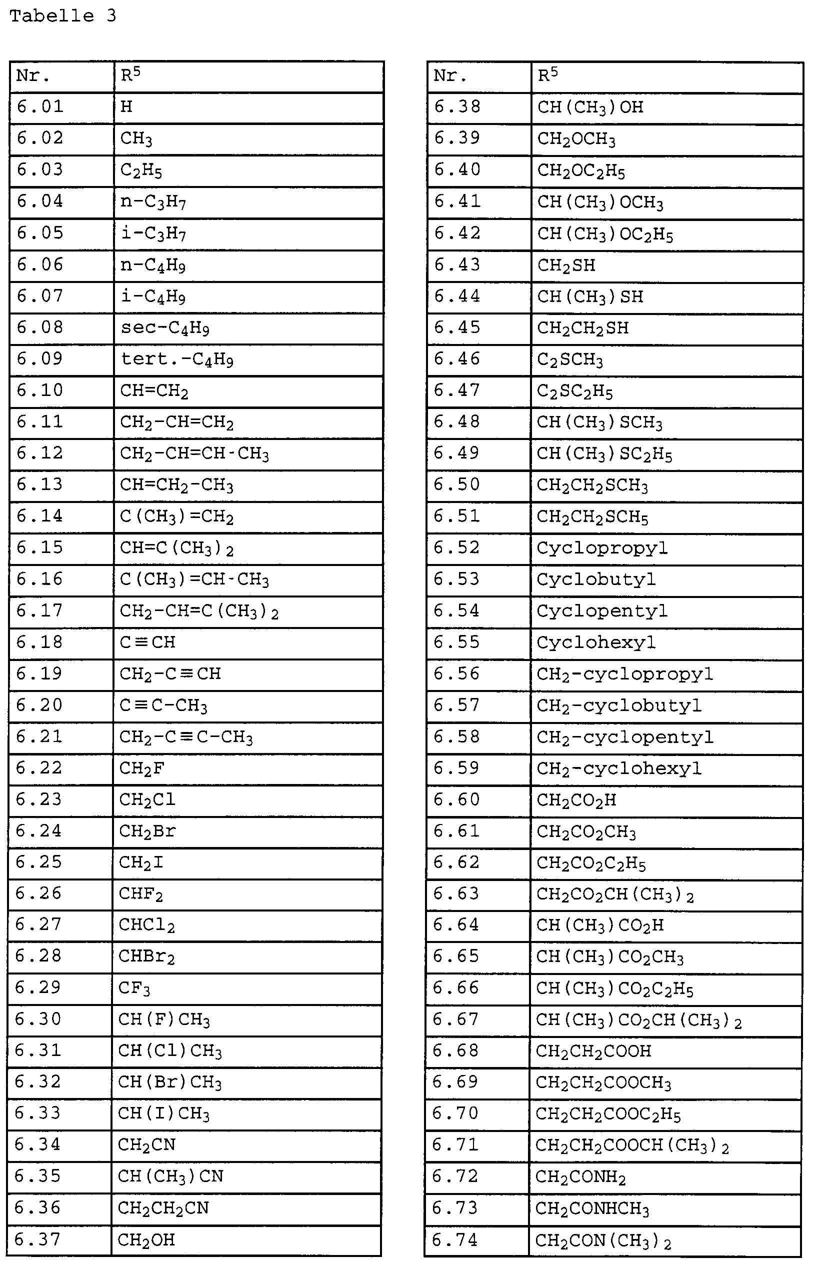

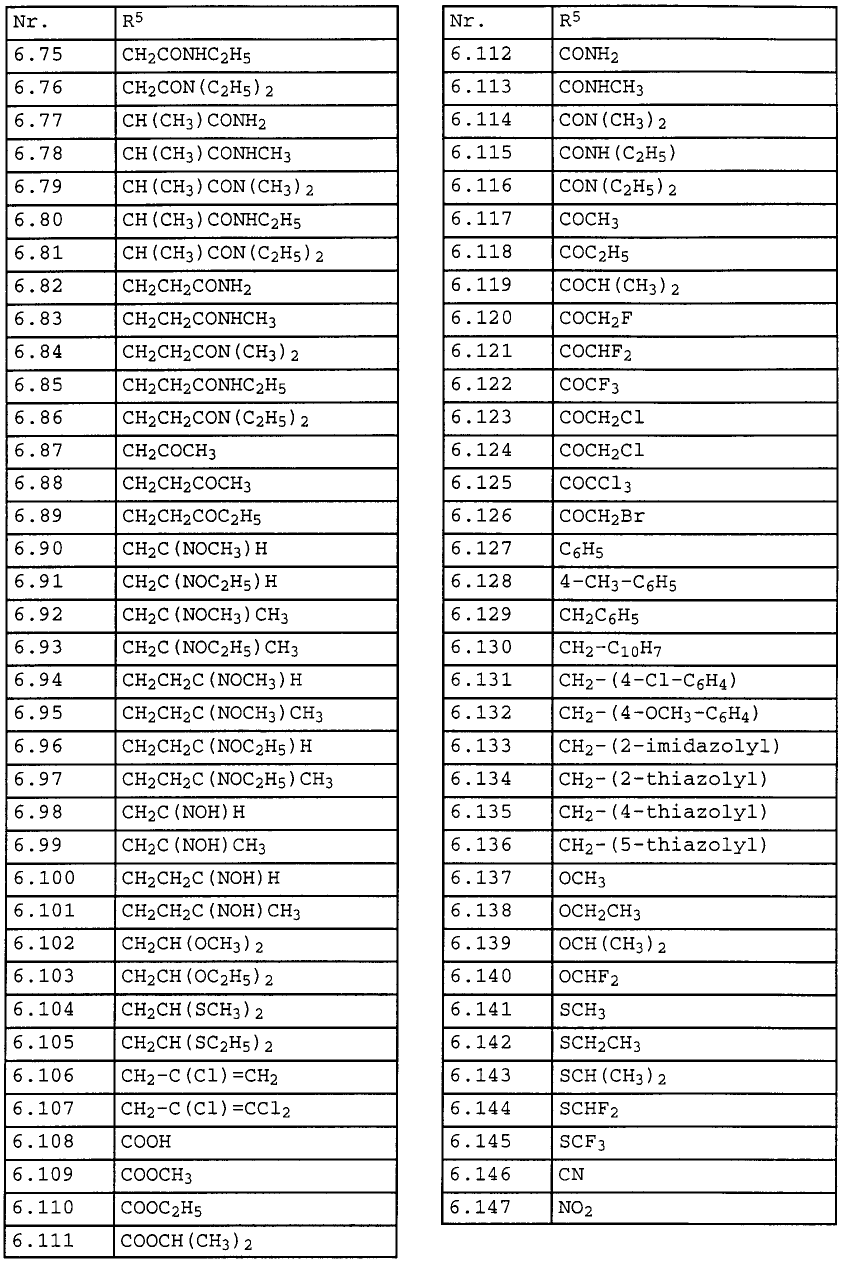

oder R⁵ und R⁶ zusammen einen Substituenten aus der Gruppe (5+6).01 bis (5+6).10 (Tabelle 4):

**Tabelle 4**

| Nr. | R⁵ + R⁶ |
|---|---|
| (5+6).01 | -(CH₂)₂- |
| (5+6).02 | -(CH₂)₃- |
| (5+6).03 | -(CH₂)₄- |
| (5+6).04 | -(CH₂)₅- |
| (5+6).06 | -(CH₂)₆- |
| (5+6).06 | -CH₂-O- |
| (5+6).07 | -CH₂-O-CH₂=CH₂- |
| (5+6).08 | -CH₂-CH₂-O-CH₂-CH₂- |
| (5+6).09 | -CH₂-CH₂-NH-CH₂-CH₂- |
| (5+6).10 | -CH₂-CH₂-N(CH₃)-CH₂-CH₂- |

R⁷ einen Substituenten aus der Gruppe 7.01 - 7.60 (Tabelle 5):

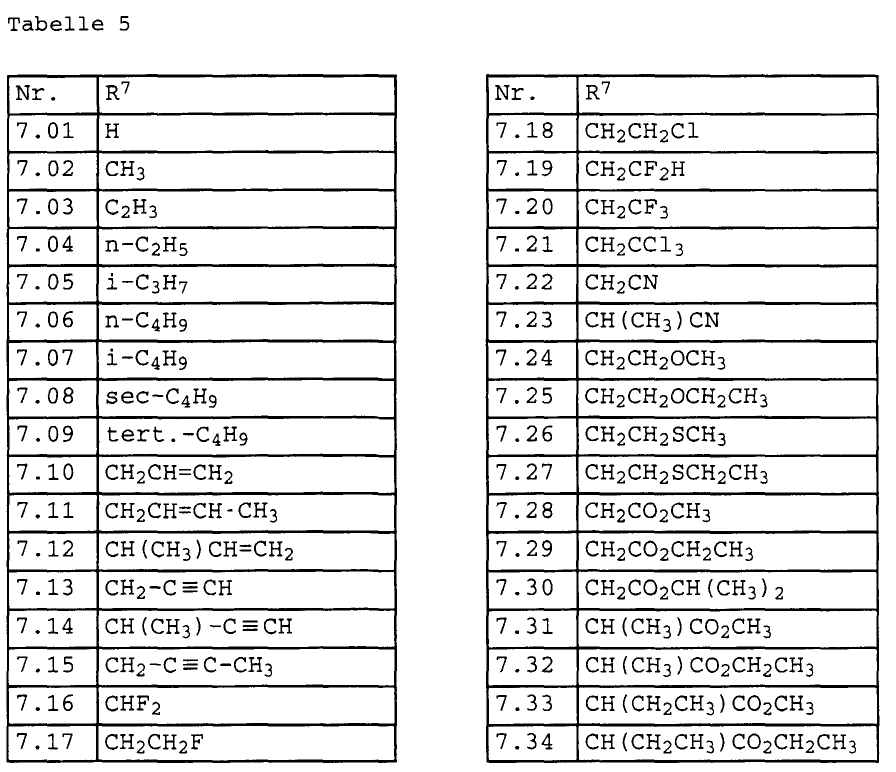

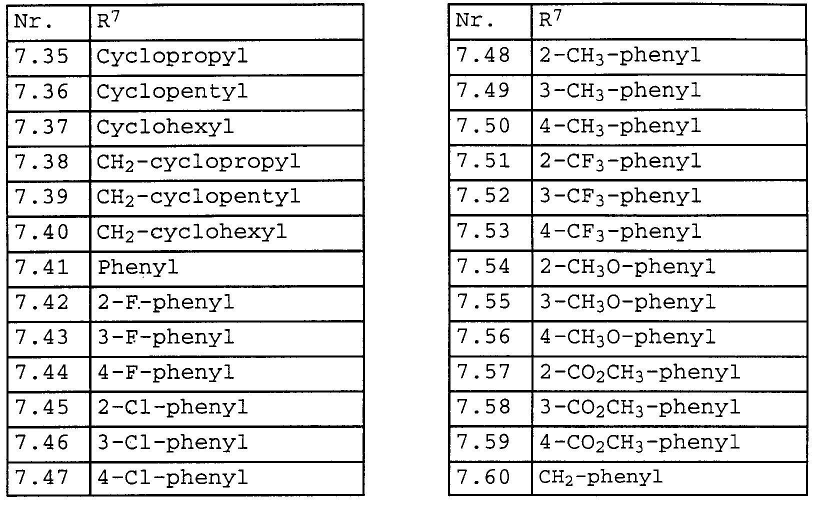

X Sauerstoff oder -N(R⁸)-;
R⁸ einen Substituenten aus der Gruppe 8.01 - 8.40 (Tabelle 6)

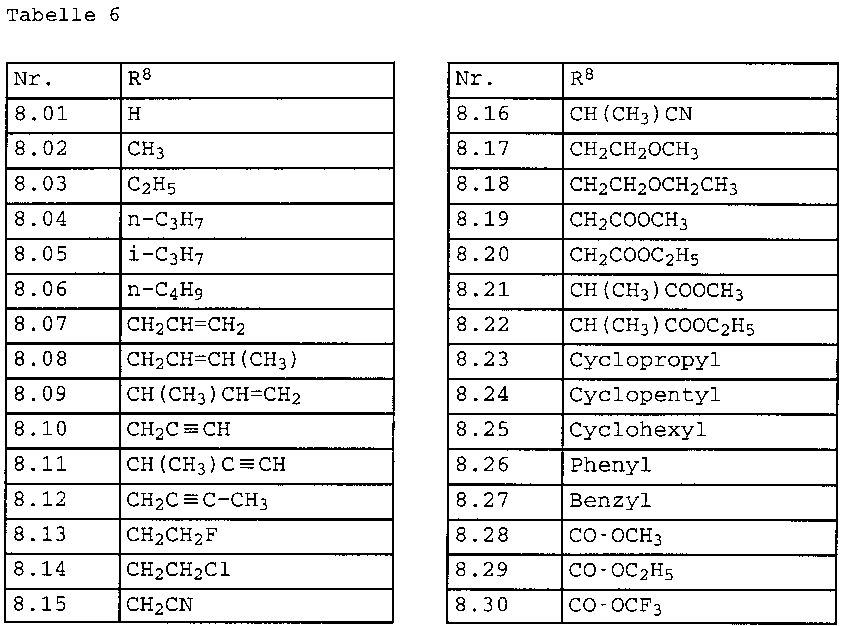

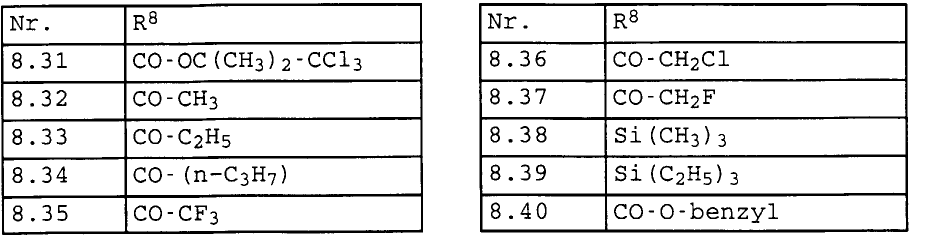

oder R⁶ und R⁸ zusammen eine der folgenden Ketten: -CH₂-CH₂-CH₂-, -CH₂-CH₂-CH₂-CH₂-, -CO-CH₂-CH₂-, -CO-CH₂-CH₂-CH₂-, -CH₂-CH₂-O-CH₂-, -CH₂-CH₂-NH-CH₂- oder -CH₂-CH₂-N(CH₃)-CH₂-.

Im Hinblick auf die Verwendung der substituierten Phthalimido-Zimtsäurederivate I als Herbizide sind die in Tabelle 7 aufgeführten Verbindungen Ia (= I mit R³ = Chlor; R¹, R⁵ und R⁶ = Wasserstoff; X = Sauerstoff) ganz besonders bevorzugt:

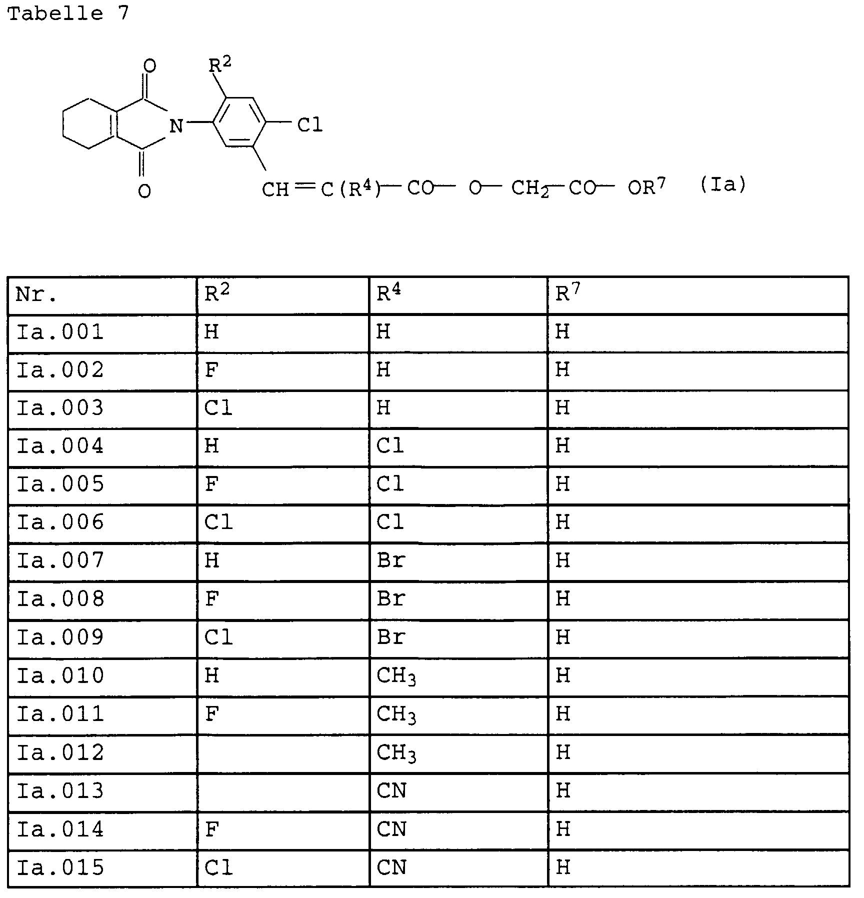

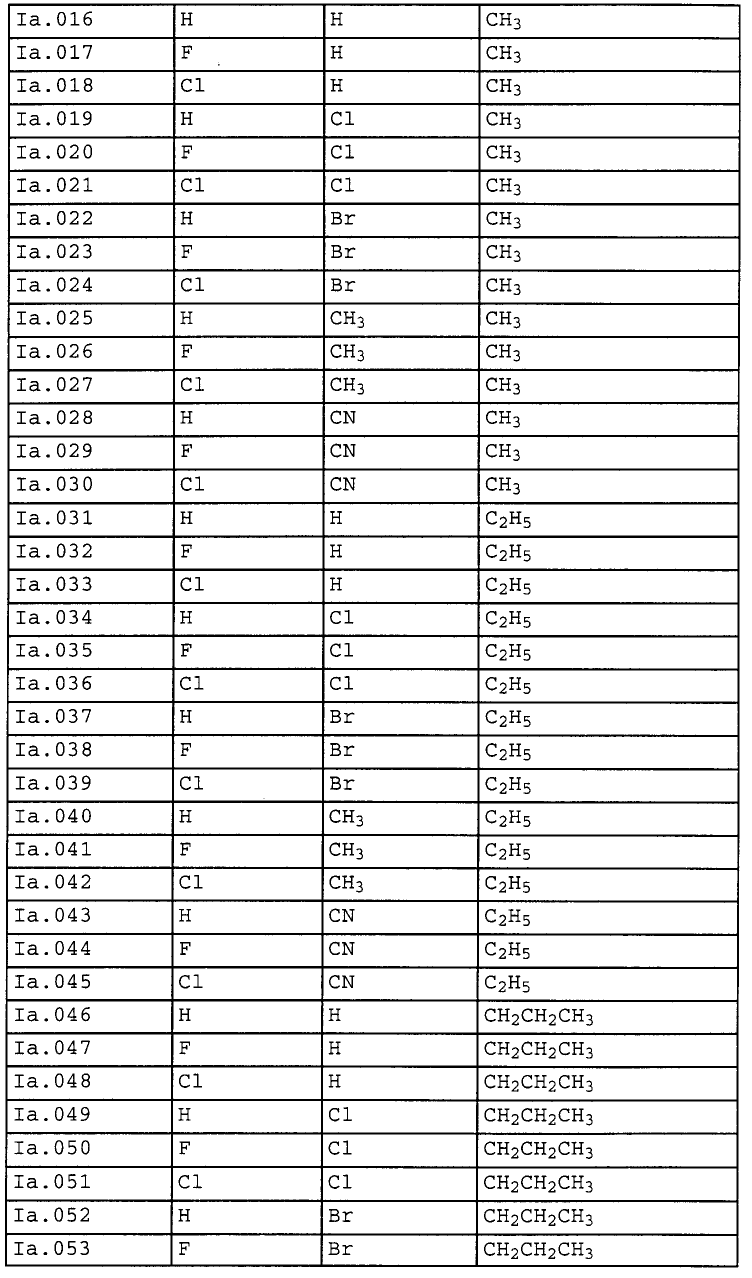

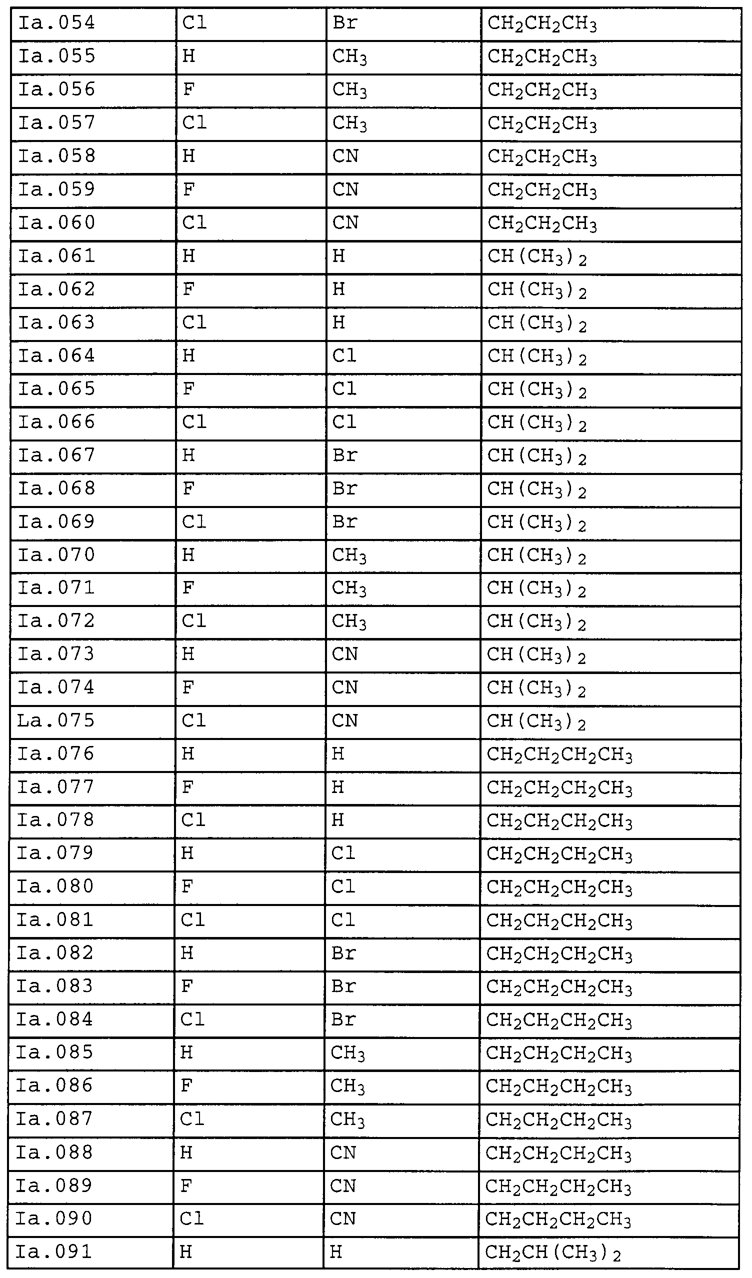

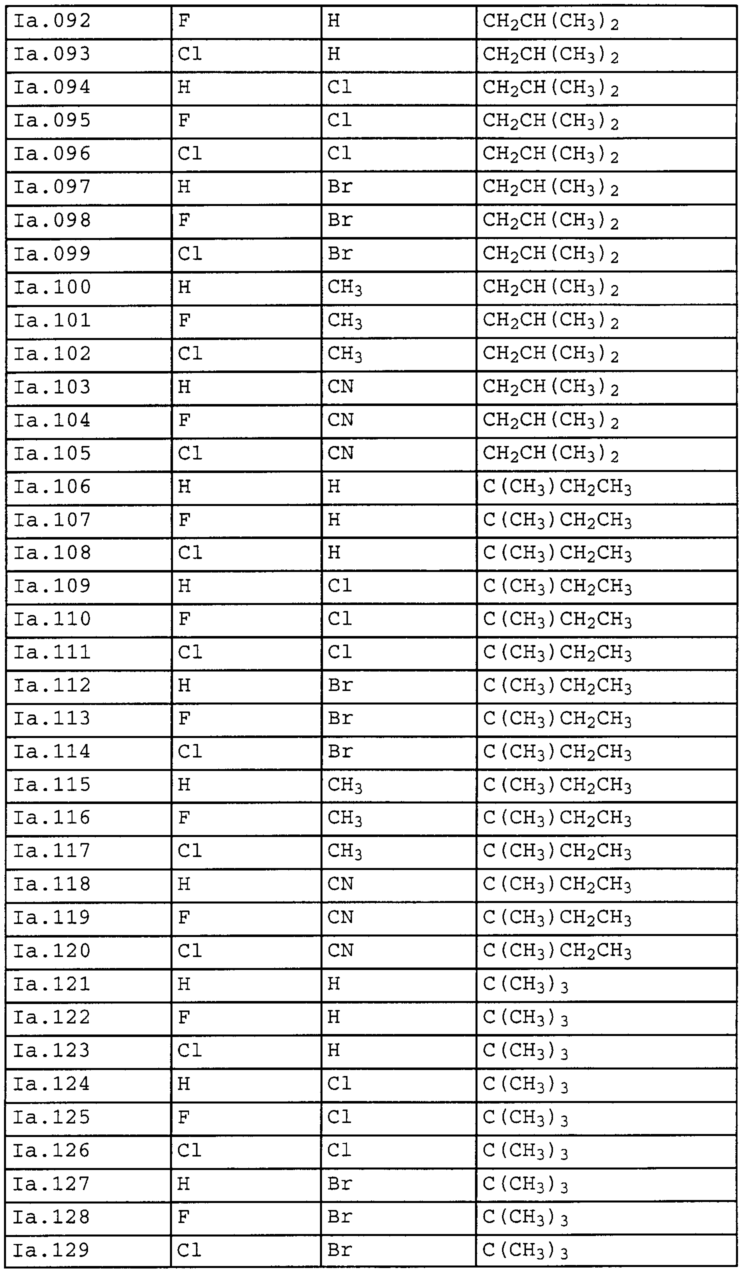

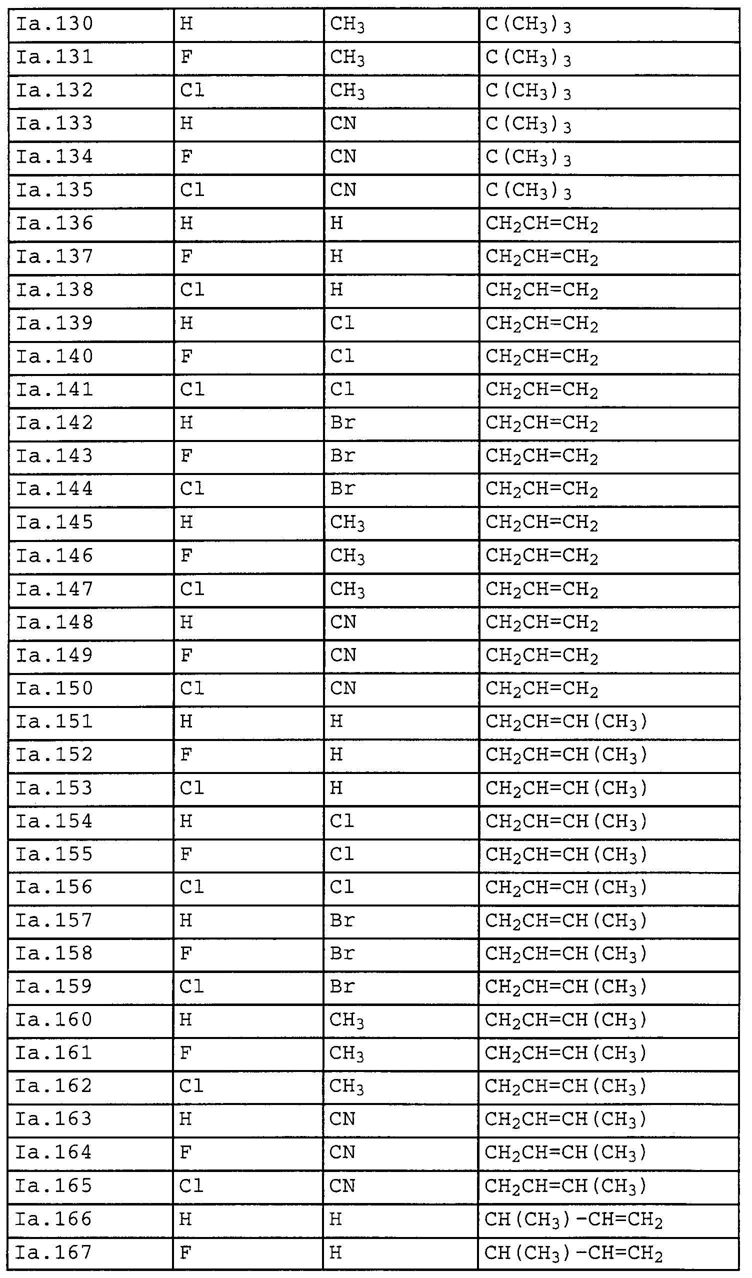

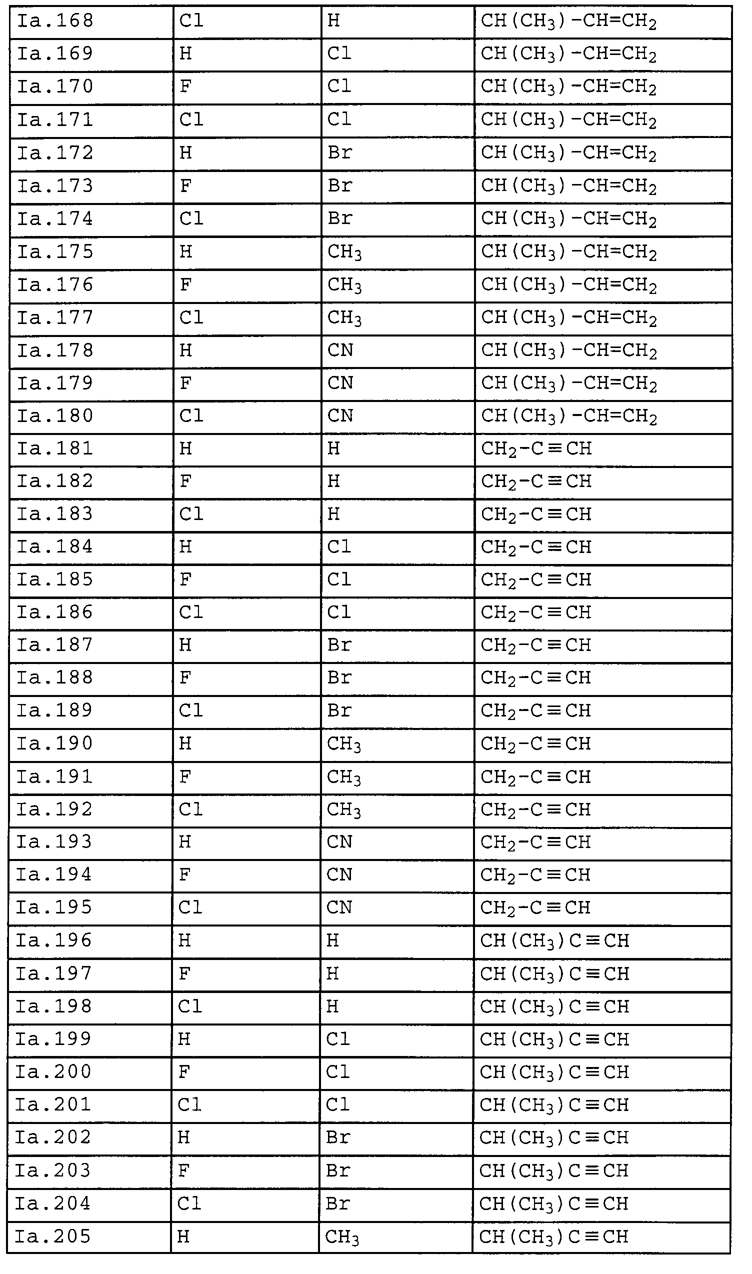

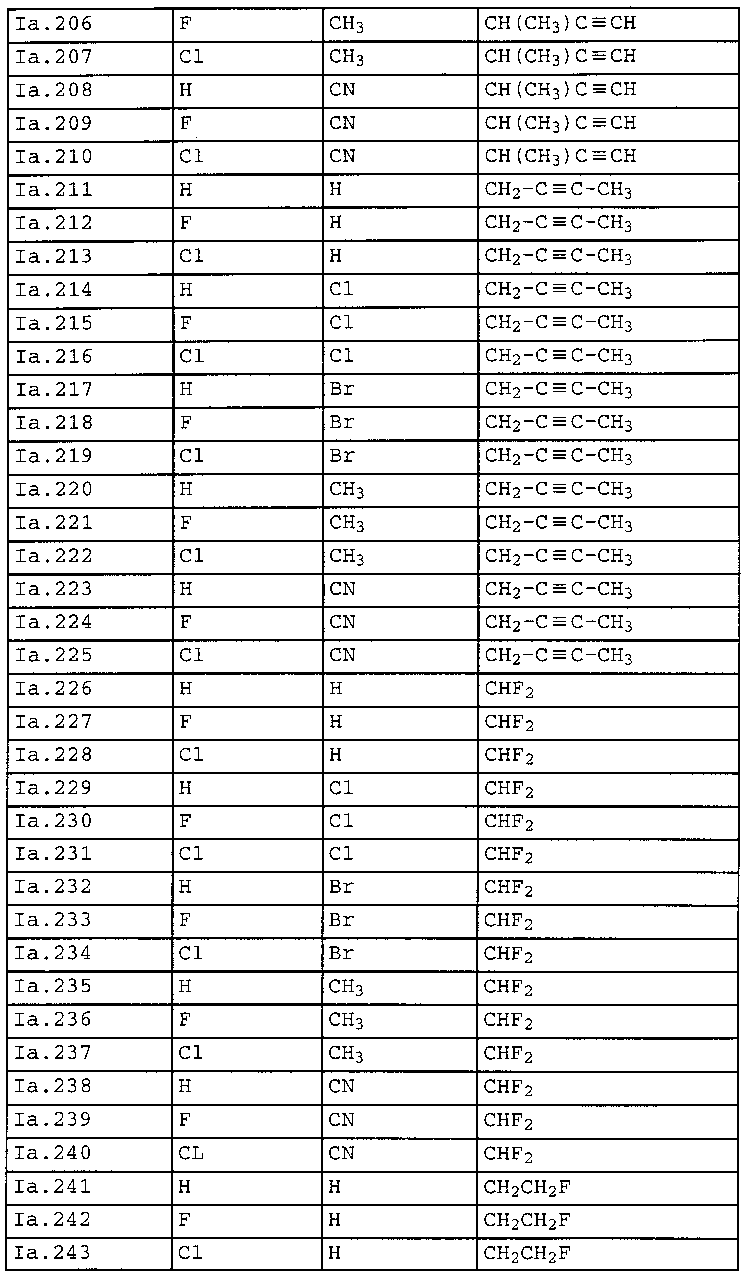

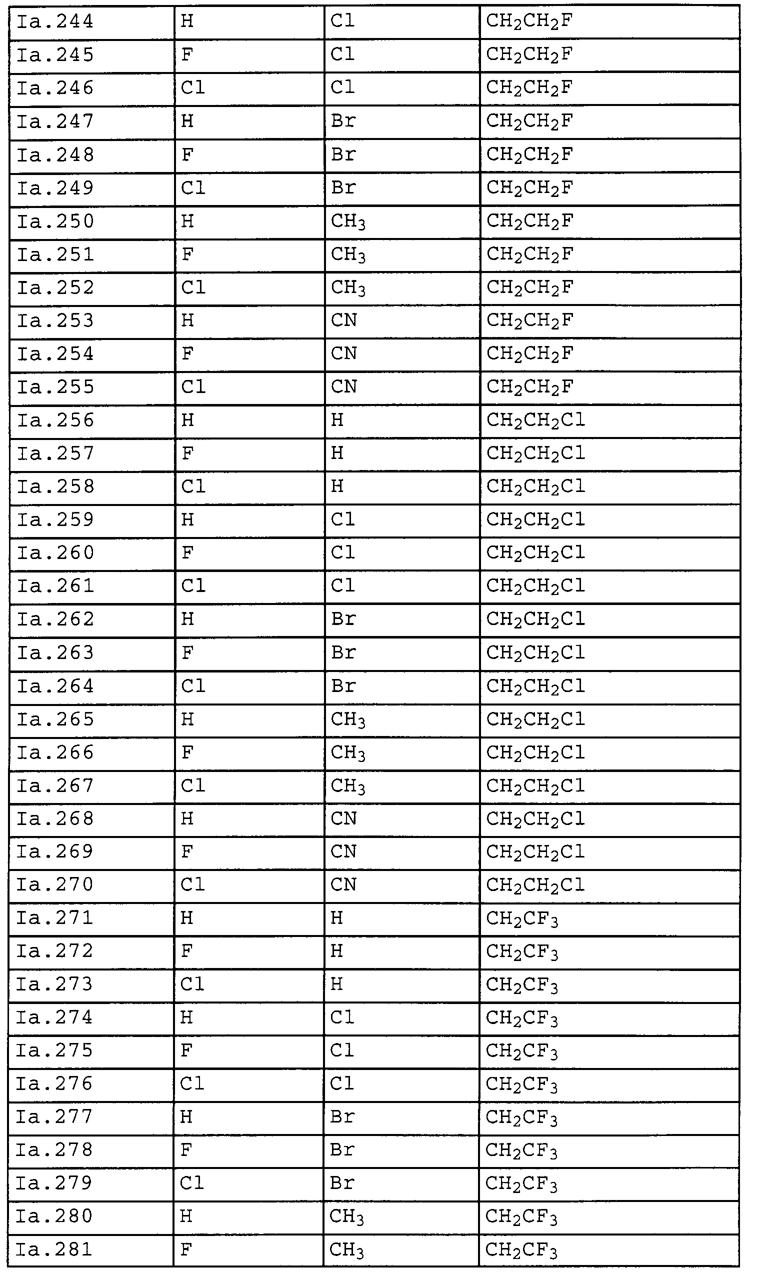

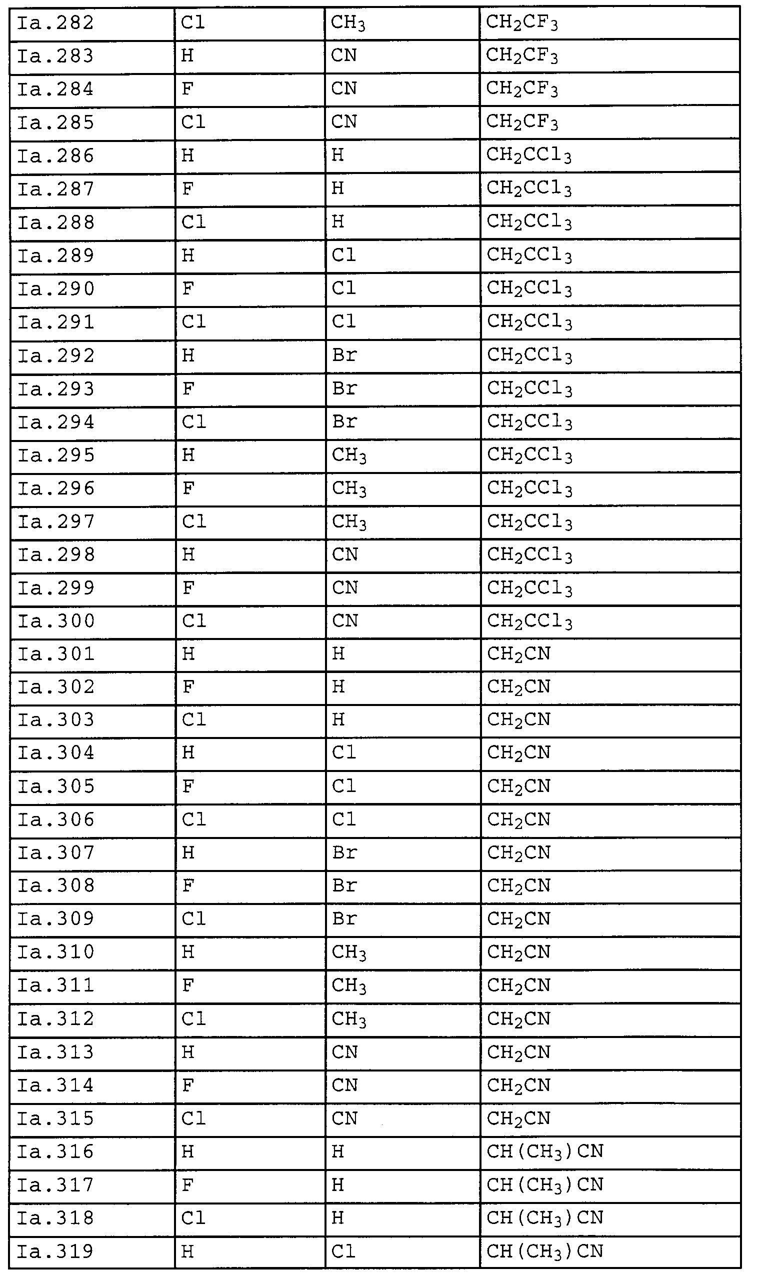

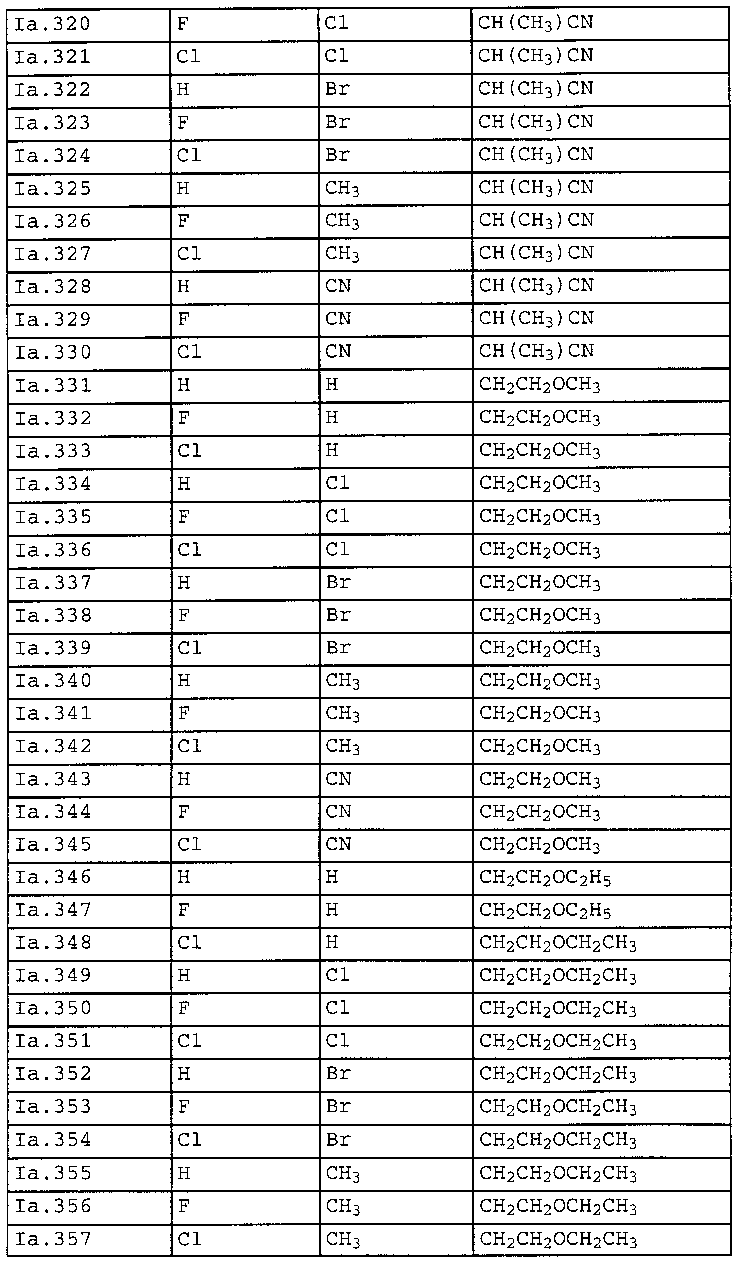

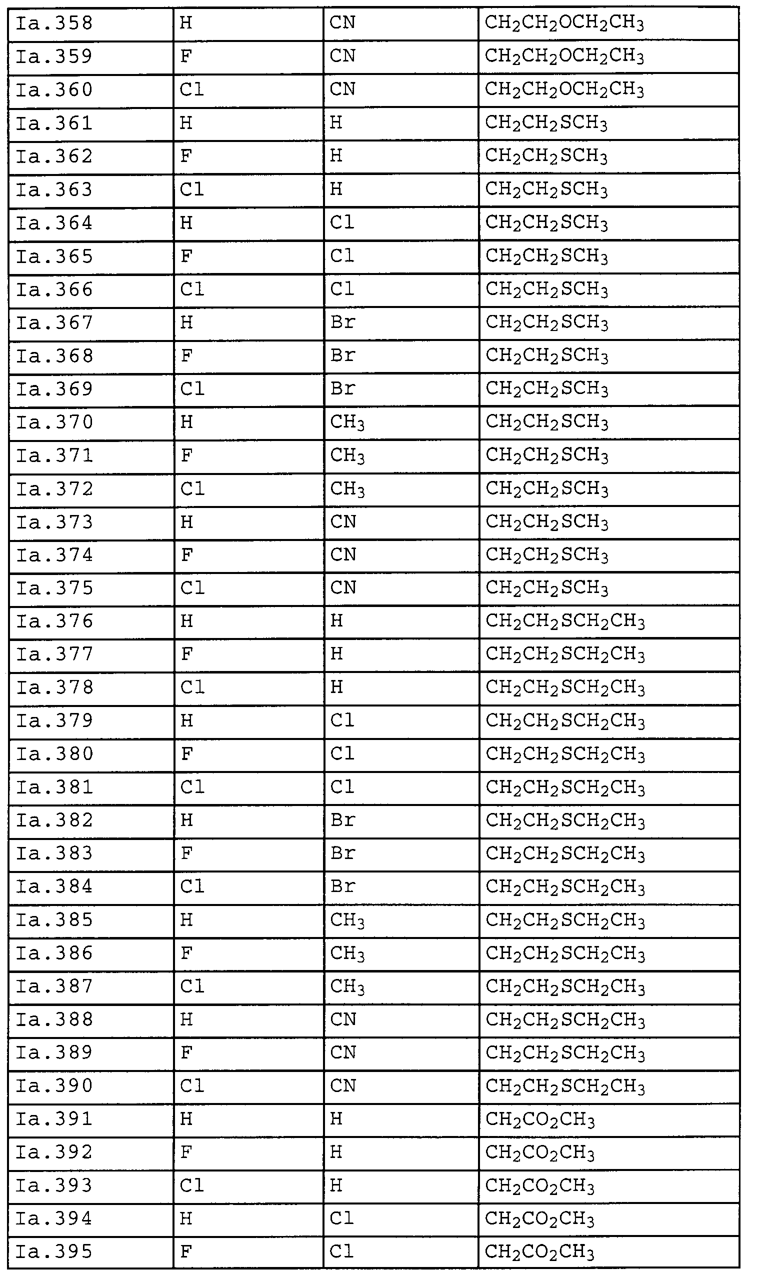

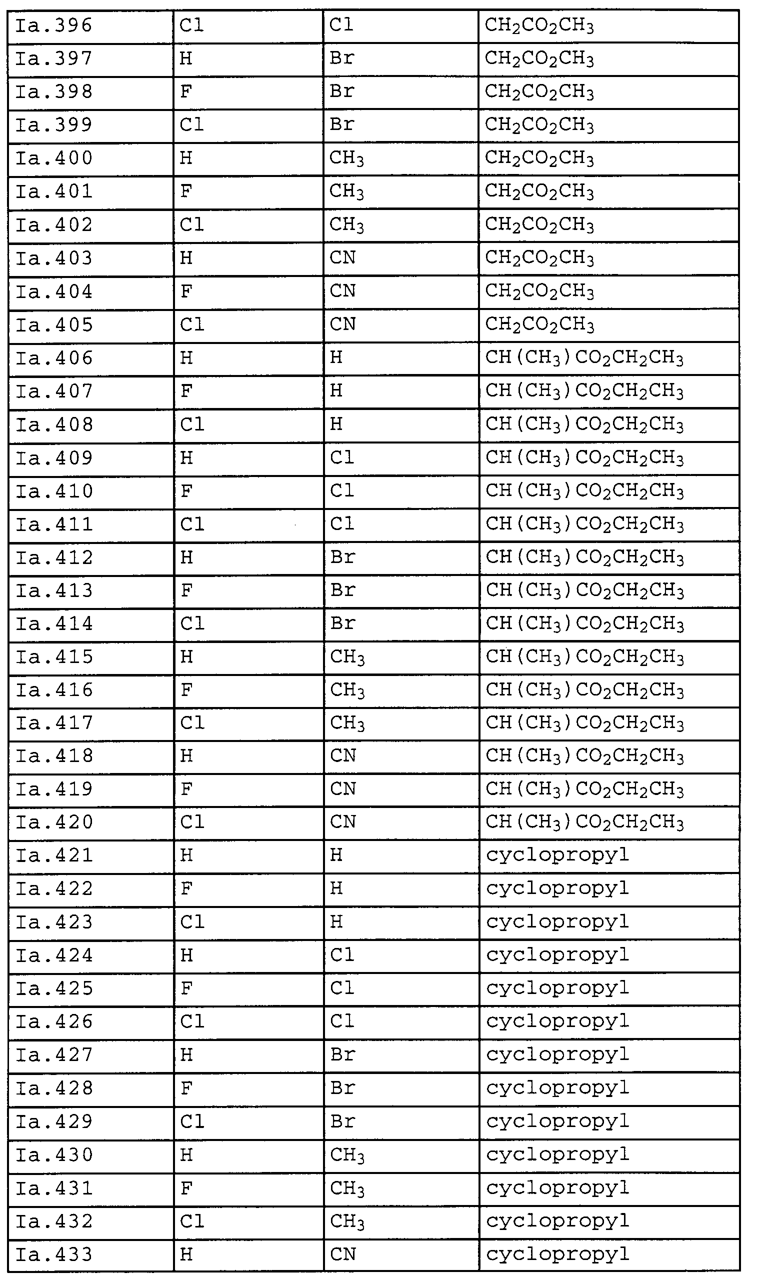

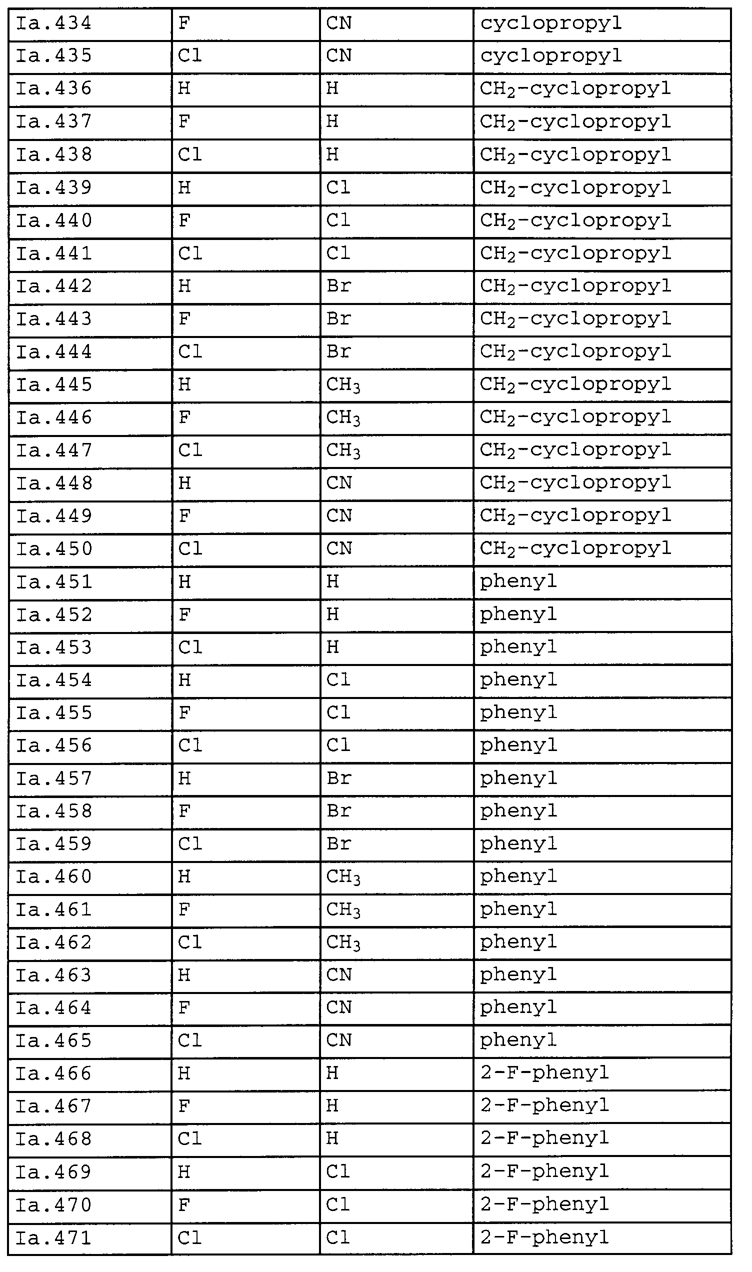

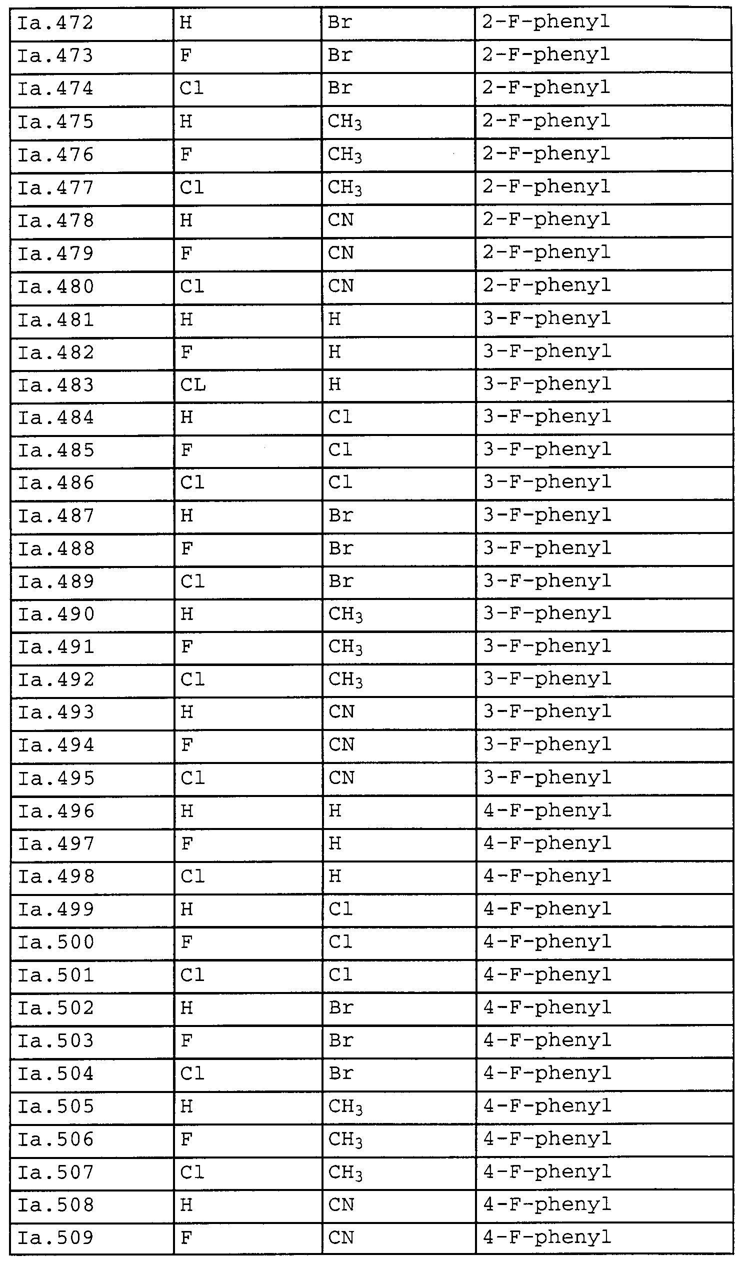

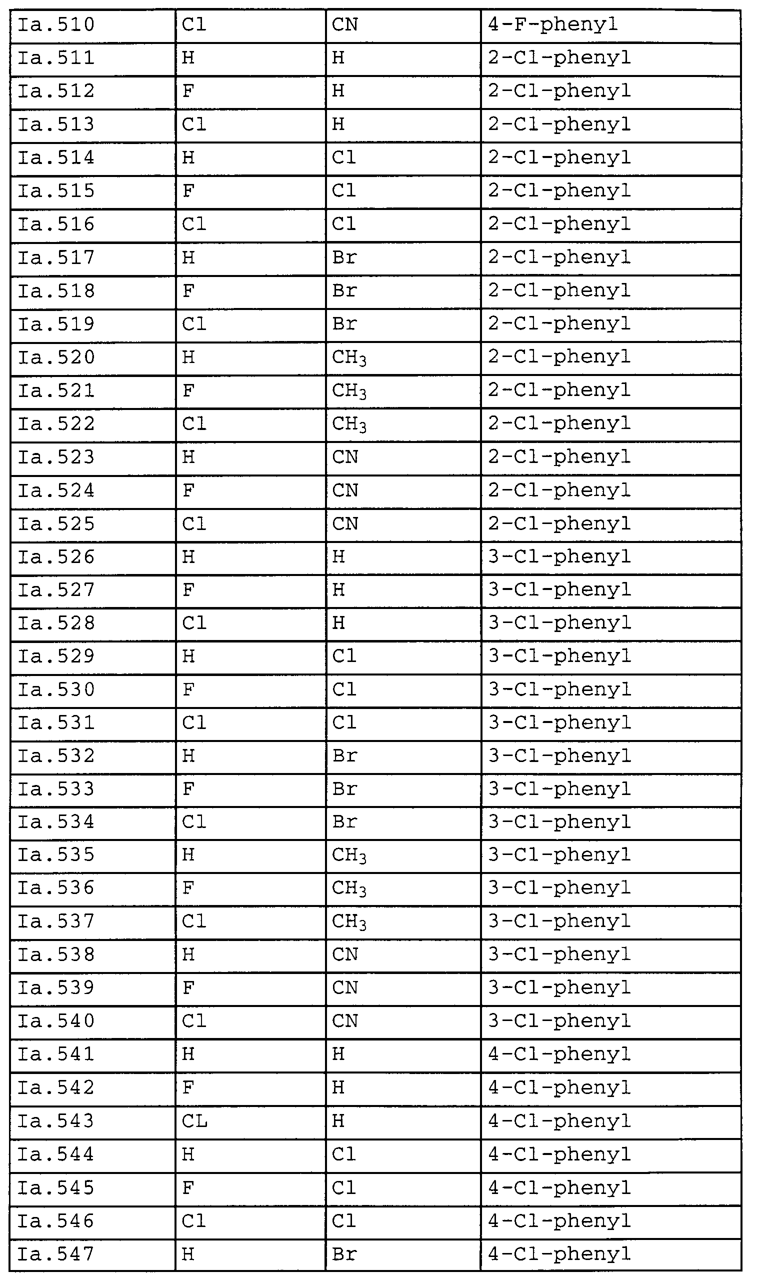

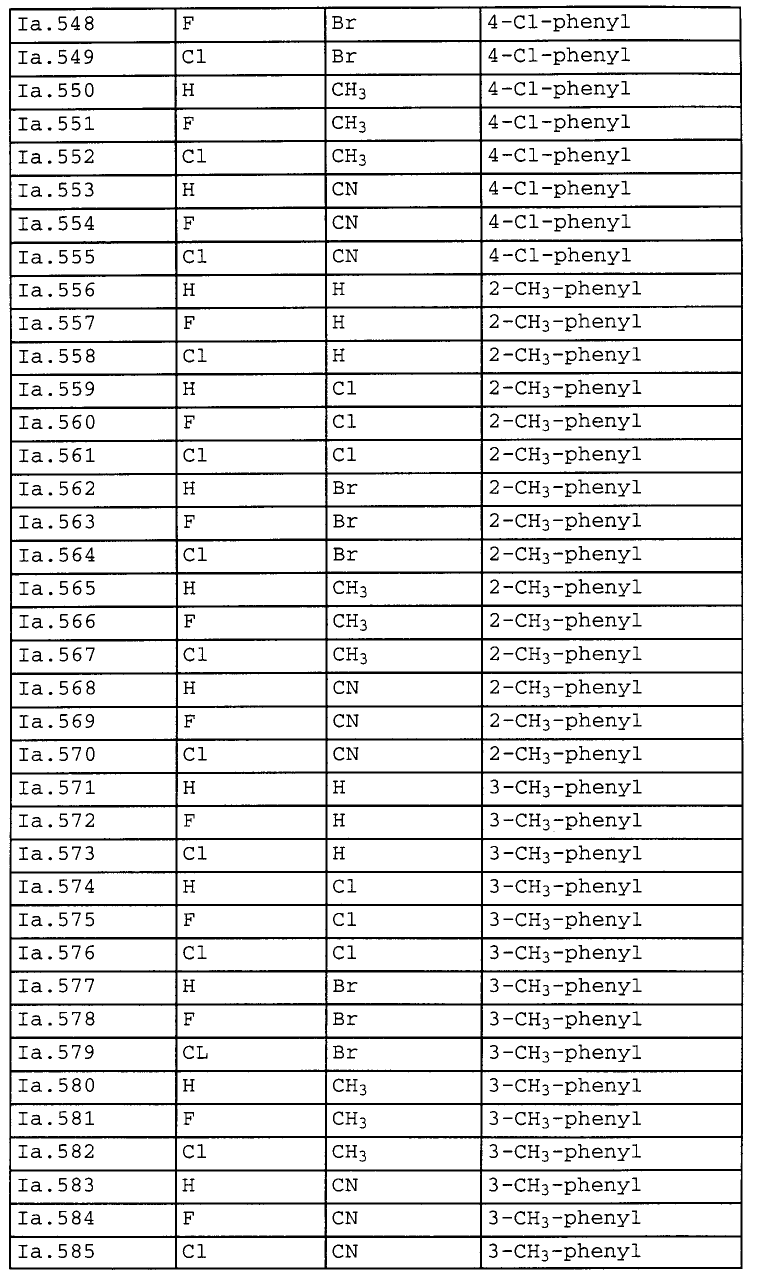

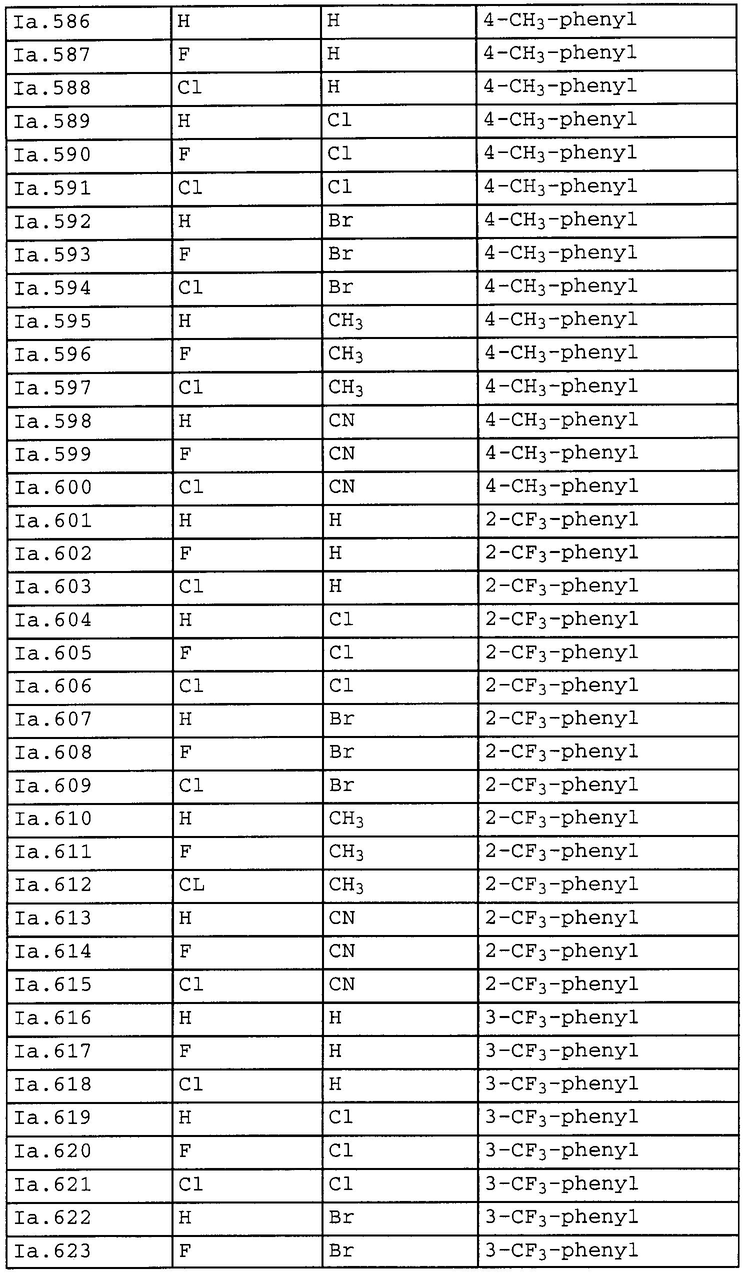

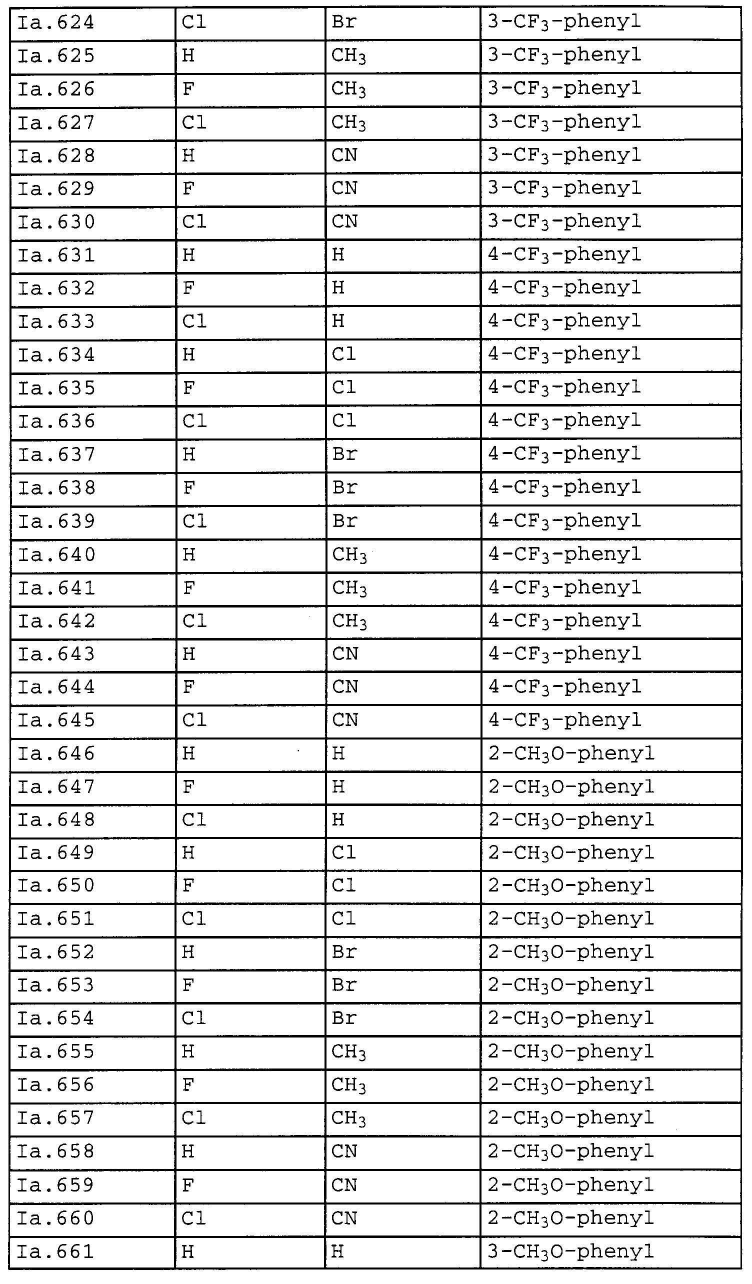

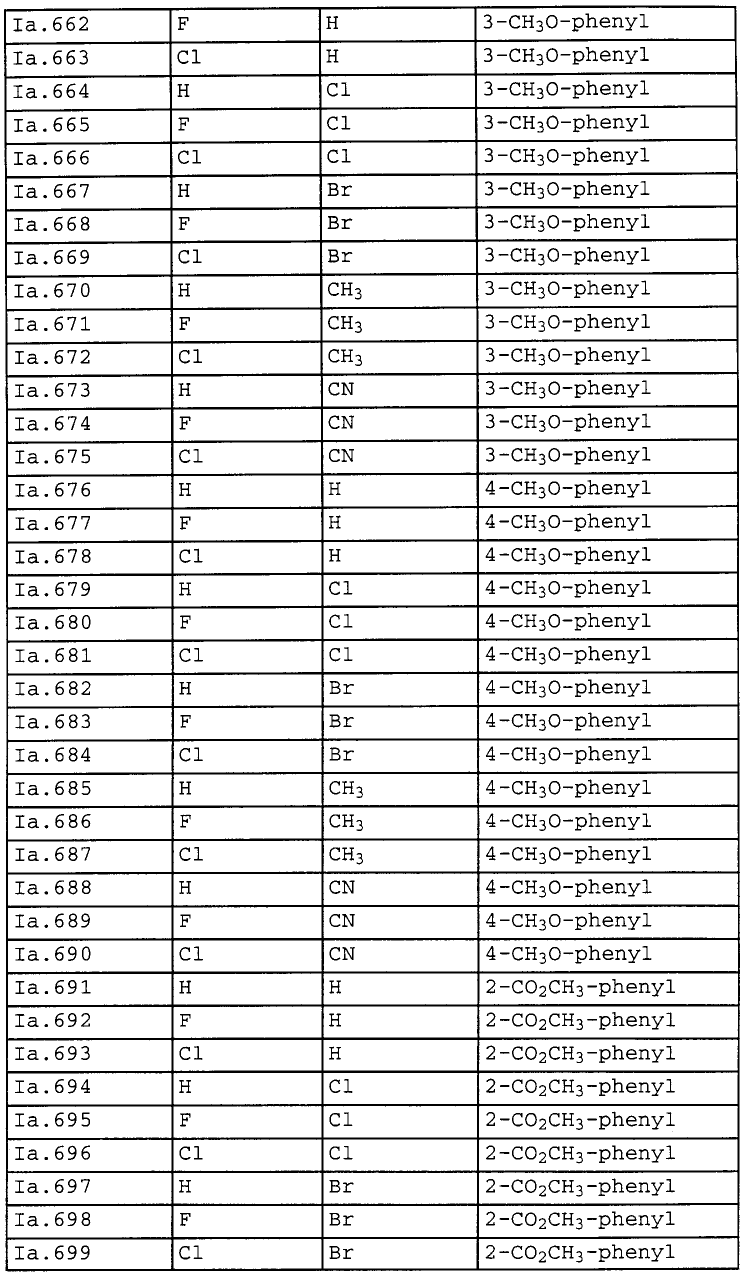

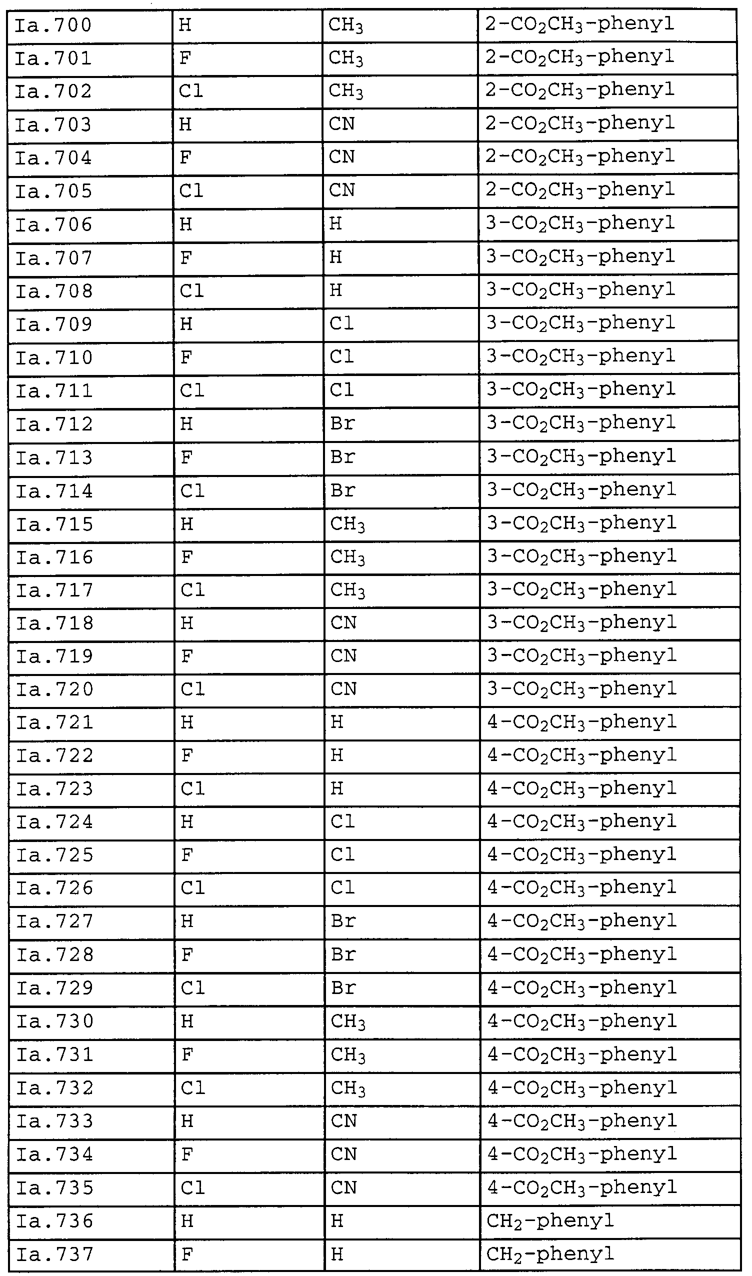

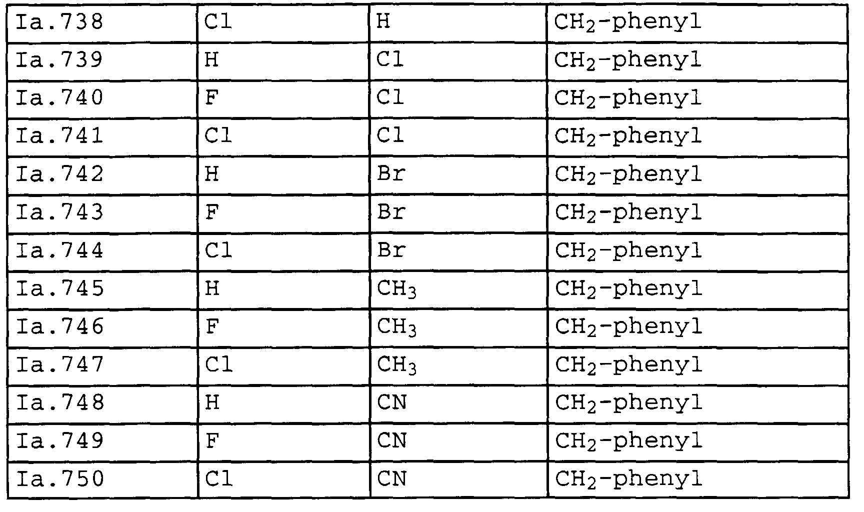

Des weiteren sind die folgenden substituierte Zimtsäurederivate der Formel I besonders bevorzugt:
- die Verbindungen Ib.001 - Ib.750, die sich von den entsprechenden Verbindungen Ia.001 - Ia.750 dadurch unterscheiden, daß R⁶ für Methyl steht: 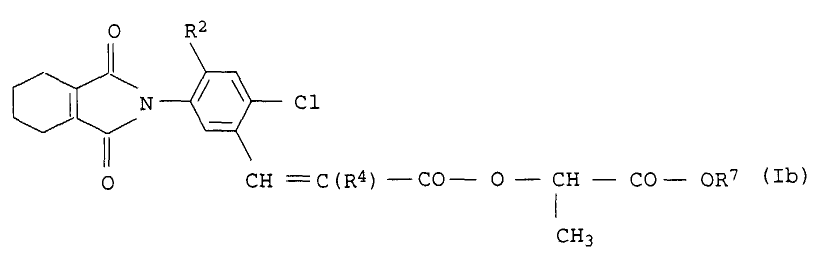
- die Verbindungen Ic.001 - Ic.750, die sich von den entsprechenden Verbindungen Ia.001 - Ia.750 dadurch unterscheiden, daß R⁶ für Ethyl steht: 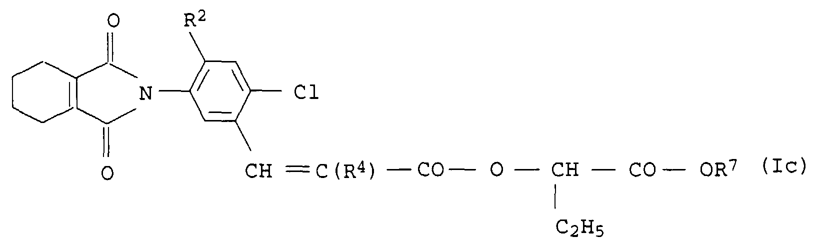
- die Verbindungen Id.001 - Id.750, die sich von den entsprechenden Verbindungen Ia.001 - Ia.750 dadurch unterscheiden, daß R⁶ für Isopropyl steht: 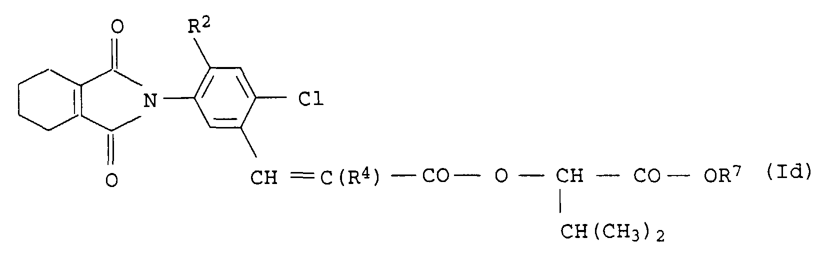
- die Verbindungen Ie.001 - Ie.750, die sich von den entsprechenden Verbindungen Ia.001 - Ia.750 dadurch unterscheiden, daß R⁶ für Cyclopropyl steht: 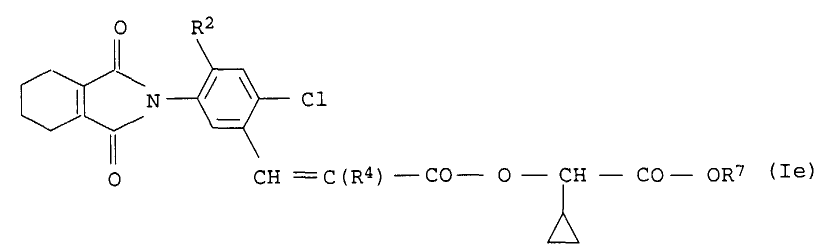
- die Verbindungen If.001 - If.750, die sich von den entsprechenden Verbindungen Ia.001 - Ia.750 dadurch unterscheiden, daß R⁵ und R⁶ für Methyl stehen: 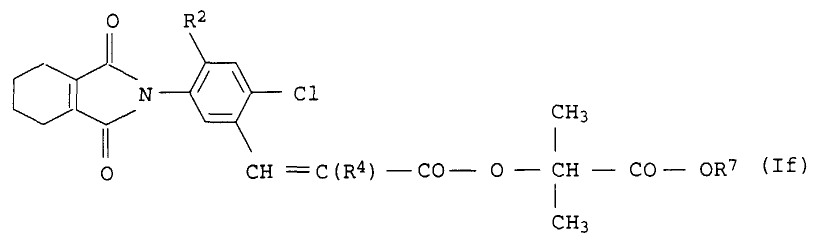
- die Verbindungen Ig.001 - Ig.750, die sich von den entsprechenden Verbindungen Ia.001 - Ia.750 dadurch unterscheiden, daß R⁵ für Ethoxy steht: 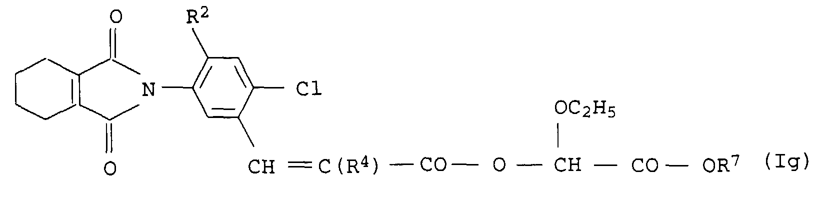
- die Verbindungen Ih.001 - Ih.750, die sich von den entsprechenden Verbindungen Ia.001 - Ia.750 dadurch unterscheiden, daß X für NH steht: 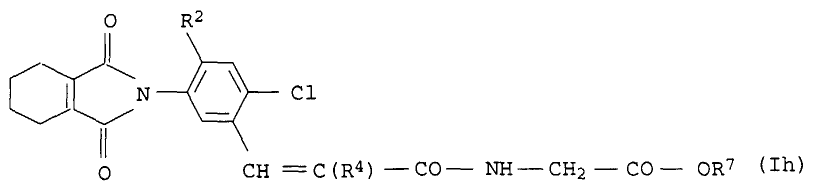
- die Verbindungen Ii.001 - Ii.750, die sich von den entsprechenden Verbindungen Ia.001 - Ia.750 dadurch unterscheiden, daß X für NH und R⁶ für Methyl stehen: 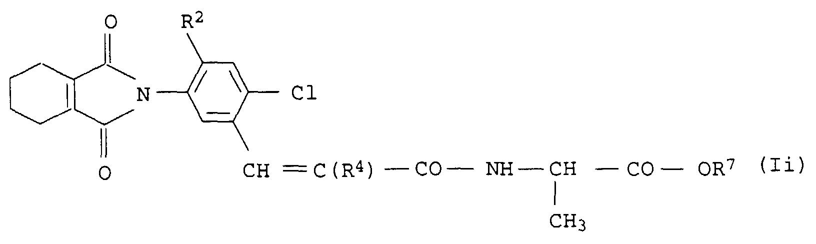
- die Verbindungen Ik.001 - Ik.750, die sich von den entsprechenden Verbindungen Ia.001 - Ia.750 dadurch unterscheiden, daß X für NH und R⁶ für Isopropyl stehen: 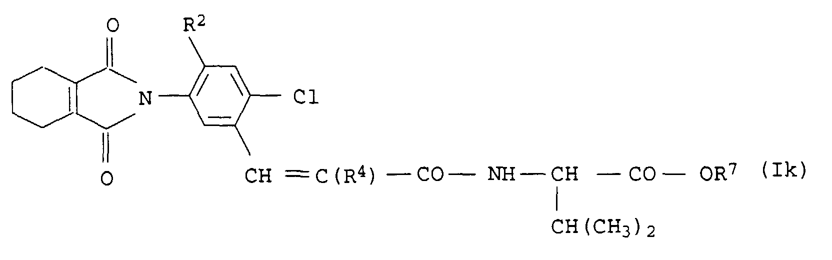
- die Verbindungen Il.001 - Il.750, die sich von den entsprechenden Verbindungen Ia.001 - Ia.750 dadurch unterscheiden, daß X für NH und R⁶ für 2-Methylpropyl stehen: 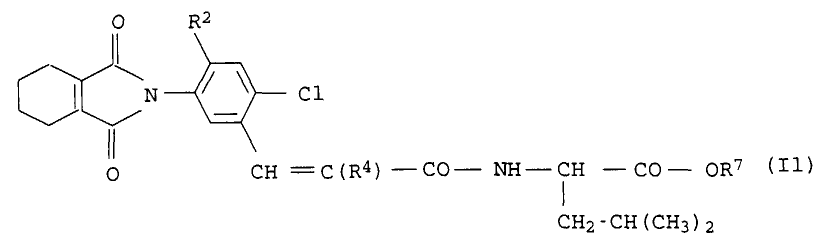
- die Verbindungen Im.001 - Im.750, die sich von den entsprechenden Verbindungen Ia.001 - Ia.750 dadurch unterscheiden, daß X für NH und R⁶ für 2-Methylthio-ethyl stehen: 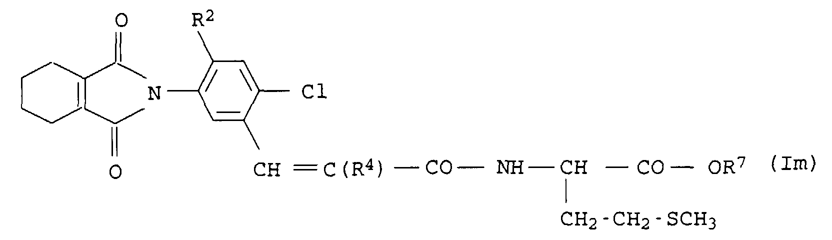
- die Verbindungen In.001 - In.750, die sich von den entsprechenden Verbindungen Ia.001 - Ia.750 dadurch unterscheiden, daß X für NH und R⁶ für Benzyl stehen: 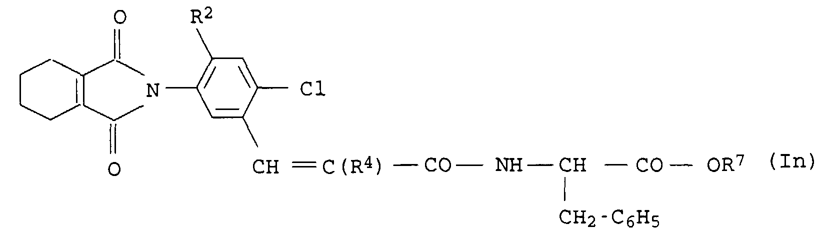
- die Verbindungen Io.001 - Io.750, die sich von den entsprechenden Verbindungen Ia.001 - Ia.750 dadurch unterscheiden, 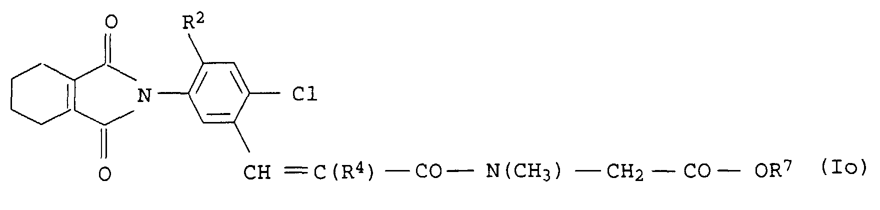 daß X für N(CH₃) steht:
- die Verbindungen Ip.001 - Ip.750, die sich von den entsprechenden Verbindungen Ia.001 - Ia.750 dadurch unterscheiden, daß X für N(R⁸) steht und R⁸ zusammen mit R⁶ eine Propylen-Kette bildet:

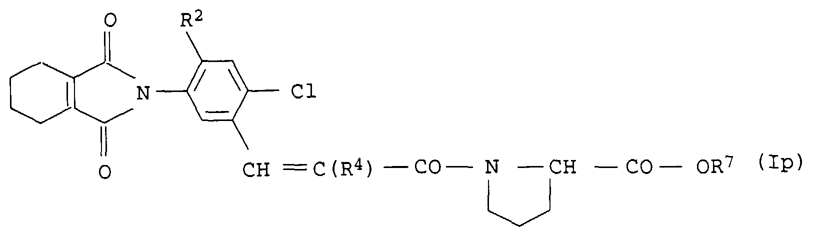

Die substituierten Phthalimido-Zimtsäurederivate der Formel I sind auf verschiedene Weise erhältlich, beispielsweise nach einem der folgenden Verfahren:

### Verfahren A:

Diazotierung von Anilinen III und Umsetzung der hierbei erhaltenen Diazoniumsalze mit Propinsäurederivaten IV auf an sich bekannte Weise (vgl. N.I. Granushchak et. al., Zh. Organ. Ximii. 1980, (16) HO12, 2578 - 2581) nach der Methode von Meerwein oder Modifikationen hiervon:

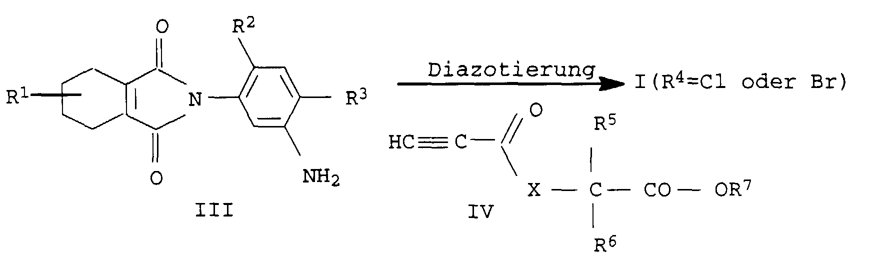

Bei dieser Methode überführt man das Anilin III zuerst in ein geeignetes Diazoniumsalz, welches dann mit IV in Gegenwart eines Kupfersalzes abreagiert.

Das Phenyldiazoniumsalz wird zweckmäßigerweise durch Umsetzung des Anilins III in einer wäßrigen Säurelösung, z.B. in Salzsäure, Bromwasserstoffsäure oder Schwefelsäure, mit einem Nitrit wie Natriumnitrit und Kaliumnitrit hergestellt und mit dem Propinsäurederivat IV in einem inerten Lösungsmittel in Gegenwart eines Kupferhalogenids wie Kupfer(I)chlorid, Kupfer(I)bromid, Kupfer(II)chlorid und Kupfer(II)bromid zur Reaktion gebracht.

Geeignete inerte Lösungsmittel sind z.B. Wasser, Acetonitil, Ketone wie Aceton, Diethylketon und Methylethylketon, Ether wie Dioxan und Tetrahydrofuran, ferner Alkohole wie Methanol und Ethanol.

Eine weitere Möglichkeit zur Herstellung des Phenyldiazoniumsalzes besteht darin, das Anilin III in einem wasserfreien System, beispielsweise in Eisessig, der Chlorwasserstoff enthält, in Dioxan, absolutem Ethanol, Tetrahydrofuran, Acetonitril oder in Aceton, mit einem Salpetrigsäureester wie tert.-Butylnitrit und Isopentylnitrit umzusetzen. In diesem Fall kann die Diazotierung in Gegenwart des Propinsäurederivates IV und des Kupferhalogenids stattfinden.

Die Reaktionstemperatur liegt normalerweise bei (-30) bis 80°C.

Üblicherweise werden die Komponenten der Diazotierungsreaktion in etwa stöchiometrischem Verhältnis eingesetzt, jedoch kann ein Überschuß einer der Komponenten, z.B. im Hinblick auf eine möglichst vollständige Umsetzung der anderen Komponente, vorteilhaft sein.

Das Propinsäurederivat IV kann in äquimolaren Mengen, im Überoder Unterschuß, bezogen auf das Phenyldiazoniumsalz, eingesetzt werden. Im allgemeinen hat sich ein großer Überschuß an Propinsäurederivat IV, bezogen auf das Phenyldiazoniumsalz, als besonders vorteilhaft erwiesen.

Das Kupferhalogenid wird normalerweise im stöchiometrischen Verhältnis eingesetzt, jedoch ist auch ein Über- oder Unterschuß möglich.

### Verfahren B:

Umsetzung eines Benzaldehyds V mit einem Ylid VI auf an sich bekannte Weise (vgl. z.B. R.S. Mali und V.J. Yadav, Synthesis (1O), 862 (1984)):

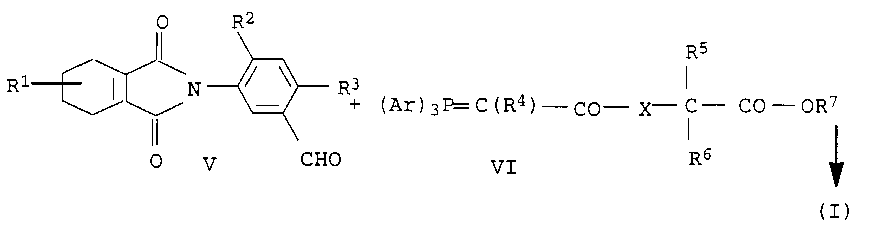

Ar steht für einen aromatischen Rest, der gewunschtenfalls substituiert sein kann, vorzugsweise für den Phenylrest.

Geeignete Lösungsmittel sind z.B. aromatische Kohlenwasserstoffe wie Benzol und Toluol, Ether wie Diethylether, Tetrahydrofuran und Dioxan, dipolar aprotische Lösungsmittel wie Dimethylsulfoxid und Dimethylformamid, oder protische Lösungsmittel wie Methanol und Ethanol. Auch Mischungen der genannten Solventien kommen in Betracht.

Normalerweise arbeitet man bei 0°C bis zur Siedetemperatur des jeweiligen Reaktionsgemisches.

Üblicherweise werden Benzaldehyd und Ylid in etwa stöchiometrischen Mengen eingesetzt, jedoch besteht auch die Möglichkeit, eine der Komponenten im Überschuß einzusetzen.

### Verfahren C:

Umsetzung eines Benzaldehyds V mit einer CH-aciden Verbindung VII auf an sich bekannte Weise (vgl. z.B. J. March, "Advanced Organic Cheimstry", S. 835 ff.):

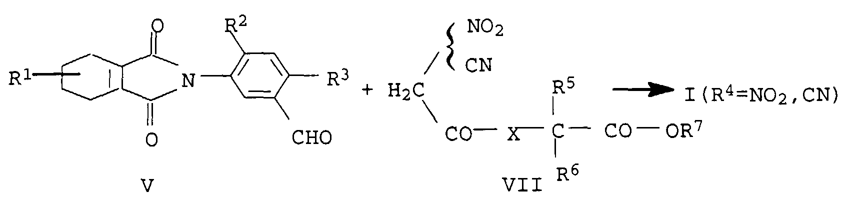

In Abhängigkeit von den jeweiligen Substituenten und den Reaktionsbedingungen kann es vorteilhaft sein, in Gegenwart einer katalytischen oder in etwa äquivalenten Menge Base, bezogen auf VII, oder in Gegenwart einer Säure zu arbeiten.

Als Basen kommen z.B. Alkalimetallalkoholate wie Natriummethanolat, Natriumethanolat und Kalium-tert.-butylat, aromatische ode aliphatische Stickstoffbasen wie Pyridin, Piperidin, Triethylamin, Ammoniumacetat und β-Alanin, ferner Metallhydride wie Natriumhydrid und Kaliumhydrid in Betracht.

Brauchbare Säuren sind insbesondere Essigsäure und Propionsäure.

Die Reatkion wird entweder lösungsmittelfrei oder in einem Überschuß an Base oder Säure oder in einem inerten Lösungs- oder Verdünnungsmittel vorgenommen., Je nach Reaktionsbedingungen eignen sich beispielsweise Alkohole wie Methanol und Ethanol oder Ether wie Diethylether und Methyl-tert.-butylether als Solventien.

Die Reaktion kann allgemein bei 0°C bis zur Siedetemperatur des Reaktionsgemisches vorgenommen werden.

Üblicherweise werden die Ausgangsverbindungen V und VII in etwa äquimolaren Mengen eingesetzt, jedoch kann auch eine der Komponenten im Überschuß eingesetzt werden.

### Verfahren D:

Umsetzung einer aktivierten Carbonsäure VIII mit einem Nukleophil IX oder einer aktivierten Carbonsäure X mit einem Alkohol HOR⁷ auf an sich bekannte Weise (vgl. z.B. J. March, "Advanced Organic Chemistry", S. 348 ff.):

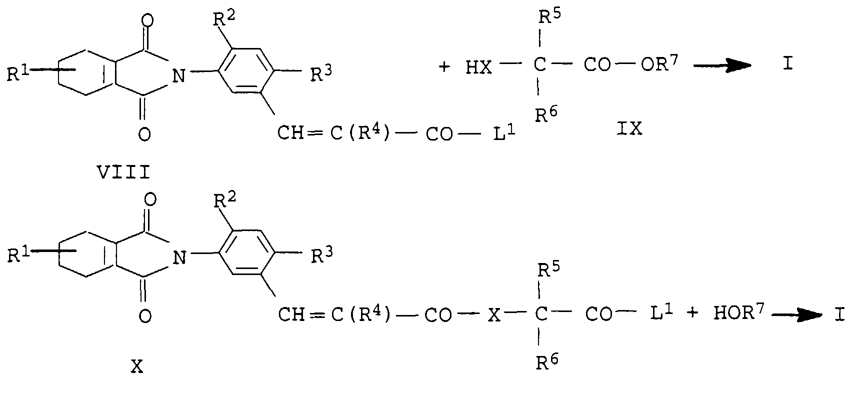

L¹ steht für eine übliche Abgangsgruppe, z.B. für Chlor, Brom oder 1-Imidazolyl.

Sofern L¹ Chlor oder Brom bedeutet, kann die Anwesenheit einer Base wie Triethylamin und Pyridin von Vorteil sein.

Als Lösungsmittel eignen sich z.B. halogenierte Kohlenwasserstoffe wie Methylenchlorid und 1,2-Dichlorethan, aromatische Kohlenwasserstoffe und Halogenkohlenwasserstoffe wie Benzol, Toluol und Chlorbenzol, Ether wie Diethylether, Tetrahydrofuran, Methyl-tert.-butylether und Dioxan, dipolar aprotische Lösungsmittel wie Dimethylsulfoxid und Dimethylformamid, oder Mischungen derartiger Solventien.

Die Reaktion wird allgemein bei (- 20)°C bis Siedetemperatur des Reaktionsgemisches vorgenommen.

Üblicherweise werden die Ausgangsverbindungen in etwa stöchiometrischen Mengen eingesetzt, sofern es sich nicht empfiehlt, eine der Komponenten im Überschuß einzusetzen.

Die aktivierten Carbonsäuren VIII und X mit L¹ = Chlor oder Brom sind ihrerseits in an sich bekannter Weise durch Umsetzung der entsprechenden Carbonsäuren XI bzw. I mit R⁷ = Wasserstoff mit einem Halogenierungsmittel, z.B. mit Thionylchlorid, Thionylbromid, Sulfurylchlorid, Sulfurylbromid, einem organischen Sulfonsäurechlorid wie Tosylchlorid, Phosphortrichlorid, Phoshortribromid, Phosphorpentachlorid, Phosphorpentabromid, Phorphoroxybromid, oder mit einem binären Halogenierungssystem wie Tetrachlormethan/ Triphenylphosphin und Tetrabrommethan/Triphenylphosphin erhältlich:

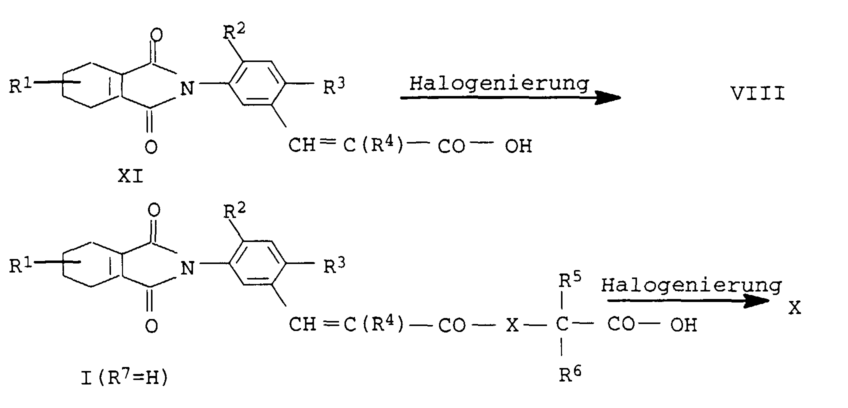

Die Halogenierung kann ohne Lösungsmittel oder in einem inerten Lösungsmittel durchgeführt werden. Geeignete Lösungsmittel sind - in Abhängigkeit vom Halogenierungsmittel - allgemein z.B. halogenierte Kohlenwasserstoffe wie Methylenchlorid, Chloroform und 1,2-Dichlorethan, aromatische Kohlenwasserstoffe und Halogenkohlenwasserstoffe wie Benzol, Toluol und Chorbenzol, dipolare aprotische Lösungsmittel wie Acetonitril, ferner Schwefelkohlenstoff, Ether wie Diethylether, Methyl-tert.-butylether und Tetrahydrofuran. Auch Mischungen dieser Solventien kommen in Betracht.

Die Halogenierung kann bei (- 30)°C bis zur Siedetemperatur des Reaktionsgemisches erfolgen.

Je nach Halogenierungsmittel wird die zu halogenierende Säure zweckmäßigerweise in stöchiometrischer Menge oder im Unterschuß eingesetzt.

Eine Variante des Verfahrens besteht darin, die aktivierte Carbonsäure VIII oder X in situ herzustellen, insbesondere wenn L¹ für 1-Imidazolyl steht oder wenn man unter Mitsunobu-Bedingungen (O. Mitsunobu, Synthesis 1981, 1) arbeitet.

### Verfahren E:

Umsetzung eines Säurederivats XII mit einem Alkylierungsmittel XIII oder einer Verbindung I, in der R⁷ = Wasserstoff ist, mit einem Alkylierungsmittel L²R⁷ in an sich bekannter Weise:

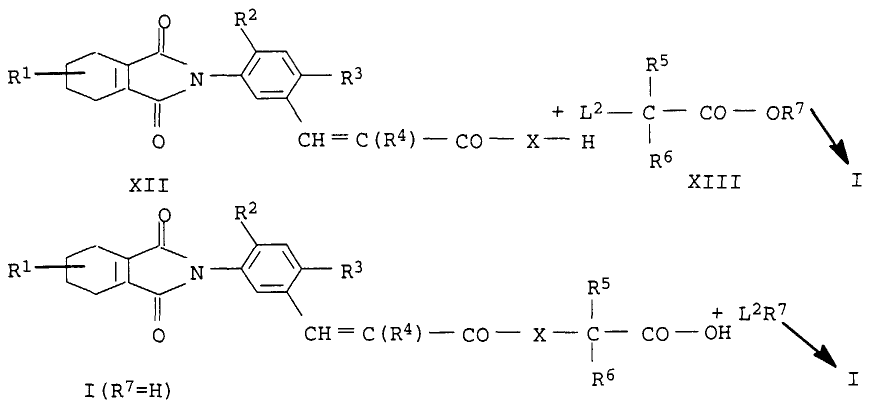

L² steht für eine übliche Abgangsgruppe wie Halogen, vorzugsweise Chlor, Brom oder Jod, Alkyl- oder Halogenalkylsulfonyloxy, vorzugsweise Methylsulfonyloxy oder Trifluormethylsulfonyloxy, Arylsulfonyloxy, vorzugsweise Toluolsulfonyloxy, und Alkoxysulfonyloxy, vorzugsweise Methoxysulfonyloxy oder Ethoxysulfonyloxy.

Zweckmäßigerweise nimmt man die Umsetzung in einem inerten Lösungsmittel vor, beispielsweise in einem Ether wie Diethylether, Methyl-tert.-butyhlether, Dimethoxyethan, Diethylenglycoldimethylether, Tetrahydrofuran und Dioxan, einem Keton wie Aceton, Diethylketon, Ethylmethylketon und Cyclohexanon, einem dipolaren aprotischen Lösungsmittel wie Acetonitril, Dimethylformamid, N-Methylpyrrolidon und Dimethylsulfoxid, einem protischen Lösungsmittel wie Methanol und Ethanol, einem aromatischen Kohlenwasserstoff oder Halogenkohlenwasserstoff wie Benzol, Chlorbenzol und 1,2-Dichlorbenzol, einem heteroaromatischen Lösungsmittel wie Pyridin und Chinolin oder in einem Gemisch derartiger Solventien.

Besonders geeignet sind Tetrahydrofuran, Aceton, Diethylketon und Dimethylformamid.

Die Alkylierungen werden normalerweise in Gegenwart einer Base vorgenommen, wobei z.B. die Hydroxide, Hydride, Alkoxide, Carbonate oder Hydrogencarbonate von Alkalimetall- und Erdalkalimetallkationen, oder tertiäre aliphatische Amine wie Triethylamin, N-Methylmorpholin und N-Ethyl-N,N-diisopropylamin, bi- und tri-cyclische Amine wie Diazabicycloundecan (DBU) und Diazabicyclooctan (DABCO), oder aromatische Stickstoffbasen wie Pyridin, 4-Dimethylaminopyridin und Chinolin, in Betracht kommen. Auch die Kombinationen verschiedener Basen ist möglich. Bevorzugte Basen sind Natriumhydrid, Natriumhydroxid, Natriumcarbonat, Kaliumcarbonat, Natriummethylat, Natriumethylat und Kalium-tert.-butylat.

Üblicherweise setzt man die Ausgangsstoffe in etwa stöchiometrischen Mengen ein, jedoch kann auch ein Überschuß der einen oder anderen Komponente im Hinblick auf die Verfahrensführung oder eine möglichst vollständige Umsetzung von XII oder von I mit R⁷ = Wasserstoff vorteilhaft sein.

Das molare Verhältnis von Säurederivat XII, oder I mit R⁷ = Wasserstoff, zu Base beträgt im allgemeinen 1:1 bis 1:3.

Die Konzentration der Ausgangsstoffe im Lösungsmittel liegt normalerweise bei etwa 0,1 bis 5,0 mol/l.

Die Umsetzung kann bei Temperaturen von 0°C bis zur Siedetemperatur des jeweiligen Reaktionsgemisches vorgenommen werden.

### Verfahren F:

Umsetzung eines Tetrahydrophtalsäureanhydrids XIV mit einem Aminozimtsäurederivat IIa in an sich bekannter Weise:

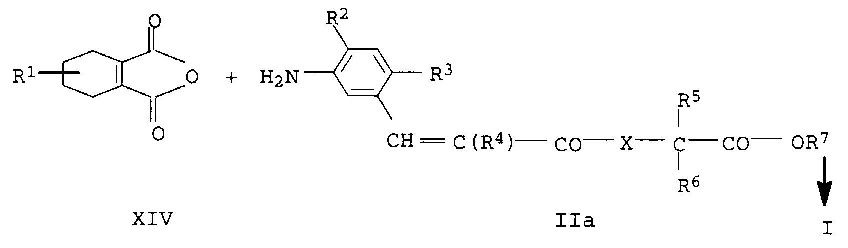

Als Lösungs-/Verdünnungsmittel eignen sich z.B. Alkancarbonsäuren wie Essigsäure, Propionsäure und Isobuttersäure, Alkancarbonsäureester wie Essigester, aprotische Solventien wie Dimethylformamid, Dimethylsulfoxid und N-Methylpyrrolidon oder Aromaten wie Toluol und die xylole. Bei Verwendung eines aprotischen Lösungsmittels empfiehlt es sich, das entstehende Reaktionswasser kontinuierlich zu entfernen bzw. eine Säurekatalyse (mit z. B. p-Toluolsulfonsäure, Trifluormethansulfonsäure etc.) vorzunehmen.

Die Reaktionstemperatur liegt gewöhnlich bei 0°C bis Siedetemperatur des Reaktionsgemisches.

In der Regel werden die Ausgangsstoffe in etwa stöchiometrischen Mengen eingesetzt, jedoch kann auch ein Über- oder Unterschuß der einen oder anderen Komponente, bis etwa 10 mol-%, vorteilhaft sein.

Die Aminozimtsäurederivate IIa sind neu und ihrerseits z.B. nach einem der unter Verfahren A bis E beschriebenen Methoden oder auf an sich bekannte Weise (vgl. z.B. Houben-Weyl, Methoden der organischen Chemie, Bd. XI/1, 4. Auflage 1957, S. 431 ff) durch Reduktion der entsprechenden Nitrozimtsäurederivate IIb erhältlich:

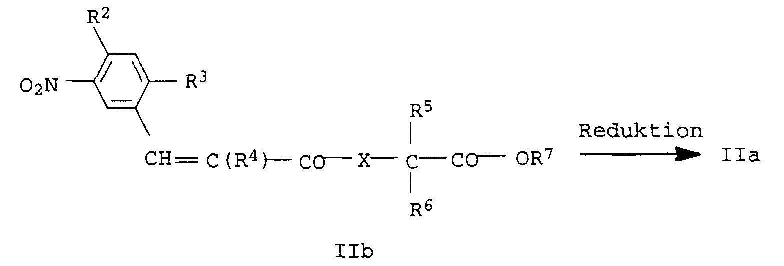

Als Reduktionsmittel kommen insbesondere
- elementare Metalle wie Eisen, Zinn und Zink,
- Wasserstoff in Gegenwart von geeigneten Katalysatoren wie Palladium oder Platin auf Kohle oder Raney-Nickel, oder
- komplexe Hydride wie LiAlH₄ und NaBH₄, ggf. in Gegenwart von Katalysatoren,
in Betracht.

Als Lösungsmittel eignen sich üblicherweise - je nach Reduktionsmittel - Carbonsäuren wie Essigsäure und Propionsäure, Alkohole wie Methanol und Ethanol, Ether wie Diethylether, Methyl-tert.-butylether, Tetrahydrofuran und Dioxan, Aromaten wie Benzol und Toluol sowie Gemische derartiger Solventien.

Die Umsetzungen können bei Temperaturen von (-100)°C bis zur Siedetemperatur des jeweiligen Reaktionsgemisches vorgenommen werden.

Üblicherweise werden die Ausgangsverbindungen in etwa stöchiometrischen Mengen eingesetzt; in Einzelfällen kann jedoch auch ein Überschuß der einen oder anderen Komponente, bis etwa 10 mol-%, vorteilhaft sein.

Die Nitrozimtsäurederivate IIb sind ebenfalls neu und z.B. nach den unter Verfahren A bis E beschriebenen Methoden herstellbar.

Sofern nicht anders angegeben werden alle vorstehend beschriebenen Verfahren zweckmäßigerweise bei Atmosphärendruck oder unter dem Eigendruck des jeweiligen Reaktionsgemisches vorgenommen.

Die für die einzelnen Verfahren angegebenen Ausgangsverbindungen sind entweder bekannt oder auf an sich bekannte Weise z. B. auch nach einem der beschriebenen Verfahren, erhältlich.

Die substituierten Phthalimido-Zimtsäurederivate der Formel I können ein oder mehrere Chiralitätszentren enthalten und fallen dann üblicherweise als Enantiomeren- oder Diastereomerengemische an. Die Mischungen können gewünschtenfalls nach den hierfür üblichen Methoden, z.B. mittels Kristallisation oder Chromatographie an einem optisch aktiven Adsorbat, in die weitgehend reinen Isomeren getrennt werden. Reine optisch aktive Isomere lassen sich beispielsweise auch aus entsprechenden optisch aktiven Ausgangsmaterialien herstellen.

Die Verbindungen I sowie deren landwirtschaftlich brauchbaren Salze eignen sich - sowohl als Isomerengemische als auch in Form der reinen Isomeren - als Herbizide. Die I enthaltenden herbiziden Mittel können in Kulturen wie Weizen, Reis, Mais, Soja und Baumwolle Unkräuter und Schadgräser sehr gut bekämpfen, ohne die Kulturpflanzen nennenswert zu schädigen. Dieser Effekt tritt vor allem bei niedrigen Aufwandmengen auf.

In Abhängigkeit von der jeweiligen Applikationsmethode können die Verbindungen I bzw. sie enthaltenden herbiziden Mittel noch in einer weiteren Zahl von Kulturpflanzen zur Beseitigung unerwünschter Pflanzen eingesetzt werden. In Betracht kommen beispielsweise folgende Kulturen:

Allium cepa, Ananas comosus, Arachis hypogaea, Asparagus officinalis, Beta vulgaris spp. altissima, Beta vulgaris spp. rapa, Brassica napus var. napus, Brassica napus var. napobrassica, Brassica rapa var. silvestris, Camellia sinensis, Carthamus tinctorius, Carya illinoinensis, Citrus limon, Citrus sinensis, Coffea arabica (Coffea canephora, Coffea liberica), Cucumis sativus, Cynodon dactylon, Daucus carota, Elaeis guineensis, Fragaria vesca, Glycine max, Gossypium hirsutum, (Gossypium arboreum, Gossypium herbaceum, Gossypium vitifolium), Helianthus annuus, Hevea brasiliensis, Hordeum vulgare, Humulus lupulus, Ipomoea batatas, Juglans regia, Lens culinaris, Linum usitatissimum, Lycopersicon lycopersicum, Malus spp., Manihot esculenta, Medicago sativa, Musa spp., Nicotiana tabacum (N.rustica), Olea europaea, Oryza sativa , Phaseolus lunatus, Phaseolus vulgaris, Picea abies, Pinus spp., Pisum sativum, Prunus avium, Prunus persica, Pyrus communis, Ribes sylvestre, Ricinus communis, Saccharum officinarum, Secale cereale, Solanum tuberosum, Sorghum bicolor (s. vulgare), Theobroma cacao, Trifolium pratense, Triticum aestivum, Triticum durum, Vicia faba, Vitis vinifera und Zea mays.

Darüber hinaus können die Verbindungen I auch in Kulturen, die durch Züchtung einschließlich gentechnischer Methoden gegen die Wirkung von Herbiziden tolerant sind, verwandt werden.

Des weiteren eignen sich die substituierten Phalimido-Zimtsäurederivate I auch zur Desikkation und/oder Defoliation von Pflanzen.

Als Desiccantien eignen sie sich insbesondere zur Austrocknung der oberirdischen Teile von Kulturpflanzen wie Kartoffel, Raps, Sonnenblume und Sojabohne. Damit wird ein vollständig mechanisches Beernten dieser wichtigen Kulturpflanzen ermöglicht.

Von wirtschaftlichem Interesse ist ferner die Ernteerleichterung, die durch das zeitlich konzentrierte Abfallen oder Vermindern der Haftfestigkeit am Baum bei Zitrusfrüchten, Oliven oder bei anderen Arten und Sorten von Kern-, Stein- und Schalenobst ermöglicht wird. Derselbe Mechanismus, das heißt die Förderung der Ausbildung von Trenngewebe zwischen Frucht- oder Blatt- und Sproßteil der Pflanzen ist auch für ein gut kontrollierbares Entblättern von Nutzpflanzen, insbesondere Baumwolle, wesentlich.

Außerdem führt die Verkürzung des Zeitintervalls, in dem die einzelnen Baumwollpflanzen reif werden, zu einer erhöhten Faserqualität nach der Ernte.

Die Verbindungen I bzw. die sie enthaltenden herbiziden Mittel können beispielsweise in Form von direkt versprühbaren wäßrigen Lösungen, Pulvern, Suspensionen, auch hochprozentigen wäßrigen, öligen oder sonstigen Suspensionen oder Dispersionen, Emulsionen, Öldispersionen, Pasten, Stäubemitteln, Streumitteln oder Granulaten durch Versprühen, Vernebeln, Verstäuben, Verstreuen oder Gießen angewendet werden. Die Anwendungsformen richten sich nach den Verwendungszwecken; sie sollten in jedem Fall möglichst die feinste Verteilung der erfindungsgemäßen Wirkstoffe gewährleisten.

Als inerte Hilfsstoffe für die Herstellung von direkt versprühbaren Lösungen, Emulsionen, Pasten oder Öldispersionen kommen im wesentlichen in Betracht: Mineralölfraktionen von mittlerem bis hohem Siedepunkt wie Kerosin und Dieselöl, ferner Kohlenteeröle sowie Öle pflanzlichen oder tierischen Ursprungs, aliphatische, cyclische und aromatische Kohlenwasserstoffe, z.B. Paraffine, Tetrahydronaphthalin, alkylierte Naphthaline und deren Derivate, alkylierte Benzole und deren Derivate, Alkohole wie Methanol, Ethanol, Propanol, Butanol und Cyclohexanol, Ketone wie Cyclohexanon, stark polare Lösungsmittel, z.B. Amine wie N-Methylpyrrolidon und Wasser.

Wäßrige Anwendungsformen können aus Emulsionskonzentraten, Suspensionen, Pasten, netzbaren Pulvern oder wasserdispergierbaren Granulaten durch Zusatz von Wasser bereitet werden. Zur Herstellung von Emulsionen, Pasten oder Öldispersionen können die Substrate als solche oder in einem Öl oder Lösungsmittel gelöst, mittels Netz-, Haft-, Dispergier- oder Emulgiermittel in Wasser homogenisiert werden. Es können aber auch aus wirksamer Substanz, Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen die Alkali-, Erdalkali-, Ammoniumsalze von aromatischen Sulfonsäuren, z.B. Lignin-, Phenol-, Naphthalin- und Dibutylnaphthalinsulfonsäure, sowie von Fettsäuren, Alkyl- und Alkylarylsulfonaten, Alkyl-, Laurylether- und Fettalkoholsulfaten, sowie Salze sulfatierter Hexa-, Hepta- und Octadecanolen sowie von Fettalkoholglykolether, Kondensationsprodukte von sulfoniertem Naphthalin und seiner Derivate mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctyl-, Octyl- oder Nonylphenol, Alkylphenyl-, Tributylphenylpolyglykolether, Alkylarylpolyetheralkohole, Isotridecylalkohol, Fettalkoholethylenoxid-Kondensate, ethoxyliertes Rizinusöl, Polyoxyethylen- oder Polyoxypropylenalkylether, Laurylalkoholpolyglykoletheracetat, Sorbitester, Lignin-Sulfitablaugen oder Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate können durch Bindung der Wirkstoffe an feste Trägerstoffe hergestellt werden. Feste Trägerstoffe sind Mineralerden wie Kieselsäuren, Kieselgele, Silikate, Talkum, Kaolin, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie Ammoniumsulfat, Ammoniumphosphat, Ammoniumnitrat, Harnstoffe und pflanzliche Produkte wie Getreidemehl, Baumrinden-, Holz- und Nußschalenmehl, Cellulosepulver oder andere feste Trägerstoffe.

Die Konzentrationen der Wirkstoffe I in den anwendungsfertigen Zubereitungen können in weiten Bereichen variiert werden, etwa vozwischen 0,01 bisund 95 Gew. %, vorzugsweise zwischen 0,5 bis 90 Gew.%. Die Wirkstoffe werden dabei in einer Reinheit von 90% bis 100%, vorzugsweise 95% bis 100% (nach NMR-Spektrum) eingesetzt.

Die folgenden Formulierungsbeispiele verdeutlichen die Herstellung solcher Zubereitungen:
I. 20 Gewichtsteile der Verbindung Nr. I.01 werden in einer Mischung gelöst, die aus 80 Gewichtsteilen alkyliertem Benzol, 10 Gewichtsteilen des Anlagerungsproduktes von 8 bis 10 Mol Ethylenoxid an 1 Mol Ölsäure-N-monoethanolamid, 5 Gewichtsteilen Calciumsalz der Dodecylbenzolsulfonsäure und 5 Gewichtsteilen des Anlagerungsproduktes von 40 Mol Ethylenoxid an 1 Mol Ricinusöl besteht. Durch Ausgießen und feines Verteilen der Lösung in 100 000 Gewichtsteilen Wasser erhält man eine wäßrige Dispersion, die 0,02 Gew.% des Wirkstoffs enthält.
II. 20 Gewichtsteile der Verbindung Nr. I.02 werden in einer Mischung gelöst, die aus 40 Gewichtsteilen Cyclohexanon, 30 Gewichtsteilen Isobutanol, 20 Gewichtsteilen des Anlagerungsproduktes von 7 Mol Ethylenoxid an 1 Mol Isooctylphenol und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gew.% des Wirkstoffs enthält.
III. 20 Gewichtsteile des Wirkstoffs Nr. I.13 werden in einer Mischung gelöst, die aus 25 Gewichtsteilen Cyclohexanon, 65 Gewichtsteilen einer Mineralölfraktion vom Siedepunkt 210 bis 280°C und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gew.% des Wirkstoffs enthält.
IV. 20 Gewichtsteile des Wirkstoffs Nr. I.14 werden mit 3 Gewichtsteilen des Natriumsalzes der Diisobutylnaphthalin-α-sulfonsäure, 17 Gewichtsteilen des Natriumsalzes einer Ligninsulfonsäure aus einer Sulfit-Ablauge und 60 Gewichtsteilen pulverförmigem Kieselsäuregel gut vermischt und in einer Hammermühle vermahlen. Durch feines Verteilen der Mischung in 20 000 Gewichtsteilen Wasser enthält man eine Spritzbrühe, die 0,1 Gew.% des Wirkstoffs enthält.
V. 3 Gewichtsteile des Wirkstoffs Nr. I.24 werden mit 97 Gewichtsteilen feinteiligem Kaolin vermischt. Man erhält auf diese Weise ein Stäubemittel, das 3 Gew.% des Wirkstoffs enthält.
VI. 20 Gewichtsteile des Wirkstoffs Nr. I.15 werden mit 2 Gewichtsteilen Calciumsalz der Dodecylbenzolsulfonsäure, 8 Gewichtsteilen Fettalkohol-polyglykolether, 2 Gewichtsteilen Natriumsalz eines Phenol-Harnstoff-Formaldehyd-Kondensates und 68 Gewichtsteilen eines paraffinischen Mineralöls innig vermischt. Man erhält eine stabile ölige Dispersion.

Die Applikation der Wirkstoffe I bzw. der herbiziden Mittel kann im Vorauflauf- oder im Nachauflaufverfahren erfolgen. Sind die Wirkstoffe für gewisse Kulturpflanzen weniger verträglich, so können Ausbringungstechniken angewandt werden, bei welchen die herbiziden Mittel mit Hilfe der Spritzgeräte so gespritzt werden, daß die Blätter der empfindlichen Kulturpflanzen nach Möglichkeit nicht getroffen werden, während die Wirkstoffe auf die Blätter darunter wachsender unerwünschter Pflanzen oder die unbedeckte Bodenfläche gelangen (post-directed, lay-by).

Die Aufwandmengen an Wirkstoff I betragen je nach Bekämpfungsziel, Jahreszeit, Zielpflanzen und Wachstumsstadium 0,001 bis 3,0, vorzugsweise 0,01 bis 2 kg/ha aktive Substanz (a.S.).

Zur Verbreiterung des Wirkungsspektrums und zur Erzielung synergistischer Effekte können die substituierten Phthalimido-Zimtsäurederivate I mit zahlreichen Vertretern anderer herbizider oder wachstumsregulierender Wirkstoffgruppen gemischt und gemeinsam ausgebracht werden. Beispielsweise kommen als Mischungspartner Diazine, 4H-3,1-Benzoxazinderivate, Benzothiadiazinone, 2,6-Dinitroaniline, N-Phenylcarbamate, Thiolcarbamate, Halogencarbonsäuren, Triazine, Amide, Harnstoffe, Diphenylether, Triazinone, Uracile, Benzofuranderivate, Cyclohexan-1,3-dionderivate, die in 2-Stellung z.B. eine Carboxy- oder Carbimino-Gruppe tragen, Chinolincarbonsäurederivate, Imidazolinone, Sulfonamide, Sulfonylharnstoffe, Aryloxy-, Heteroaryloxyphenoxypropionsäuren sowie deren Salze, Ester und Amide und andere in Betracht.

Außerdem kann es von Nutzen sein, die Verbindungen I allein oder in Kombination mit anderen Herbiziden auch noch mit weiteren Pflanzenschutzmitteln gemischt, gemeinsam auszubringen, beispielsweise mit Mitteln zur Bekämpfung von Schädlingen oder phytopathogenen Pilzen bzw. Bakterien. Von Interesse ist ferner die Mischbarkeit mit Mineralsalzlösungen, welche zur Behebung von Ernährungs- und Spurenelementmängeln eingesetzt werden. Es können auch nichtphytotoxische Öle und Ölkonzentrate zugesetzt werden.

### Herstellungsbeispiele

### Beispiel 1

### N-{4-Chlor-5-[2-(N-ethoxycarbonylmethyl-N-methyl)aminocarbonyl-2-chlor]-ethen-1-yl-2-fluorphenyl}-3,4,5,6-tetrahydrophthalimid (Verbindung Nr. I.17)

Zu einer Lösung von 5 mmol N-[4-Chlor-5-(2-hydroxycarbonyl-2-chlor)ethen-1-yl-2-fluorphenyl]-3,4,5,6-tetrahydrophthalimid in 50 ml abs. Tetrahydrofuran wurden 6 mmol Carbonyldimidazol getropft, wonach man eine Stunde bei ca. 20°C rührte. Anschließend gab man 5 mmol Sarcosinethylester-Hydrochlorid in das Reaktionsgemisch. Dann tropfte man 5 mmol Triethylamin in 5 ml Tetrahydrofuran zu. Nach 48 Stunden Rühren bei etwa 20°C entfernte man das Lösungsmittel am Wasserstrahlvakuum. Der Rückstand wurde in Wasser aufgenommen, wonach man das Produkt mit Essigsäureethylester extrahierte. Die vereinigten organischen Phasen wurden über Natriumsulfat getrocknet und dann eingeengt. Die Reinigung des Rohproduktes erfolgte chromatographisch an Kieselgel (Eluent: Petrolether/Methyl-tert-butylether = 1:2). Ausbeute: 0,97 g (40 %).

### Beispiel 2

### N-{4-Chlor-3-[2-(1-allyloxycarbonyl-eth-1-oxycarbonyl)ethen-1-yl]-phenyl)-3,4,5,6-tetrahydrophthalimid (Verbindung Nr. I.11)

Zu einer Mischung aus 5,4 mmol N-[4-Chlor-3-(2-hydroxycarbonyl-ethen-1-yl)-phenyl]-[-3,4,5,6-tetrahydrophthalimid, 5,4 mmol Kaliumcarbonat und 50 ml abs. Dimethylformid tropfte man bei 50°C eine Lösung von 6,5 mmol 2-Brompropionsäureallylester in 5 ml abs. Dimethylformid. Nach 10 Std. Rühren bei 50°C wurde man das Reaktionsgemisch abgekühlt. Dann entfernte man das Lösungsmittel am Wasserstrahlvakuum. Der Rückstand wurde in Wasser aufgenommen, wonach man das Produkt mit Methyl-tert-butylether extrahierte. Die vereinigten organischen Phasen wurden über Natriumsulfat getrocknet und dann eingeengt. Ausbeute: 2,3 g (96 %).

### Beispiel 3

### N-{4-Chlor-5-[2-chlor-2-(1-methoxycarbonyl-1-cyclopropyl-methoxycarbonyl)ethen-1-yl]-2-fluorphenyl}-3,4,5,6-tetrahydrophthalimid (Verbindung Nr. I.16)

Zu einer Lösung von 7,2 mmol N-{4-Chlor-5-(2-hydroxycarbonyl-2-chlor)-ethen-1-yl-2-fluorphenyl}-3,4,5,6-tetrahydrophtalimid und 7,2 mmol 1-Cyclopropyl-1-hydroxyessigsäuremethylester in 30 ml abs. Tetrahydrofuran wurden 7,9 mmol Triphenylphosphin gegeben. Anschließend tropfte man 7,9 mmol Azodicarbonsäurediethylester, in einem Gemisch aus 10 ml Tetrahydrofuran und 10 ml Toluol, zu. Nach 2 Stunden Rühren bei etwa 20°C wurden noch 3,95 mmol Triphenylphosphin und 3,95 mmol Azodicarbonsäurediethylester zu dem Reaktionsgemisch gegeben, wonach man 12 Std. bei ca. 20°C rührte. Dann gab man 1 ml Wasser und, nach weiteren 10 Minuten Rühren, drei Spatelspitzen Natriumsulfat in die Reaktionsmischung. Schließlich filtrierte man die ungelösten Anteile ab und engte das Filtrat im Wasserstrahlvakuum ein. Die Reinigung des Rohproduktes erfolgte mittels Chromatographie an Kieselgel (Eluent: Petrolether/Diethylether = 2:1). Ausbeute: 0,98 g (28 %).

In Tabelle 8 sind ganz besonders bevorzugte substituierte Phthalimido-Zimtsäurederivate der Formel I aufgeführt, die entsprechend den Beispielen hergestellt wurden oder herstellbar sind:

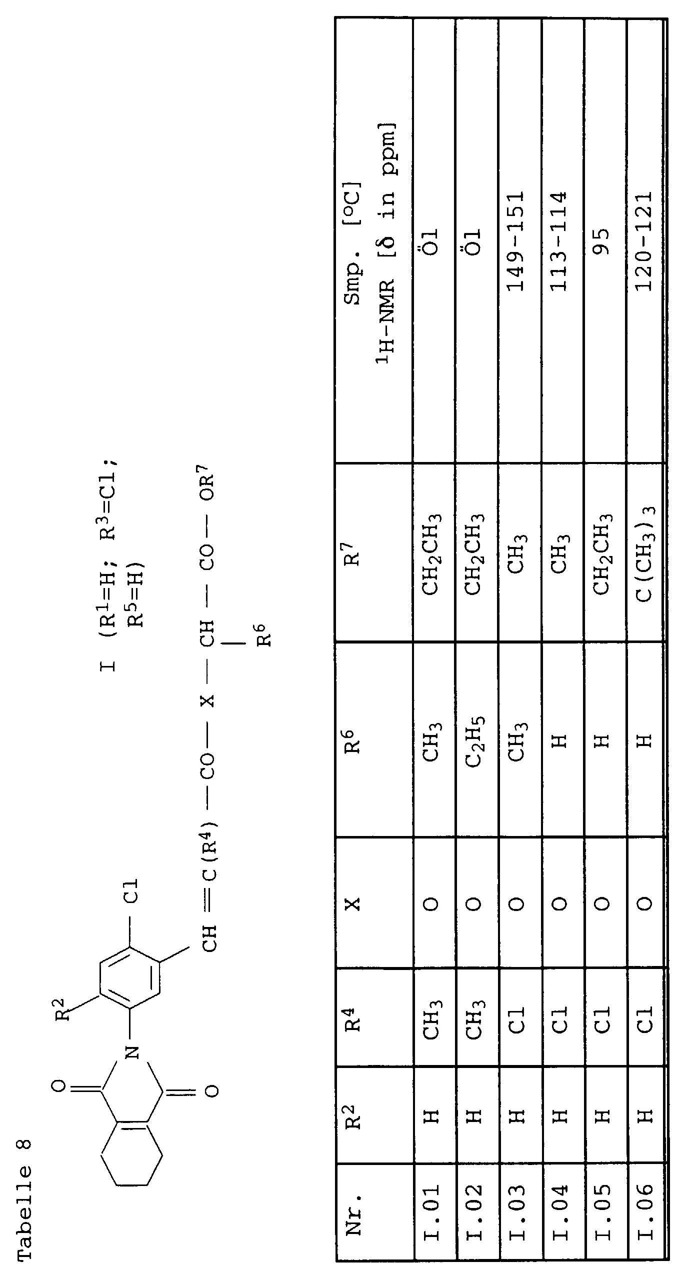

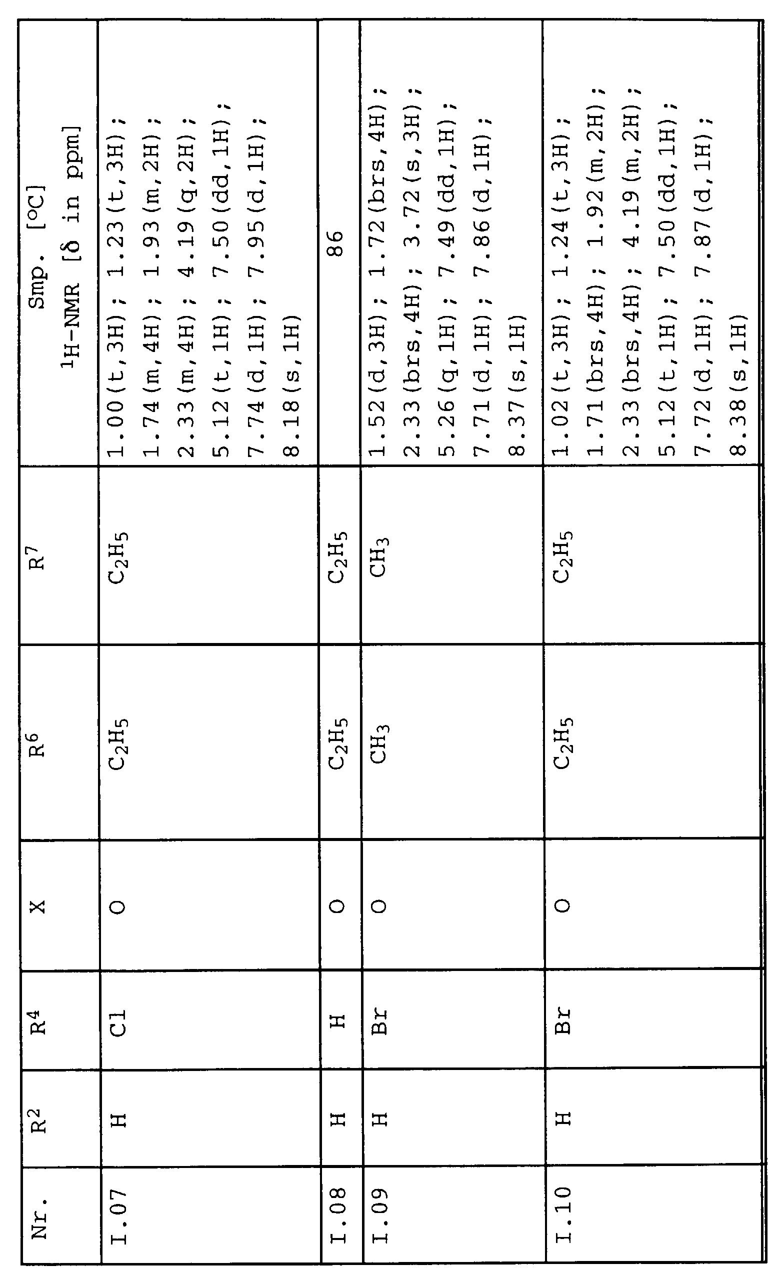

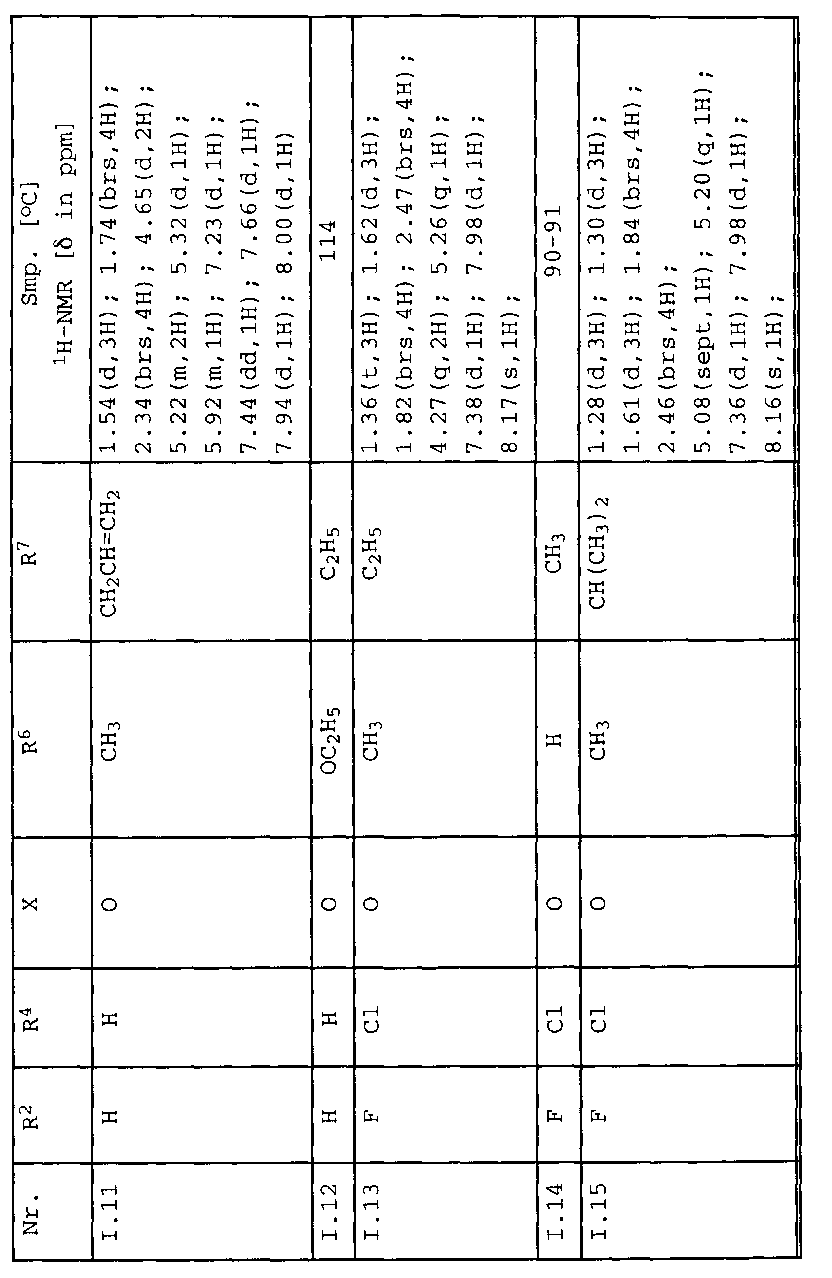

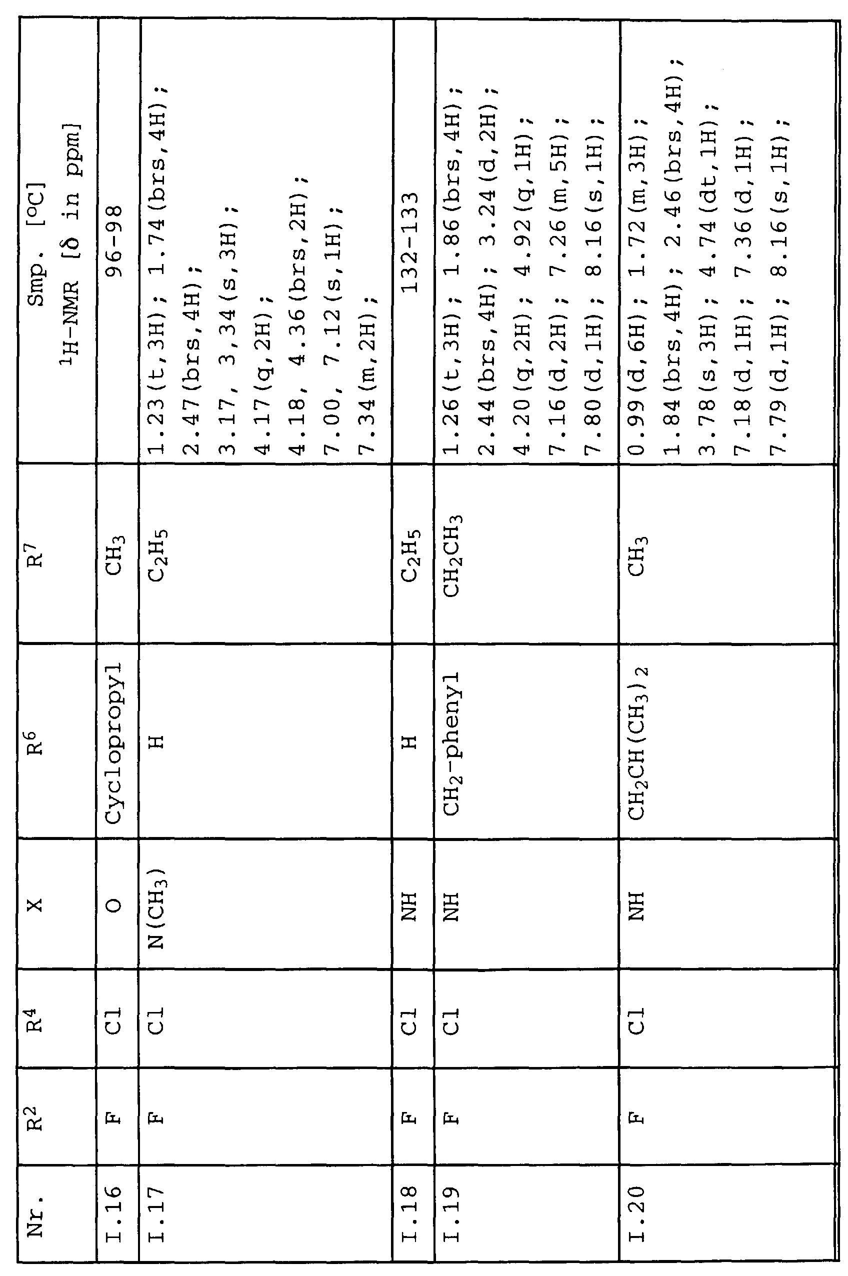

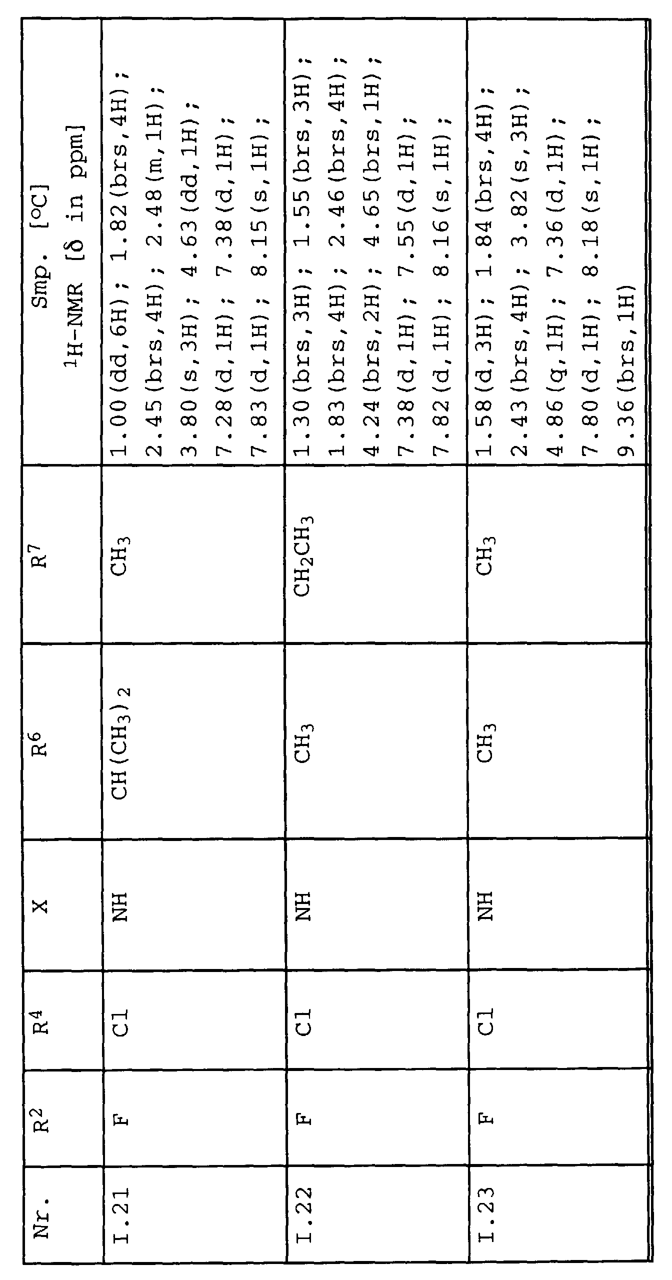

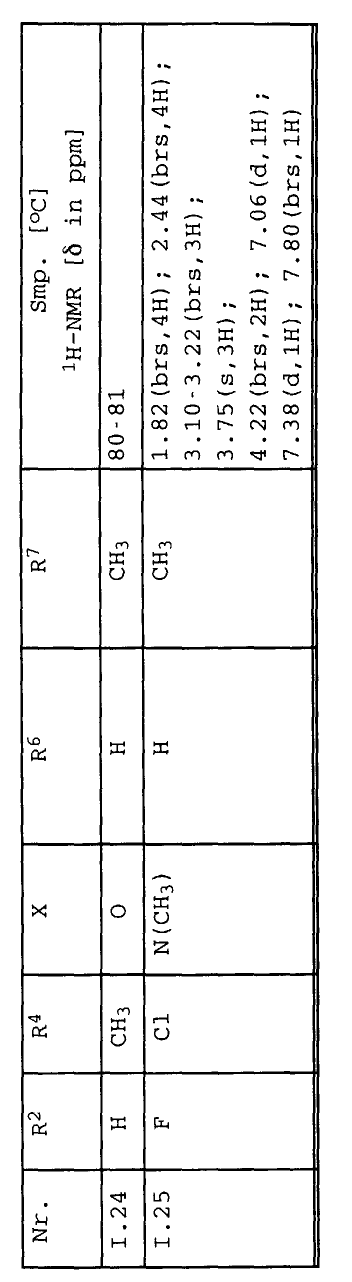

### Anwendungsbeispeile (herbizide Wirksamkeit)

Die herbizide Wirkung der substituierten Phthalimido-Zimtsäurederivate I ließ sich durch die folgenden Gewächshausversuche zeigen:

Als Kulturgefäße dienten Plastikblumentöpfe mit lehmigem Sand mit etwa 3,0 % Humus als Substrat. Die Samen der Testpflanzen wurden nach Arten getrennt eingesät.

Bei Vorauflaufbehandlung wurden die in Wasser suspendierten oder emulgierten Wirkstoffe direkt nach Einsaat mittels fein verteilender Düsen aufgebracht. Die Gefäße wurden leicht beregnet, um Keimung und Wachstum zu fördern, und anschließend mit durchsichtigen Plastikhauben abgedeckt, bis die Pflanzen angewachsen waren. Diese Abdeckung bewirkt ein gleichmäßiges Keimen der Testpflanzen, sofern dies nicht durch die Wirkstoffe beeinträchtigt wurde.

Zum Zweck der Nachauflaufbehandlung wurden die Testpflanzen je nach Wuchsform erst bis zu einer Wuchshöhe von 3 bis 15 cm angezogen und erst dann mit den in Wasser suspendierten oder emulgierten Wirkstoffen behandelt. Die Testpflanzen wurden dafür entweder direkt gesät und in den gleichen Gefäßen aufgezogen oder sie werden erst als Keimpflanzen getrennt angezogen und einige Tage vor der Behandlung in die Versuchsgefäße verpflanzt. Die Aufwandmenge für die Nachauflaufbehandlung betrug 0,0625, 0,0313, 0,0156 oder 0,0078 kg/ha a.S. (aktive Substanz).

Die Pflanzen wurden artenspezifisch bei Temperaturen von 10 - 25°C bzw. 20 - 35°C gehalten. Die Versuchsperiode erstreckte sich über 2 bis 4 Wochen. Während dieser Zeit wurden die Pflanzen gepflegt, und ihre Reaktion auf die einzelnen Behandlungen wurde ausgewertet.

Bewertet wurde nach einer Skala von 0 bis 100. Dabei bedeutet 100 kein Aufgang der Pflanzen bzw. völlige Zerstörung zumindest der oberirdischen Teile und 0 keine Schädigung oder normaler Wachstumsverlauf.

Die in den Gewächshausversuchen verwendeten Pflanzen setzten sich aus folgenden Arten zusammen:

| Lateinischer Name | Deutscher Name | Englischer Name |
|---|---|---|
| Amaranthus retroflexus | Rauhhaariger Amarant | carelessweed; redroot pigweed; common amaranth |
| Chenopodium album | Weißer Gänsefuß | lambsquarters; pigweed; fat-hen; white goosefoot |
| Chrysanthemum coronarium | Kronenwucherblume | crown daisy; garland chrysanthumum |
| Galium aparine Ipomoea subspecies | Klettenlabkraut Windearten | catchweed bedstraw morningglory |
| Matricaria inodora | Geruchlose Kamille | false chamomille; scentless chamomille; false mayweed; |
| Polygonum persicaria | Flohknöterich | lady's thumb; redshank |
| Sida spinosa | Stachelige Samtmalve | prickly sida; teaweed |
| Solanum nigrum | Schwarzer Nachtschatten | black nightshade |
| Triticum aestivum | Winterweizen | winter wheat |

Bei einer Aufwandmenge von 0,0625 oder 0,0313 kg/ha a.S. zeigte die Verbindung Nr. I.24 im Nachauflaufverfahren eine sehr gute Wirkung gegen Galium aparine und Solanum nigrum in der Kultur Weizen.

Die Verbindung Nr. I.01 bekämpf te bei einer Aufwandmenge von 0,0156 oder 0,0078 kg/ha a.S. im Nachauflaufverfahren Chenopodium album, Chrysanthemum coronarium, Ipomoea subspecies und Polygonum persicaria besser als die aus der EP-A 240 659 bekannte Vergleichsverbindung A

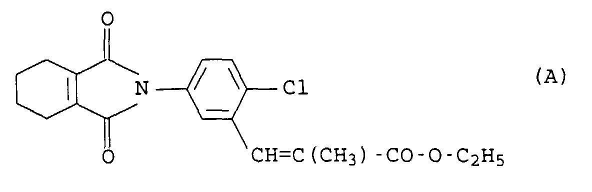

Die Verbindung Nr. I.08 war bei einer Aufwandmenge von 0,0625 oder 0,0313 kg/ha a.S. im Nachauflaufverfahren wirksamer im Test auf Amaranthus retroflexus, Chenopodium album, Ipomoea subspecies, Matricaria inodora und Sida spinosa als die aus der EP-A 240 659 bekannten Vergleichsverbindungen B und C:

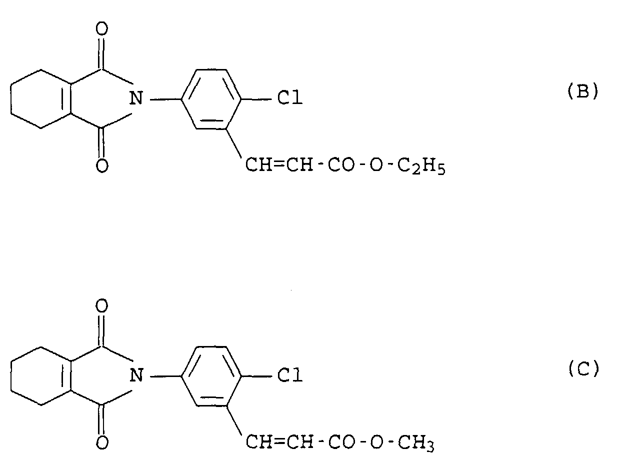

### Anwendungsbeispiele (desiccative/defoliante Wirksamkeit)

Als Testpflanzen dienten junge, 4-blättrige (ohne Keimblätter) Baumwollpflanzen, die unter Gewächshausbedingungen angezogen wurden (rel. Luftfeuchtigkeit 50 bis 70 %; Tag-/Nachttemperatur 27/20°C).

Die jungen Baumwollpflanzen wurden tropfnaß mit wässrigen Aufbereitungen der Wirkstoffe (unter Zusatz von 0,15 Gew.-% des Fettalkoholalkoxylats Plurafac LF 700, bezogen auf die Spritzbrühe) blattbehandelt. Die ausgebrachte Wassermenge betrug umgerechnet 1000 l/ha. Nach 13 Tagen wurde die Anzahl der abgeworfenen Blätter und der Grad der Entblätterung in % bestimmt.

Bei den unbehandelten Kontrollpflanzen trat kein Blattfall auf.

## Patentansprüche

1. Substituierte Phthalimido-Zimtsäurederivate der Formel I 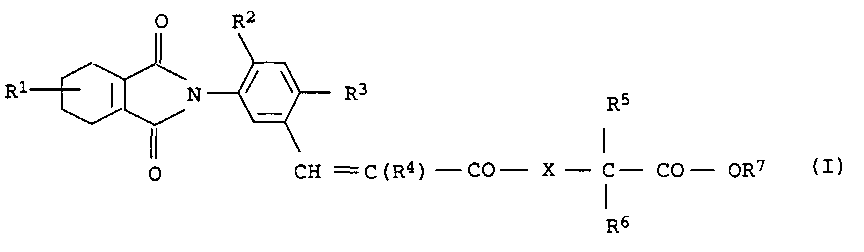 in der die Substituenten folgende Bedeutung haben:
R¹ Wasserstoff oder C₁-C₄-Alkyl;
R² Wasserstoff oder Halogen;
R³ Cyano, Nitro, Halogen, C₁-C₆-Alkyl, C₁-C₆-Halogenalkyl, C₁-C₆-Alkoxy oder C₁-C₆-Halogenalkoxy;
R⁴ Wasserstoff, Cyano, Nitro, Halogen oder C₁-C₆-Alkyl;
R⁵ Wasserstoff, C₁-C₆-Alkyl, C₂-C₆-Alkenyl, C₂-C₆-Alkinyl, C₁-C₆-Halogenalkyl, C₁-C₆-Cyanoalkyl, C₁-C₆-Hydroxyalkyl, C₁-C₆-Mercaptoalkyl, C₁-C₆-Alkoxy-C₁-C₆-alkyl oder C₁-C₆-Alkylthio-C₁-C₆-alkyl;
R⁶ einen der unter R⁵ genannten Reste oder Cyano, Nitro, C₁-C₆-Alkoxy, C₁-C₆-Halogenalkoxy, C₁-C₆-Alkylthio, C₁-C₆-Halogenalkylthio, C₃-C₆-Cycloalkyl, C₃-C₆-Cycloalkyl-C₁-C₆-alkyl, (C₁-C₆-Alkoxy)carbonyl-C₁-C₆-alkyl, Hydroxycarbonyl-C₁-C₆-alkyl, (C₁-C₆-Alkylamino)carbonyl-C₁-C₆-alkyl, Di(C₁-C₆-alkyl)aminocarbonyl-C₁-C₆-alkyl, Aminocarbonyl-C₁-C₆-alkyl, (C₁-C₆-Alkyl)carbonyl-C₁-C₆-alkyl, C₁-C₆-Alkoxyimino-C₁-C₆-alkyl, Hydroxyimino-C₁-C₆-alkyl, Di-(C₁-C₆-alkoxy)-C₁-C₆-alkyl, Di-(C₁-C₆-alkylthio)-C₁-C₆-alkyl, C₃-C₆-Halogenalkenyl, (C₁-C₆-Alkoxy)carbonyl, Hydroxycarbonyl, (C₁-C₆-Alkylamino)carbonyl, Di-(C₁-C₆-alkyl)aminocarbonyl, Aminocarbonyl-(C₁-C₆-alkyl)carbonyl, (C₁-C₆-Halogenalkyl)carbonyl, Aryl, Hetaryl, Aryl-C₁-C₆-alkyl oder Hetaryl-C₁-C₆-alkyl, wobei die Aryl- und Heteroarylringe gewünschtenfalls ein bis drei Reste tragen können, ausgewählt aus der Gruppe bestehend aus Cyano, Nitro, Halogen, C₁-C₆-Alkyl, C₁-C₆-Halogenalkyl, C₁-C₆-Alkoxy, C₁-C₆-Halogenalkoxy, C₁-C₆-Alkylthio, C₁-C₆-Halogenalkylthio, Hydroxy, Mercapto und (C₁-C₆-Alkoxy)carbonyl;
oder R⁵ und R⁶ zusammen eine zwei- bis sechsgliedrige Alkylenkette, in der eine Methyleneinheit durch Sauerstoff oder eine C₁-C₄-Alkyliminogruppe ersetzt sein kann;
R⁷ Wasserstoff, C₁-C₆-Alkyl, C₃-C₆-Alkenyl, C₃-C₆-Alkinyl, C₂-C₆-Halogenalkyl, C₁-C₆-Cyanoalkyl, C₁-C₆-Alkoxy-C₂-C₆-alkyl, C₁-C₆-Alkylthio-C₂-C₆-alkyl, (C₁-C₆-Alkoxy)carbonyl-C₁-C₆-alkyl, C₃-C₆-Cycloalkyl, C₃-C₆-Cycloalkyl-C₁-C₆-alkyl,
Phenyl oder Benzyl, wobei die Phenylringe jeweils ein bis drei Reste tragen können, ausgewählt aus der Gruppe bestehend aus Cyano, Nitro, Halogen, C₁-C₆-Alkyl, C₁-C₆-Halogenalkyl, C₁-C₆-Alkoxy, C₁-C₆-Halogenalkoxy, C₁-C₆-Alkylthio, C₁-C₆-Halogenalkylthio und (C₁-C₆-Alkoxy)carbonyl;
X Schwefel oder -N(R⁸)-, wobei
R⁸ für einen der unter R⁷ genannten Reste oder für (C₁-C₆-Alkoxy)carbonyl, (C₁-C₆-Halogenalkoxy)carbonyl, (C₁-C₆-Alkyl)carbonyl, (C₁-C₆-Halogenalkyl)carbonyl, Tri-(C₁-C₆-alkyl)-silyl, Aryloxycarbonyl, Arylmethoxycarbonyl steht
oder zusammen mit R⁶ für eine drei- bis 5-gliedrige Alkylenkette, in der eine nicht endständige Methyleneinheit durch Sauerstoff oder eine C₁-C₄-Alkyliminogruppe oder in der die N-gebundene Methyleneinheit durch eine Carbonylgruppe ersetzt sein kann,
sowie die landwirtschaftliche brauchbaren Salze von I.

2. Substituierte Phthalimido-Zimtsäurederivate der Formel I nach Anspruch 1, wobei R³ für Halogen oder Cyano und X für -N(R⁸)- stehen.

3. Substituierte Phthalimido-Zimtsäurederivate der Formel I nach Anspruch 1, wobei R² für Wasserstoff, Fluor oder Chlor steht.

4. Amino- und Nitrozimtsäurederivate der Formeln IIa und IIb 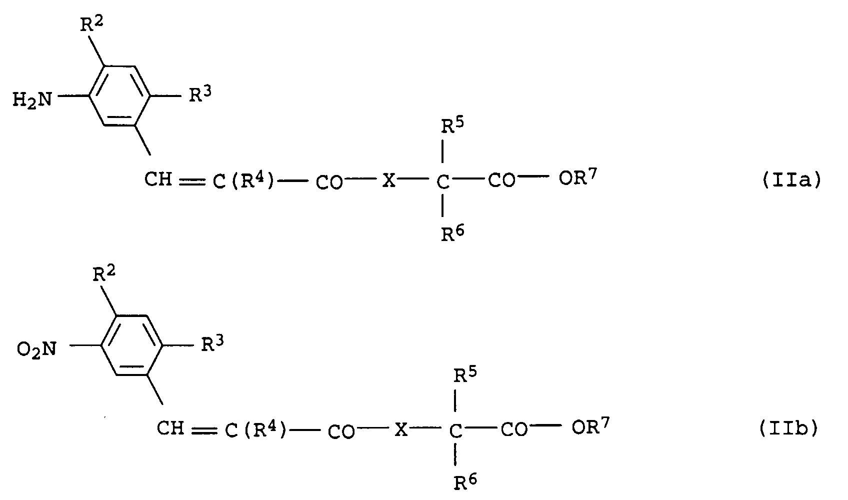 in der die Substituenten R² bis R⁷ und die Variable X die in Anspruch 1 angegebenen Bedeutungen haben.

5. Verwendung der substituierten Phthalimido-Zimtäurederivate der Formel I und der landwirtschaftlich brauchbaren Salze von I, gemäß Anspruch 1, als Herbizide oder zur Desiccation und/oder Defoliation von Pflanzen.

6. Herbizides Mittel, enthaltend eine herbizid wirksame Menge mindestens eines substituierten Phthalimido-Zimtsäurederivats der Formel I oder ein landwirtschaftlich brauchbares Salz von I, gemäß Anspruch 1, und mindestens einen inerten flüssigen und/oder festen Trägerstoff sowie gewünschtenfalls mindestens ein Adjuvants.

7. Mittel zur Desiccation und/oder Defoliation von Pflanzen, enthaltend eine desiccant und/oder defoliant wirksame Menge mindestens eines substituierten Phthalimido-Zimtsäurederivats der Formel I oder ein landwirtschaftlich brauchbares Salz von I, gemäß Anspruch 1, und mindestens einen inerten flüssigen und/oder festen Trägerstoff sowie gewünschtenfalls mindestens ein Adjuvants.

8. Verfahren zur Herstellung von herbizid wirksamen Mitteln, dadurch gekennzeichnet, daß man eine herbizid wirksame Menge mindestens eines substituierten Phthalimido-Zimtsäurederivats der Formel I oder ein landwirtschaftlich brauchbares Salz von I, gemäß Anspruch 1, und mindestens einen inerten flüssigen und/oder festen Trägerstoff sowie gewünschtenfalls mindestens ein Adjuvants mischt.

9. Verfahren zur Herstellung von desiccant und/oder defoliant wirksamen Mitteln, dadurch gekennzeichnet, daß man eine desiccant und/oder defoliant wirksame Menge mindestens eines substituierten Phthalimido-Zimtsäurederivats der Formel I oder ein landwirtschaftlich brauchbares Salz von I, gemäß Anspruch 1, und mindestens einen inerten flüssigen und/oder festen Trägerstoff sowie gewünschtenfalls mindestens ein Adjuvants mischt.

10. Verfahren zur Bekämpfung von unerwünschtem Pflanzenwuchs, dadurch gekennzeichnet, daß man eine herbizid wirksame Menge mindestens eines substituierten Phthalimido-Zimtsäurederivats der Formel I oder eines landwirtschaftlich brauchbaren Salzes von I, gemäß Anspruch 1, auf Pflanzen, deren Lebensraum oder auf Saatgut einwirken läßt.

11. Verfahren zur Desiccation und/oder Defoliation von Pflanzen, dadurch gekennzeichnet, daß man eine desiccant und/oder defoliant wirksame Menge mindestens eines substituierten Phthalimido-Zimtsäurederivats der Formel I oder eines landwirtschaftlich brauchbaren Salzes von I, gemäß Anspruch 1, auf Pflanzen einwirken läßt.

12. Verfahren zur Herstellung von substituierten Phthalimido-Zimtsäurederivaten der Formel I gemäß Anspruch 1, wobei R⁴ für Chlor oder Brom steht, dadurch gekennzeichnet, daß man ein Anilin der Formel III 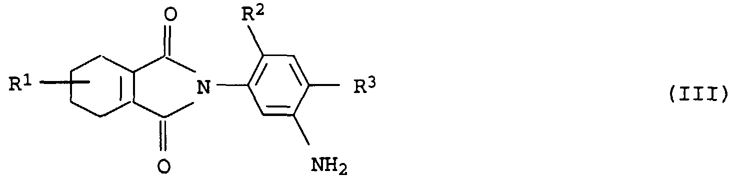 und ein Propinsäurederivat IV 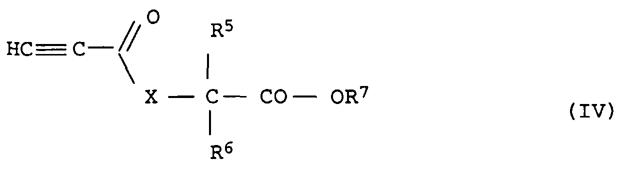 auf an sich bekannte Weise einer Arylierungsreaktion nach Meerwein unterwirft.

13. Verfahren zur Herstellung von substituierten Phthalimido-Zimtsäurederivaten der Formel I gemäß Anspruch 1, dadurch gekennzeichnet, daß man einen Benzaldehyd der Formel V 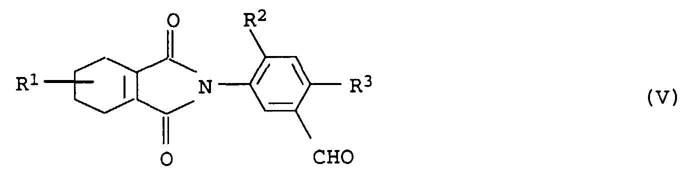 mit einem Ylid der Formel VI 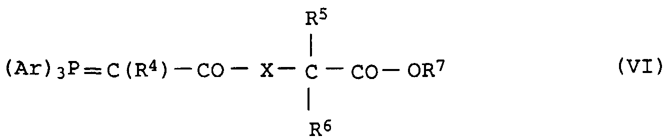 wobei Ar für einen aromatischen Rest steht, der gewünschtenfalls substituiert sein kann, umsetzt.

14. Verfahren zur Herstellung von substituierten Phthalimido-Zimtsäurederivaten der Formel I gemäß Anspruch 1, wobei R⁴ für Nitro oder Cyano steht, dadurch gekennzeichnet, daß man einen Benzaldehyd der Formel V 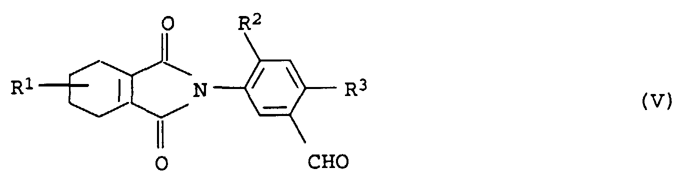 auf an sich bekannte Weise mit einer CH-aciden Verbindung der Formel VII 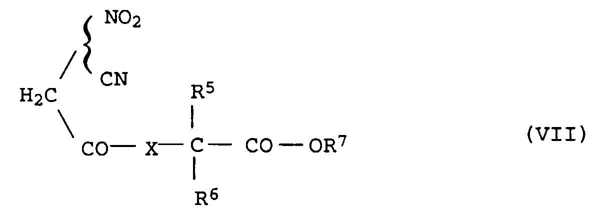 umsetzt.

15. Verfahren zur Herstellung von substituierten Phthalimido-Zimtsäurederivaten der Formel I gemäß Anspruch 1, dadurch gekennzeichnet, daß man auf an sich bekannte Weise entweder eine aktivierte Carbonsäure der Formel VIII 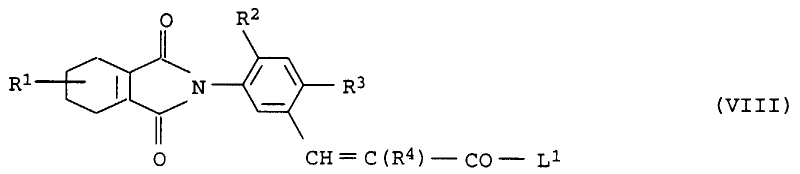 wobei L¹ für eine übliche Abgangsgruppe steht, mit einem Nukleophil der Formel IX 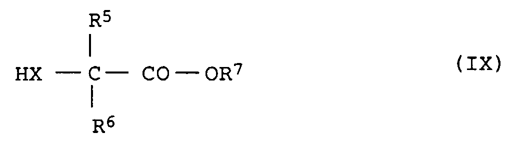 oder eine aktivierte Carbonsäure der Formel X 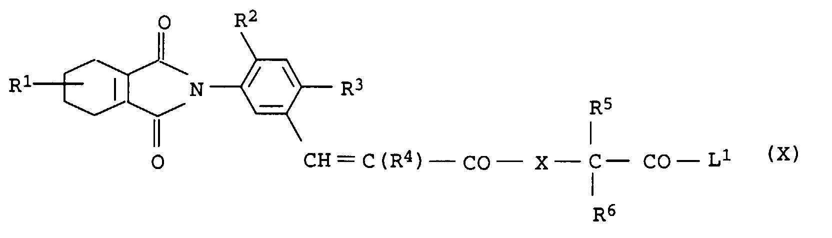 mit einem Alkohol HOR⁷ umsetzt.

16. Verfahren zur Herstellung von substituierten Phthalimido-Zimtsäurederivaten der Formel I gemäß Anspruch 1, dadurch gekennzeichnet, daß man auf an sich bekannte Weise entweder ein Säurederivat XII 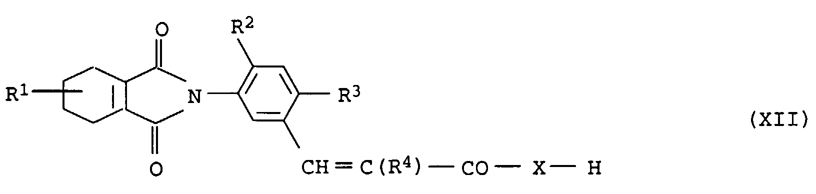 mit einem Alkylierungsmittel XIII 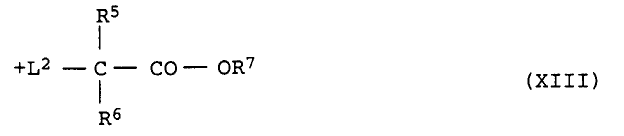 wobei L² für eine übliche Abgangsgruppe steht,
oder eine Verbindung I gemäß Anspruch 1, wobei R⁷ für Wasserstoff steht, mit einem Alkylierungsmittel L²R⁷ umsetzt.

17. Verfahren zur Herstellung von substituierten Phthalimido-Zimtsäurederivaten der Formel I gemäß Anspruch 1, dadurch gekennzeichnet, daß man ein Tetrahydrophthalsäureanhydrid der Formel XIV 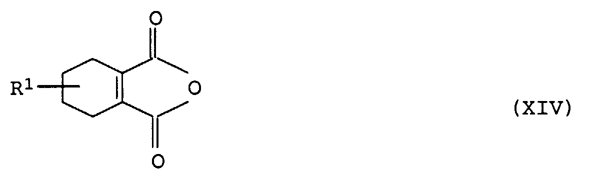 auf an sich bekannte Weise mit einem Aminozimtsäurederivat IIa gemäß Anspruch 4 umsetzt.

18. Verfahren zur Herstellung von Nitrozimtsäurederivaten der Formel IIb gemäß Anspruch 4, dadurch gekennzeichnet, daß man die entsprechend substituierten Aminozimtsäurederivate IIa auf an sich bekannte Weise reduziert.
